# EUROPEAN PATENT APPLICATION

(11) **EP 2 319 943 A1**
(43) Date of publication of application: **11.05.2011**
(21) Application number: 10190164.3
(22) Date of filing: 05.11.2010
(51) Int. Cl.: C12Q 1/68

(54) **Methods of predicting therapeutic efficacy of cancer therapy**

(30) Priority: 05.11.2009 EP 09175108; 24.03.2010 EP 10157499; 19.08.2010 EP 10173402
(71) Applicant: Epigenomics AG, 10178 Berlin (DE); Technische Universität München, 80333 München (DE)
(72) Inventor: Lofton-Day, Catherine, Seattle, WA 98103 (US); Tetzner, Reimo, 10439 Berlin (DE); Ebert, Matthias Philip Alexander, 81735 Munich (DE); Tänzer, Marc, 81669 Munich (DE)
(74) Representative: Krauss, Jan

(57) **Abstract**

The present invention relates to novel methods and kits for predicting, prognosing, and/or monitoring therapeutic efficacy of cancer therapy on a subject having malignant tumor or cell proliferative disorder.

SEQUENCE LISTING

<110>  Epigenomics AG
       Technischen Universitaet Muenchen

<120>  Methods of predicting therapeutic efficacy of cancer therapy

<130>  FB23937

<150>  EP09175108.1
<151>  2009.11.05

<150>  EP10157499.4
<151>  2010.03.24

<150>  EP10173402.8
<151>  2010.08.19

<160>  58

<170>  PatentIn version 3.5

<210>  1
<211>  6354
<212>  DNA
<213>  Homo sapiens

<400>  1
gatcttggtt cactgcaacc tccgccccgc aggttcaagg gattctcctg cctcagcctc      60

cggagtagct gggattacag gtgcctgcca ccccacccag ctaatttttg tatttttagt     120

agaaacgggg tttcaccata ttggccaggc tggtctcgaa ctccagacct caggtgatcc     180

acccgcctcg gcctcccaaa gtgctgggat cacatggtgt gagccaccgc cgcccggcct     240

cttttttttt tttttttttt tttttttttt tgagatagtc tccctctgtt gtccaggctg     300

gagtacagtg gcatgatctc ggctcactgc aacctctgcc tcctgggtca agcaattctc     360

ttgcctcagc ctcctgagtg gctgggatta caggctagcg ccaccacact gggctaattt     420

ttgtattttt agtagagatg gggtttcacc atcttggcca ggctggtctc gaactcttga     480

cctcaagtga tccggcctcc caaagtgctg ggattacaga cgtgagccac tgcgcccggc     540

ctatttatct tgattactga gttttttggc accctctcaa attctgtccc agaagtcagt     600

atctcactca tctcacccat gtcctggctg cttagagatt gtctgggaag tggagaccct     660

ccccagctcc ctcctccttc tccagggcca aagacagcaa ggaatagggc gactgtttac     720

atcagctcct gtcctagaga tcccctgaga ttggacagct gagcccgtgg agagggattc     780

cccaccccca agtaggcaac cacccactct cctccagagg gaacgaggga tccatctcag     840

ttctcatcct tgaccactcc cctccagctc tgattgtata acctgaaacc cacacacggt     900

ttgtaacttt tttttttttt ttccgagacg gagtctcgct ctgtttccag gctggagtgc     960

agtggcgcga tcttggctca ctgcaatctc cgcctcccgg gttcaagcga ttcccctggc    1020

tcagcctccc tagtagctgg gactacagac gcgcaccacc atgcccggct aatttgtttg    1080

taaccttttt aacctgtctg gcttttccac agggagagga tttgcatatg ctgtgtcctg    1140

tgtgatgaaa ggagcccttt ttacacaccc cctctttcaa tgcactcaga cttcaaagcc    1200

catcactcat tcattcaaca aacacccact aagcacctct tgaacccggt tcaactatct    1260

cccctctagg cactcaccca acctcgggca ctctccgcgc ctcctctgag ctcctcccgc 1320
gtcccataca gcccctgctc actgctggaa aatattcctg ttaagctccg tctccccacc 1380
agtcctgctt gctttctgtg tgtctgggat aggttgcaaa actggaggtg acaaatgtgg 1440
gcaggaaatg gagggctccc ttatactcct agggtcggcc tcagccctgc catttcctgc 1500
ccaataccgc ggatgcaact ggcatgggac ccggaagtgt gtggtaaagc gccggggctt 1560
cgcctggccg cccctcccgg acgcttgcct gcagtcagcg aagcttttcc aacccaggtc 1620
tgggccctgc gagctccagg tgcctctgcc ccgcggcgct ggcgaagtcg aagcccgaga 1680
acgcccaccg cagcgatgcg aaggccgctt ctggggtggg gctgaggctg cagctgcctc 1740
cctttcgcac caaggacccc aaccttcagc gacgcagccg ccgctcccgc ccaggctggg 1800
aggcactgta gggatccgac gcttcaggtg gccaaagagc gactttctct gattccaggg 1860
ccctggcggg gcaggctcca gtaccgcact cggcggaggc cgaaggtctg tggggcagga 1920
caggagttcc tcgtgccgtc ggaagggccg aggacgaagg agggcgctaa ttcactcccc 1980
actgggctgg cggcaacgcc gaatcccgca gtgaccgcgg agggccaagg tgaaaactgc 2040
tgggggcgcc gagggcaggt gtggggaggg gcggctccag ggagcaagga gcccacctgc 2100
ctcgccgccg cagctgccct gggtcgaccg cccacgcttt cccctgggtc acgacctccg 2160
gatccaactg tgcccggctc ctgccccttc ccctttgctg ctgctgcgcg ggctgtaact 2220
tgacgtctag gctggggcgt ccagggcgca gtctccgctc aggtcgccag cgctccactc 2280
gctccactgg gccacagacc cgctggcgct ggggtcccgc tggcgctggg gtcccgctgg 2340
cgctggggtc ccgctggcgc tggggtcccg ctggcgctgg ggtcccgctg gcgctggggt 2400
cccgctggcg ctggggtccc gcggttgtgc acgtgagtcc gcgtggcccc gtcccgcgcg 2460
gttgtgcacg tgagtccgcg cggccgcgtc ccgccccgct ccagggagcc agcgcgccgc 2520
cacctgggat gttaggactc tcgttgtgtt ctttggactg ctttggggga tttcggcgta 2580
cccctaggat ctaggagttt tggaagttgt ttgagagaaa ccagctctgg gagggcctcg 2640
catccagtct cctgtctcgg cttcggaccg gggttccggg ttaaggtgct cagaggaaca 2700
gctgatcctt ctacccttgc gcagggcaga gatccccaaa cctctttcta aaatgcaggg 2760
tcttagtctt tctcagggag tcagtgaacc tagactccca gtctcttgag cccaagcatg 2820
aatagggaac tggggaccac caccatgctc atatttctgg tggccaggaa gtccaggcag 2880
gcttttgccc actgcagacg gatctctttc ttcaggggtc aagaaaggct ctgtacagta 2940
agccaaccaa gccccaccag cagagctgcg ctgcaaccag gttcctagtg tctttcccac 3000
actcccagtt ccaagcgaca caggacacag ccccaattcc acctcccccc acccacttcc 3060
tgccctgttg cctcatcttc cctgctacac actccaagtc atccacccaa cccccagctc 3120
ctggtaactc agccccagct cctggaagtc cagccccagc cctgctacca ccacttccac 3180
cttggcctcc cctgagaaca agtggagggc aaatagagcc caggcttgaa ttcactcgtt 3240
tgaaaaacaa ctcaagccaa accctgtggg agcagtgcct gcctggggca cctcaggctc 3300
atgtgcacct ctgaattctt tcgggcacct gccccaaatg taaagaggct gctacccaat 3360

gcctgggccc ctctgactct ctgctcctct ctggctgtct ctctgttttt gtcgtctctc 3420
accttcccct gtcatggcca gtacttggtc ctcaggccag aaaaggtgga tcccgtgtcc 3480
ctggatttta ctaatgccag gagtcacata aatactccta tatatacaca gagagggccc 3540
gcgcatgctt ggaaatcctc agataccaaa gaacaaagtg caggacacaa cagagatact 3600
gtacatattg agaaatgtgt acatccccca aatgcagaga aatgcacacc aacatacaga 3660
gacacacctc acaaacacat tcacagaaac agacacactc acactccaag aaatactaag 3720
acacccatga agggaacatt tcagacatgc acaggaccct gaactcacat ggacacagac 3780
acccaggagc aggtgggccc tgactcaggt gcacacagcc taacacacag tactcacaca 3840
caagctcccc taagtctaaa tgccgcaaga gactcccaca gaaagaaaat tcccccagag 3900
gcctccaagg ccctgcctgg aaggaagagg aagaaagtgc ccgccaggca ccgaaatgct 3960
aaggcactgt aaagtgaaaa tcatttcccc aactccgtgg gaacagcact tccattccca 4020
tctcttaggg aaacagggaa gtggtttaac tcgttctgca aatcaactcc agatccacaa 4080
gagtgccctc cccctcggcg gggagaggcc aggctcccag tgctgcagcc cagtgaatgc 4140
tgatccgctg aggcccaccc caggccacag ggtgctgggc aaactcacca agcccgcacg 4200
tctgcctaac atctggctgg gccaggaaga gtgcctggtg gacacccagc ctggcgacac 4260
acaggaggca cggccggtga agaaaatgga tctgtgcacc aagggcctgc accatctcgc 4320
cgggctctgc cctgacagac cattccttac ctttcttgat cacggactgg tctaggaggc 4380
tcattcccgg ctcgtccatt gcctgcaaat agcagagggc agcggtgagg gcgcataaag 4440
ccctagccgc ccatgtcttc ccaagcccct gccacccagg gtctctgtct gctccgccca 4500
aggcgcacca gcttcgcttg gggcggcgac gggcaggccc aggaatcgct atgtccaggc 4560
actgagctgg agccctgggc atctcactgt gcagtgcaaa aagggaattt tgaatcttac 4620
actggtaact attatgcttc tcttcgcccc aggtgcaggc tgccccatgg atgccacacg 4680
tggcagcggg gagaaaagaa gtgattcggt ccctgccgag ggttgctgcc acccacgcac 4740
tgctggcgga ctcagtgcca ggcagccttg atggccaggc aacgtctaac ccaggaggtg 4800
ggacacatcc tcggagtcac cagtgagatg tggcgagctg ggagaaaagt cggcttcctg 4860
catttgtcac cttctgcctc cagccatggt agtaaaccac cgcggccagt cccgacccat 4920
cccggatccc tcgggtctca cctgcaggtc ctccagaccg ggggccgccg ccagccccgg 4980
aaggccgaga ggtgcgtctg ccaggccgtg cgcgccgtcg gccaggccgt gcaggagccg 5040
gggcacggca gtggcatagt cacggcgcgg gtcaaggccc agggcgcggg cgggcggtgc 5100
cagcaggccg ggaggctcct cgtgggcgcg ggcggctgcg cggggcgcgg cccaggcggc 5160
ctgaggaggc tgcggctgcg ccaggggcgc caggccgcca tatgggtccc ctgccaggtg 5220
gggaaaggct gcggcggcgt cgggcgcctg accgtagggc agcggtggct gcgggtaggg 5280
cggcgggaag tagggcggct ggaattcggc ggcagctgtg gcggccggcg tgtggcagag 5340
cgggggcgcc ggcccgtagg ccgcctgggg cagagacgac aggcgggccc cgccggcagc 5400
tgctcccagc ccgtcggggc gctcctgggg aaggagaggg gtgccttagg tgacccactg 5460

gcagctgaaa ggaccccagc ctgaaaggat cctcctcagc tcgggagggt tcagctgcag 5520

tccctcccgt ttcttggggc ccgaggttta tttccgagcc acccgcgggc gcaggagcct 5580

caggctgggg acagcggtca cttgcgcatc gcgttctcag ctggcgaagt ccctgcggcc 5640

ccctccctcc gagatttcgt tagcgcggga caggagggtt ggaaagtcca gcactcaggc 5700

gccccgatcc cccggccgaa tatgtcccgg gcccgagact actcaccatg gcggagtagg 5760

tgtgcaccag catggggcgg tgagtgccgg gcgcgaggcg tggcggcggc ggagccctag 5820

cccgacggcg agcgctgcgg gcagaggcgc cgggagccgg cacagcccgc ggaggtcacg 5880

gtgcggtagc tgggtgcgac ccagccggga tcctagtggg cgtggggtcc tcagcggtgg 5940

ccgttcccgg gcgggtccat ggaggcagga cggacgggca ctgatcgcac ttggaaaagt 6000

cggtcaacgg gtgcccagcg cccgcccttc ctcctctgag cgtagcctgc tccatgcact 6060

ccagttatag tggcgggggc gcgccgggcc tccgggggcg cgcattaaag agacaagcta 6120

tggcagttct ctcccttttc cccagcggct gccacagggg aggcagtggc tttggcggga 6180

tcgggtaggg tgggtagggg gcgtgaattc agcccacatt gatcagagcc gccttctggg 6240

ctcgcagctc gcagctgcag tgaaacagaa atattcgagg ctgggacgca agggccctac 6300

atttgcctgg ctctatcaca tccttgcctt taagcaaagc actattcttc gctg 6354

<210> 2
<211> 6354
<212> DNA
<213> Artificial Sequence

<220>
<223> TFAP2E CpG methylated Sense Bisulfite Sequence

<400> 2
gattttggtt tattgtaatt ttcgtttcgt aggtttaagg gatttttttg ttttagtttt 60

cggagtagtt gggattatag gtgtttgtta ttttatttag ttaatttttg tatttttagt 120

agaaacgggg ttttattata ttggttaggt tggtttcgaa ttttagattt taggtgattt 180

attcgtttcg gttttttaaa gtgttgggat tatatggtgt gagttatcgt cgttcggttt 240

tttttttttt tttttttttt tttttttttt tgagatagtt tttttttgtt gtttaggttg 300

gagtatagtg gtatgatttc ggtttattgt aatttttgtt ttttgggtta agtaattttt 360

ttgttttagt tttttgagtg gttgggatta taggttagcg ttattatatt gggttaattt 420

ttgtattttt agtagagatg gggttttatt attttggtta ggttggtttc gaatttttga 480

ttttaagtga ttcggttttt taaagtgttg ggattataga cgtgagttat tgcgttcggt 540

ttatttattt tgattattga gttttttggt atttttttaa attttgtttt agaagttagt 600

attttattta ttttatttat gttttggttg tttagagatt gtttgggaag tggagatttt 660

ttttagtttt tttttttttt tttagggtta aagatagtaa ggaatagggc gattgtttat 720

attagttttt gttttagaga ttttttgaga ttggatagtt gagttcgtgg agagggattt 780

tttattttta agtaggtaat tatttatttt tttttagagg gaacgaggga tttattttag 840

tttttatttt tgattatttt tttttagttt tgattgtata atttgaaatt tatatacggt 900

ttgtaatttt tttttttttt tttcgagacg gagtttcgtt ttgtttttag gttggagtgt 960
agtggcgcga ttttggttta ttgtaatttt cgtttttcgg gtttaagcga tttttttggt 1020
ttagtttttt tagtagttgg gattatagac gcgtattatt atgttcggtt aatttgtttg 1080
taattttttt aatttgtttg gtttttttat agggagagga tttgtatatg ttgtgttttg 1140
tgtgatgaaa ggagtttttt ttatatattt ttttttttaa tgtatttaga ttttaaagtt 1200
tattatttat ttatttaata aatatttatt aagtattttt tgaattcggt ttaattattt 1260
tttttttagg tatttattta atttcgggta tttttcgcgt tttttttgag tttttttcgc 1320
gttttatata gtttttgttt attgttggaa aatatttttg ttaagtttcg tttttttatt 1380
agttttgttt gttttttgtg tgtttgggat aggttgtaaa attggaggtg ataaatgtgg 1440
gtaggaaatg gagggttttt ttatattttt agggtcggtt ttagttttgt tattttttgt 1500
ttaatatcgc ggatgtaatt ggtatgggat tcggaagtgt gtggtaaagc gtcggggttt 1560
cgtttggtcg tttttttcgg acgtttgttt gtagttagcg aagttttttt aatttaggtt 1620
tgggttttgc gagttttagg tgtttttgtt tcgcggcgtt ggcgaagtcg aagttcgaga 1680
acgtttatcg tagcgatgcg aaggtcgttt ttggggtggg gttgaggttg tagttgtttt 1740
tttttcgtat taaggatttt aatttttagc gacgtagtcg tcgttttcgt ttaggttggg 1800
aggtattgta gggattcgac gttttaggtg gttaaagagc gatttttttt gattttaggg 1860
ttttggcggg gtaggtttta gtatcgtatt cggcggaggt cgaaggtttg tggggtagga 1920
taggagtttt tcgtgtcgtc ggaagggtcg aggacgaagg agggcgttaa tttatttttt 1980
attgggttgg cggtaacgtc gaatttcgta gtgatcgcgg agggttaagg tgaaaattgt 2040
tgggggcgtc gagggtaggt gtggggaggg gcggttttag ggagtaagga gtttatttgt 2100
ttcgtcgtcg tagttgtttt gggtcgatcg tttacgtttt tttttgggtt acgattttcg 2160
gatttaattg tgttcggttt ttgttttttt ttttttgttg ttgttgcgcg ggttgtaatt 2220
tgacgtttag gttggggcgt ttagggcgta gttttcgttt aggtcgttag cgttttattc 2280
gttttattgg gttatagatt cgttggcgtt ggggtttcgt tggcgttggg gtttcgttgg 2340
cgttggggtt tcgttggcgt tggggtttcg ttggcgttgg ggtttcgttg gcgttggggt 2400
ttcgttggcg ttggggtttc gcggttgtgt acgtgagttc gcgtggtttc gtttcgcgcg 2460
gttgtgtacg tgagttcgcg cggtcgcgtt tcgtttcgtt ttagggagtt agcgcgtcgt 2520
tatttgggat gttaggattt tcgttgtgtt ttttggattg ttttggggga tttcggcgta 2580
tttttaggat ttaggagttt tggaagttgt ttgagagaaa ttagttttgg gagggtttcg 2640
tatttagttt tttgtttcgg tttcggatcg ggtttcgggg ttaaggtgtt tagaggaata 2700
gttgattttt ttatttttgc gtagggtaga gatttttaaa ttttttttta aaatgtaggg 2760
ttttagtttt ttttagggag ttagtgaatt tagattttta gttttttgag tttaagtatg 2820
aatagggaat tggggattat tattatgttt atatttttgg tggttaggaa gtttaggtag 2880
gtttttgttt attgtagacg gatttttttt tttaggggtt aagaaaggtt ttgtatagta 2940
agttaattaa gttttattag tagagttgcg ttgtaattag gtttttagtg ttttttttat 3000

atttttagtt ttaagcgata taggatatag ttttaatttt attttttttt atttattttt 3060
tgttttgttg ttttattttt tttgttatat attttaagtt atttatttaa tttttagttt 3120
ttggtaattt agttttagtt tttggaagtt tagttttagt tttgttatta ttatttttat 3180
tttggttttt tttgagaata agtggagggt aaatagagtt taggtttgaa tttattcgtt 3240
tgaaaaataa tttaagttaa attttgtggg agtagtgttt gtttggggta ttttaggttt 3300
atgtgtattt ttgaattttt tcgggtattt gttttaaatg taaagaggtt gttatttaat 3360
gtttgggttt ttttgatttt ttgttttttt ttggttgttt ttttgttttt gtcgtttttt 3420
attttttttt gttatggtta gtatttggtt tttaggttag aaaaggtgga tttcgtgttt 3480
ttggatttta ttaatgttag gagttatata aatattttta tatatatata gagagggttc 3540
gcgtatgttt ggaaattttt agatattaaa gaataaagtg taggatataa tagagatatt 3600
gtatatattg agaaatgtgt atatttttta aatgtagaga aatgtatatt aatatataga 3660
gatatatttt ataaatatat ttatagaaat agatatattt atattttaag aaatattaag 3720
atatttatga agggaatatt ttagatatgt ataggatttt gaatttatat ggatatagat 3780
atttaggagt aggtgggttt tgatttaggt gtatatagtt taatatatag tatttatata 3840
taagtttttt taagtttaaa tgtcgtaaga gatttttata gaaagaaaat ttttttagag 3900
gtttttaagg ttttgtttgg aaggaagagg aagaaagtgt tcgttaggta tcgaaatgtt 3960
aaggtattgt aaagtgaaaa ttattttttt aatttcgtgg gaatagtatt tttattttta 4020
ttttttaggg aaatagggaa gtggtttaat tcgttttgta aattaatttt agatttataa 4080
gagtgttttt tttttcggcg gggagaggtt aggtttttag tgttgtagtt tagtgaatgt 4140
tgattcgttg aggtttattt taggttatag ggtgttgggt aaatttatta agttcgtacg 4200
tttgtttaat atttggttgg gttaggaaga gtgtttggtg gatatttagt ttggcgatat 4260
ataggaggta cggtcggtga agaaaatgga tttgtgtatt aagggtttgt attatttcgt 4320
cgggttttgt tttgatagat tattttttat tttttttgat tacggattgg tttaggaggt 4380
ttattttcgg ttcgtttatt gtttgtaaat agtagagggt agcggtgagg gcgtataaag 4440
ttttagtcgt ttatgttttt ttaagttttt gttatttagg gtttttgttt gtttcgttta 4500
aggcgtatta gtttcgtttg gggcggcgac gggtaggttt aggaatcgtt atgtttaggt 4560
attgagttgg agttttgggt attttattgt gtagtgtaaa aagggaattt tgaattttat 4620
attggtaatt attatgtttt ttttcgtttt aggtgtaggt tgttttatgg atgttatacg 4680
tggtagcggg gagaaaagaa gtgattcggt ttttgtcgag ggttgttgtt atttacgtat 4740
tgttggcgga tttagtgtta ggtagttttg atggttaggt aacgtttaat ttaggaggtg 4800
ggatatattt tcggagttat tagtgagatg tggcgagttg ggagaaaagt cggttttttg 4860
tatttgttat tttttgtttt tagttatggt agtaaattat cgcggttagt ttcgatttat 4920
ttcggatttt tcgggtttta tttgtaggtt ttttagatcg ggggtcgtcg ttagtttcgg 4980
aaggtcgaga ggtgcgtttg ttaggtcgtg cgcgtcgtcg gttaggtcgt gtaggagtcg 5040
gggtacggta gtggtatagt tacggcgcgg gttaaggttt agggcgcggg cgggcggtgt 5100

tagtaggtcg ggaggttttt cgtgggcgcg ggcggttgcg cggggcgcgg tttaggcggt 5160

ttgaggaggt tgcggttgcg ttaggggcgt taggtcgtta tatgggtttt ttgttaggtg 5220

gggaaaggtt gcggcggcgt cgggcgtttg atcgtagggt agcggtggtt gcgggtaggg 5280

cggcgggaag tagggcggtt ggaattcggc ggtagttgtg gcggtcggcg tgtggtagag 5340

cgggggcgtc ggttcgtagg tcgtttgggg tagagacgat aggcgggttt cgtcggtagt 5400

tgtttttagt tcgtcggggc gtttttgggg aaggagaggg gtgttttagg tgatttattg 5460

gtagttgaaa ggattttagt ttgaaaggat tttttttagt tcgggagggt ttagttgtag 5520

tttttttcgt tttttggggt tcgaggttta ttttcgagtt attcgcgggc gtaggagttt 5580

taggttgggg atagcggtta tttgcgtatc gcgtttttag ttggcgaagt ttttgcggtt 5640

tttttttttc gagatttcgt tagcgcggga taggagggtt ggaaagttta gtatttaggc 5700

gtttcgattt ttcggtcgaa tatgtttcgg gttcgagatt atttattatg gcggagtagg 5760

tgtgtattag tatggggcgg tgagtgtcgg gcgcgaggcg tggcggcggc ggagttttag 5820

ttcgacggcg agcgttgcgg gtagaggcgt cgggagtcgg tatagttcgc ggaggttacg 5880

gtgcggtagt tgggtgcgat ttagtcggga ttttagtggg cgtggggttt ttagcggtgg 5940

tcgttttcgg gcgggtttat ggaggtagga cggacgggta ttgatcgtat ttggaaaagt 6000

cggttaacgg gtgtttagcg ttcgtttttt tttttttgag cgtagtttgt tttatgtatt 6060

ttagttatag tggcgggggc gcgtcgggtt ttcgggggcg cgtattaaag agataagtta 6120

tggtagtttt tttttttttt tttagcggtt gttatagggg aggtagtggt tttggcggga 6180

tcgggtaggg tgggtagggg gcgtgaattt agtttatatt gattagagtc gttttttggg 6240

ttcgtagttc gtagttgtag tgaaatagaa atattcgagg ttgggacgta agggttttat 6300

atttgtttgg ttttattata tttttgtttt taagtaaagt attatttttc gttg 6354

<210> 3
<211> 6354
<212> DNA
<213> Artificial Sequence

<220>
<223> TFAP2E Sense Bisulfite Sequence non-methylated

<400> 3
gattttggtt tattgtaatt tttgttttgt aggtttaagg gatttttttg ttttagtttt 60

tggagtagtt gggattatag gtgtttgtta ttttatttag ttaatttttg tatttttagt 120

agaaatgggg ttttattata ttggttaggt tggttttgaa ttttagattt taggtgattt 180

atttgttttg gttttttaaa gtgttgggat tatatggtgt gagttattgt tgtttggttt 240

tttttttttt tttttttttt tttttttttt tgagatagtt tttttttgtt gtttaggttg 300

gagtatagtg gtatgatttt ggtttattgt aatttttgtt ttttgggtta agtaattttt 360

ttgttttagt tttttgagtg gttgggatta taggttagtg ttattatatt gggttaattt 420

ttgtattttt agtagagatg gggttttatt attttggtta ggttggtttt gaatttttga 480

ttttaagtga tttggttttt taaagtgttg ggattataga tgtgagttat tgtgtttggt 540

ttatttattt tgattattga gttttttggt atttttttaa attttgtttt agaagttagt 600
attttattta ttttatttat gttttggttg tttagagatt gtttgggaag tggagatttt 660
ttttagtttt tttttttttt tttagggtta aagatagtaa ggaatagggt gattgtttat 720
attagttttt gttttagaga ttttttgaga ttggatagtt gagtttgtgg agagggattt 780
tttattttta agtaggtaat tatttatttt tttttagagg gaatgaggga tttattttag 840
tttttatttt tgattatttt tttttagttt tgattgtata atttgaaatt tatatatggt 900
ttgtaatttt tttttttttt ttttgagatg gagttttgtt ttgtttttag gttggagtgt 960
agtggtgtga ttttggttta ttgtaatttt tgttttttgg gtttaagtga tttttttggt 1020
ttagtttttt tagtagttgg gattatagat gtgtattatt atgtttggtt aatttgtttg 1080
taattttttt aatttgtttg gtttttttat agggagagga tttgtatatg ttgtgttttg 1140
tgtgatgaaa ggagtttttt ttatatattt ttttttttaa tgtatttaga ttttaaagtt 1200
tattatttat ttatttaata aatatttatt aagtattttt tgaatttggt ttaattattt 1260
tttttttagg tatttattta attttgggta ttttttgtgt tttttttgag ttttttttgt 1320
gttttatata gtttttgttt attgttggaa aatatttttg ttaagttttg tttttttatt 1380
agttttgttt gttttttgtg tgtttgggat aggttgtaaa attggaggtg ataaatgtgg 1440
gtaggaaatg gagggttttt ttatattttt agggttggtt ttagttttgt tattttttgt 1500
ttaatattgt ggatgtaatt ggtatgggat ttggaagtgt gtggtaaagt gttggggttt 1560
tgtttggttg ttttttttgg atgtttgttt gtagttagtg aagttttttt aatttaggtt 1620
tgggttttgt gagttttagg tgtttttgtt ttgtggtgtt ggtgaagttg aagtttgaga 1680
atgtttattg tagtgatgtg aaggttgttt ttggggtggg gttgaggttg tagttgtttt 1740
ttttttgtat taaggatttt aatttttagt gatgtagttg ttgtttttgt ttaggttggg 1800
aggtattgta gggatttgat gttttaggtg gttaaagagt gatttttttt gattttaggg 1860
ttttggtggg gtaggtttta gtattgtatt tggtggaggt tgaaggtttg tggggtagga 1920
taggagtttt ttgtgttgtt ggaagggttg aggatgaagg agggtgttaa tttatttttt 1980
attgggttgg tggtaatgtt gaattttgta gtgattgtgg agggttaagg tgaaaattgt 2040
tgggggtgtt gagggtaggt gtggggaggg gtggttttag ggagtaagga gtttatttgt 2100
tttgttgttg tagttgtttt gggttgattg tttatgtttt tttttgggtt atgatttttg 2160
gatttaattg tgtttggttt ttgttttttt ttttttgttg ttgttgtgtg ggttgtaatt 2220
tgatgtttag gttggggtgt ttagggtgta gtttttgttt aggttgttag tgttttattt 2280
gttttattgg gttatagatt tgttggtgtt ggggttttgt tggtgttggg gttttgttgg 2340
tgttggggtt ttgttggtgt tggggttttg ttggtgttgg ggttttgttg gtgttggggt 2400
tttgttggtg ttggggtttt gtggttgtgt atgtgagttt gtgtggtttt gttttgtgtg 2460
gttgtgtatg tgagtttgtg tggttgtgtt ttgttttgtt ttagggagtt agtgtgttgt 2520
tatttgggat gttaggattt ttgttgtgtt ttttggattg ttttggggga ttttggtgta 2580
tttttaggat ttaggagttt tggaagttgt ttgagagaaa ttagttttgg gagggttttg 2640

tatttagttt tttgttttgg ttttggattg gggttttggg ttaaggtgtt tagaggaata 2700
gttgattttt ttatttttgt gtagggtaga gatttttaaa ttttttttta aaatgtaggg 2760
ttttagtttt ttttagggag ttagtgaatt tagattttta gttttttgag tttaagtatg 2820
aatagggaat tggggattat tattatgttt atatttttgg tggttaggaa gtttaggtag 2880
gtttttgttt attgtagatg gatttttttt tttaggggtt aagaaaggtt ttgtatagta 2940
agttaattaa gttttattag tagagttgtg ttgtaattag gtttttagtg ttttttttat 3000
atttttagtt ttaagtgata taggatatag ttttaatttt attttttttt atttattttt 3060
tgttttgttg ttttattttt tttgttatat attttaagtt atttatttaa tttttagttt 3120
ttggtaattt agttttagtt tttggaagtt tagttttagt tttgttatta ttatttttat 3180
tttggttttt tttgagaata agtggagggt aaatagagtt taggtttgaa tttatttgtt 3240
tgaaaaataa tttaagttaa attttgtggg agtagtgttt gtttggggta ttttaggttt 3300
atgtgtattt ttgaattttt ttgggtattt gttttaaatg taaagaggtt gttatttaat 3360
gtttgggttt ttttgatttt ttgttttttt ttggttgttt ttttgttttt gttgtttttt 3420
attttttttt gttatggtta gtatttggtt tttaggttag aaaaggtgga ttttgtgttt 3480
ttggatttta ttaatgttag gagttatata aatattttta tatatatata gagagggttt 3540
gtgtatgttt ggaaattttt agatattaaa gaataaagtg taggatataa tagagatatt 3600
gtatatattg agaaatgtgt atatttttta aatgtagaga aatgtatatt aatatataga 3660
gatatatttt ataaatatat ttatagaaat agatatattt atattttaag aaatattaag 3720
atatttatga agggaatatt ttagatatgt ataggatttt gaatttatat ggatatagat 3780
atttaggagt aggtgggttt tgatttaggt gtatatagtt taatatatag tatttatata 3840
taagtttttt taagtttaaa tgttgtaaga gatttttata gaaagaaaat ttttttagag 3900
gtttttaagg ttttgtttgg aaggaagagg aagaaagtgt ttgttaggta ttgaaatgtt 3960
aaggtattgt aaagtgaaaa ttattttttt aattttgtgg gaatagtatt tttattttta 4020
ttttttaggg aaatagggaa gtggtttaat ttgttttgta aattaatttt agatttataa 4080
gagtgttttt ttttttggtg gggagaggtt aggtttttag tgttgtagtt tagtgaatgt 4140
tgatttgttg aggtttattt taggttatag ggtgttgggt aaatttatta agtttgtatg 4200
tttgtttaat atttggttgg gttaggaaga gtgtttggtg gatatttagt ttggtgatat 4260
ataggaggta tggttggtga agaaaatgga tttgtgtatt aagggtttgt attattttgt 4320
tgggttttgt tttgatagat tattttttat tttttttgat tatggattgg tttaggaggt 4380
ttatttttgg tttgtttatt gtttgtaaat agtagagggt agtggtgagg gtgtataaag 4440
ttttagttgt ttatgttttt ttaagttttt gttatttagg gtttttgttt gttttgttta 4500
aggtgtatta gttttgtttg gggtggtgat gggtaggttt aggaattgtt atgtttaggt 4560
attgagttgg agttttgggt attttattgt gtagtgtaaa aagggaattt tgaattttat 4620
attggtaatt attatgtttt tttttgtttt aggtgtaggt tgttttatgg atgttatatg 4680
tggtagtggg gagaaaagaa gtgatttggt ttttgttgag ggttgttgtt atttatgtat 4740

tgttggtgga tttagtgtta ggtagttttg atggttaggt aatgtttaat ttaggaggtg 4800
ggatatattt ttggagttat tagtgagatg tggtgagttg ggagaaaagt tggttttttg 4860
tatttgttat tttttgtttt tagttatggt agtaaattat tgtggttagt tttgatttat 4920
tttggatttt ttgggtttta tttgtaggtt ttttagattg ggggttgttg ttagttttgg 4980
aaggttgaga ggtgtgtttg ttaggttgtg tgtgttgttg gttaggttgt gtaggagttg 5040
gggtatggta gtggtatagt tatggtgtgg gttaaggttt agggtgtggg tgggtggtgt 5100
tagtaggttg ggaggttttt tgtgggtgtg ggtggttgtg tggggtgtgg tttaggtggt 5160
ttgaggaggt tgtggttgtg ttaggggtgt taggttgtta tatgggtttt ttgttaggtg 5220
gggaaaggtt gtggtggtgt tgggtgtttg attgtagggt agtggtggtt gtgggtaggg 5280
tggtgggaag tagggtggtt ggaatttggt ggtagttgtg gtggttggtg tgtggtagag 5340
tgggggtgtt ggtttgtagg ttgtttgggg tagagatgat aggtgggttt tgttggtagt 5400
tgtttttagt ttgttggggt gtttttgggg aaggagaggg gtgttttagg tgatttattg 5460
gtagttgaaa ggattttagt ttgaaaggat tttttttagt ttgggagggt ttagttgtag 5520
ttttttttgt tttttggggt ttgaggttta tttttgagtt atttgtgggt gtaggagttt 5580
taggttgggg atagtggtta tttgtgtatt gtgtttttag ttggtgaagt ttttgtggtt 5640
tttttttttt gagattttgt tagtgtggga taggagggtt ggaaagttta gtatttaggt 5700
gttttgattt tttggttgaa tatgttttgg gtttgagatt atttattatg gtggagtagg 5760
tgtgtattag tatggggtgg tgagtgttgg gtgtgaggtg tggtggtggt ggagttttag 5820
tttgatggtg agtgttgtgg gtagaggtgt tgggagttgg tatagtttgt ggaggttatg 5880
gtgtggtagt tgggtgtgat ttagttggga ttttagtggg tgtggggttt ttagtggtgg 5940
ttgtttttgg gtgggtttat ggaggtagga tggatgggta ttgattgtat ttggaaaagt 6000
tggttaatgg gtgtttagtg tttgtttttt tttttttgag tgtagtttgt tttatgtatt 6060
ttagttatag tggtgggggt gtgttgggtt tttgggggtg tgtattaaag agataagtta 6120
tggtagtttt tttttttttt tttagtggtt gttatagggg aggtagtggt tttggtggga 6180
ttgggtaggg tgggtagggg gtgtgaattt agtttatatt gattagagtt gttttttggg 6240
tttgtagttt gtagttgtag tgaaatagaa atatttgagg ttgggatgta agggttttat 6300
atttgtttgg ttttattata tttttgtttt taagtaaagt attatttttt gttg 6354

<210> 4
<211> 6354
<212> DNA
<213> Artificial Sequence

<220>
<223> TFAP2E Methylated Antisense Bisulfite Sequence

<400> 4
aatcttaatt cactacaacc tccgccccgc aaattcaaaa aattctccta cctcaacctc 60

cgaaataact aaaattacaa atacctacca ccccacccaa ctaattttta tatttttaat 120

aaaaacgaaa tttcaccata ttaaccaaac taatctcgaa ctccaaacct caaataatcc 180

acccgcctcg acctcccaaa atactaaaat cacataatat aaaccaccgc cgcccgacct 240
cttttttttt tttttttttt tttttttttt taaaataatc tccctctatt atccaaacta 300
aaatacaata acataatctc gactcactac aacctctacc tcctaaatca aacaattctc 360
ttacctcaac ctcctaaata actaaaatta caaactaacg ccaccacact aaactaattt 420
ttatattttt aataaaaata aaatttcacc atcttaacca aactaatctc gaactcttaa 480
cctcaaataa tccgacctcc caaaatacta aaattacaaa cgtaaaccac tacgcccgac 540
ctatttatct taattactaa attttttaac accctctcaa attctatccc aaaaatcaat 600
atctcactca tctcacccat atcctaacta cttaaaaatt atctaaaaaa taaaaaccct 660
ccccaactcc ctcctccttc tccaaaacca aaaacaacaa aaaataaaac gactatttac 720
atcaactcct atcctaaaaa tcccctaaaa ttaaacaact aaacccgtaa aaaaaaattc 780
cccaccccca aataaacaac cacccactct cctccaaaaa aaacgaaaaa tccatctcaa 840
ttctcatcct taaccactcc cctccaactc taattatata acctaaaacc cacacacgat 900
ttataacttt tttttttttt ttccgaaacg aaatctcgct ctatttccaa actaaaatac 960
aataacgcga tcttaactca ctacaatctc cgcctcccga attcaaacga ttcccctaac 1020
tcaacctccc taataactaa aactacaaac gcgcaccacc atacccgact aatttattta 1080
taaccttttt aacctatcta acttttccac aaaaaaaaaa tttacatata ctatatccta 1140
tataataaaa aaaacccttt ttacacaccc cctctttcaa tacactcaaa cttcaaaacc 1200
catcactcat tcattcaaca aacacccact aaacacctct taaacccgat tcaactatct 1260
cccctctaaa cactcaccca acctcgaaca ctctccgcgc ctcctctaaa ctcctcccgc 1320
gtcccataca acccctactc actactaaaa aatattccta ttaaactccg tctccccacc 1380
aatcctactt actttctata tatctaaaat aaattacaaa actaaaaata acaaatataa 1440
acaaaaaata aaaaactccc ttatactcct aaaatcgacc tcaaccctac catttcctac 1500
ccaataccgc gaatacaact aacataaaac ccgaaaatat ataataaaac gccgaaactt 1560
cgcctaaccg cccctcccga acgcttacct acaatcaacg aaacttttcc aacccaaatc 1620
taaaccctac gaactccaaa tacctctacc ccgcgacgct aacgaaatcg aaacccgaaa 1680
acgcccaccg caacgatacg aaaaccgctt ctaaaataaa actaaaacta caactacctc 1740
cctttcgcac caaaaacccc aaccttcaac gacgcaaccg ccgctcccgc ccaaactaaa 1800
aaacactata aaaatccgac gcttcaaata accaaaaaac gactttctct aattccaaaa 1860
ccctaacgaa acaaactcca ataccgcact cgacgaaaac cgaaaatcta taaaacaaaa 1920
caaaaattcc tcgtaccgtc gaaaaaaccg aaaacgaaaa aaaacgctaa ttcactcccc 1980
actaaactaa cgacaacgcc gaatcccgca ataaccgcga aaaaccaaaa taaaaactac 2040
taaaaacgcc gaaaacaaat ataaaaaaaa acgactccaa aaaacaaaaa acccacctac 2100
ctcgccgccg caactaccct aaatcgaccg cccacgcttt cccctaaatc acgacctccg 2160
aatccaacta tacccgactc ctaccccttc ccctttacta ctactacgcg aactataact 2220
taacgtctaa actaaaacgt ccaaaacgca atctccgctc aaatcgccaa cgctccactc 2280

gctccactaa accacaaacc cgctaacgct aaaatcccgc taacgctaaa atcccgctaa 2340
cgctaaaatc ccgctaacgc taaaatcccg ctaacgctaa aatcccgcta acgctaaaat 2400
cccgctaacg ctaaaatccc gcgattatac acgtaaatcc gcgtaacccc gtcccgcgcg 2460
attatacacg taaatccgcg cgaccgcgtc ccgccccgct ccaaaaaacc aacgcgccgc 2520
cacctaaaat attaaaactc tcgttatatt ctttaaacta ctttaaaaaa tttcgacgta 2580
cccctaaaat ctaaaaattt taaaaattat ttaaaaaaaa ccaactctaa aaaaacctcg 2640
catccaatct cctatctcga cttcgaaccg aaattccgaa ttaaaatact caaaaaaaca 2700
actaatcctt ctacccttac gcaaaacaaa aatccccaaa cctctttcta aaatacaaaa 2760
tcttaatctt tctcaaaaaa tcaataaacc taaactccca atctcttaaa cccaaacata 2820
aataaaaaac taaaaaccac caccatactc atatttctaa taaccaaaaa atccaaacaa 2880
acttttaccc actacaaacg aatctctttc ttcaaaaatc aaaaaaaact ctatacaata 2940
aaccaaccaa accccaccaa caaaactacg ctacaaccaa attcctaata tctttcccac 3000
actcccaatt ccaaacgaca caaaacacaa ccccaattcc acctcccccc acccacttcc 3060
taccctatta cctcatcttc cctactacac actccaaatc atccacccaa cccccaactc 3120
ctaataactc aaccccaact cctaaaaatc caaccccaac cctactacca ccacttccac 3180
cttaacctcc cctaaaaaca aataaaaaac aaataaaacc caaacttaaa ttcactcgtt 3240
taaaaaacaa ctcaaaccaa accctataaa aacaatacct acctaaaaca cctcaaactc 3300
atatacacct ctaaattctt tcgaacacct accccaaata taaaaaaact actacccaat 3360
acctaaaccc ctctaactct ctactcctct ctaactatct ctctattttt atcgtctctc 3420
accttcccct atcataacca atacttaatc ctcaaaccaa aaaaaataaa tcccgtatcc 3480
ctaaatttta ctaataccaa aaatcacata aatactccta tatatacaca aaaaaaaccc 3540
gcgcatactt aaaaatcctc aaataccaaa aaacaaaata caaaacacaa caaaaatact 3600
atacatatta aaaaatatat acatccccca aatacaaaaa aatacacacc aacatacaaa 3660
aacacacctc acaaacacat tcacaaaaac aaacacactc acactccaaa aaatactaaa 3720
acacccataa aaaaaacatt tcaaacatac acaaaaccct aaactcacat aaacacaaac 3780
acccaaaaac aaataaaccc taactcaaat acacacaacc taacacacaa tactcacaca 3840
caaactcccc taaatctaaa taccgcaaaa aactcccaca aaaaaaaaat tcccccaaaa 3900
acctccaaaa ccctacctaa aaaaaaaaaa aaaaaaatac ccgccaaaca ccgaaatact 3960
aaaacactat aaaataaaaa tcatttcccc aactccgtaa aaacaacact tccattccca 4020
tctcttaaaa aaacaaaaaa ataatttaac tcgttctaca aatcaactcc aaatccacaa 4080
aaataccctc cccctcgacg aaaaaaaacc aaactcccaa tactacaacc caataaatac 4140
taatccgcta aaacccaccc caaaccacaa aatactaaac aaactcacca aacccgcacg 4200
tctacctaac atctaactaa accaaaaaaa atacctaata aacacccaac ctaacgacac 4260
acaaaaaaca cgaccgataa aaaaaataaa tctatacacc aaaaacctac accatctcgc 4320
cgaactctac cctaacaaac cattccttac ctttcttaat cacgaactaa tctaaaaaac 4380

tcattcccga ctcgtccatt acctacaaat aacaaaaaac aacgataaaa acgcataaaa 4440

ccctaaccgc ccatatcttc ccaaacccct accacccaaa atctctatct actccgccca 4500

aaacgcacca acttcgctta aaacgacgac gaacaaaccc aaaaatcgct atatccaaac 4560

actaaactaa aaccctaaac atctcactat acaatacaaa aaaaaaattt taaatcttac 4620

actaataact attatacttc tcttcgcccc aaatacaaac taccccataa ataccacacg 4680

taacaacgaa aaaaaaaaaa ataattcgat ccctaccgaa aattactacc acccacgcac 4740

tactaacgaa ctcaatacca aacaacctta ataaccaaac aacgtctaac ccaaaaaata 4800

aaacacatcc tcgaaatcac caataaaata taacgaacta aaaaaaaaat cgacttccta 4860

catttatcac cttctacctc caaccataat aataaaccac cgcgaccaat cccgacccat 4920

cccgaatccc tcgaatctca cctacaaatc ctccaaaccg aaaaccgccg ccaaccccga 4980

aaaaccgaaa aatacgtcta ccaaaccgta cgcgccgtcg accaaaccgt acaaaaaccg 5040

aaacacgaca ataacataat cacgacgcga atcaaaaccc aaaacgcgaa cgaacgatac 5100

caacaaaccg aaaaactcct cgtaaacgcg aacgactacg cgaaacgcga cccaaacgac 5160

ctaaaaaaac tacgactacg ccaaaaacgc caaaccgcca tataaatccc ctaccaaata 5220

aaaaaaaact acgacgacgt cgaacgccta accgtaaaac aacgataact acgaataaaa 5280

cgacgaaaaa taaaacgact aaaattcgac gacaactata acgaccgacg tataacaaaa 5340

cgaaaacgcc gacccgtaaa ccgcctaaaa caaaaacgac aaacgaaccc cgccgacaac 5400

tactcccaac ccgtcgaaac gctcctaaaa aaaaaaaaaa ataccttaaa taacccacta 5460

acaactaaaa aaaccccaac ctaaaaaaat cctcctcaac tcgaaaaaat tcaactacaa 5520

tccctcccgt ttcttaaaac ccgaaattta tttccgaacc acccgcgaac gcaaaaacct 5580

caaactaaaa acaacgatca cttacgcatc gcgttctcaa ctaacgaaat ccctacgacc 5640

ccctccctcc gaaatttcgt taacgcgaaa caaaaaaatt aaaaaatcca acactcaaac 5700

gccccgatcc cccgaccgaa tatatcccga acccgaaact actcaccata acgaaataaa 5760

tatacaccaa cataaaacga taaataccga acgcgaaacg taacgacgac gaaaccctaa 5820

cccgacgacg aacgctacga acaaaaacgc cgaaaaccga cacaacccgc gaaaatcacg 5880

atacgataac taaatacgac ccaaccgaaa tcctaataaa cgtaaaatcc tcaacgataa 5940

ccgttcccga acgaatccat aaaaacaaaa cgaacgaaca ctaatcgcac ttaaaaaaat 6000

cgatcaacga atacccaacg cccgcccttc ctcctctaaa cgtaacctac tccatacact 6060

ccaattataa taacgaaaac gcgccgaacc tccgaaaacg cgcattaaaa aaacaaacta 6120

taacaattct ctcccttttc cccaacgact accacaaaaa aaacaataac tttaacgaaa 6180

tcgaataaaa taaataaaaa acgtaaattc aacccacatt aatcaaaacc gccttctaaa 6240

ctcgcaactc gcaactacaa taaaacaaaa atattcgaaa ctaaaacgca aaaaccctac 6300

atttacctaa ctctatcaca tccttacctt taaacaaaac actattcttc gcta 6354

<210> 5
<211> 6354
<212> DNA

<213>  Artificial Sequence

<220>
<223>  TFAP2E Antisense Bisulfite Sequence  non-methylated

<400>  5
aatcttaatt cactacaacc tccaccccac aaattcaaaa aattctccta cctcaacctc      60

caaaataact aaaattacaa atacctacca ccccacccaa ctaattttta tatttttaat     120

aaaaacaaaa tttcaccata ttaaccaaac taatctcaaa ctccaaacct caaataatcc     180

acccacctca acctcccaaa atactaaaat cacataatat aaaccaccac cacccaacct     240

cttttttttt tttttttttt tttttttttt taaaataatc tccctctatt atccaaacta     300

aaatacaata acataatctc aactcactac aacctctacc tcctaaatca aacaattctc     360

ttacctcaac ctcctaaata actaaaatta caaactaaca ccaccacact aaactaattt     420

ttatattttt aataaaaata aaatttcacc atcttaacca aactaatctc aaactcttaa     480

cctcaaataa tccaacctcc caaaatacta aaattacaaa cataaaccac tacacccaac     540

ctatttatct taattactaa attttttaac accctctcaa attctatccc aaaaatcaat     600

atctcactca tctcacccat atcctaacta cttaaaaatt atctaaaaaa taaaaaccct     660

ccccaactcc ctcctccttc tccaaaacca aaaacaacaa aaaataaaac aactatttac     720

atcaactcct atcctaaaaa tcccctaaaa ttaaacaact aaacccataa aaaaaaattc     780

cccaccccca aataaacaac cacccactct cctccaaaaa aaacaaaaaa tccatctcaa     840

ttctcatcct taaccactcc cctccaactc taattatata acctaaaacc cacacacaat     900

ttataacttt tttttttttt ttccaaaaca aaatctcact ctatttccaa actaaaatac     960

aataacacaa tcttaactca ctacaatctc cacctcccaa attcaaacaa ttcccctaac    1020

tcaacctccc taataactaa aactacaaac acacaccacc atacccaact aatttattta    1080

taaccttttt aacctatcta acttttccac aaaaaaaaaa tttacatata ctatatccta    1140

tataataaaa aaaacccttt ttacacaccc cctctttcaa tacactcaaa cttcaaaacc    1200

catcactcat tcattcaaca aacacccact aaacacctct taaacccaat tcaactatct    1260

cccctctaaa cactcaccca acctcaaaca ctctccacac ctcctctaaa ctcctcccac    1320

atcccataca acccctactc actactaaaa aatattccta ttaaactcca tctccccacc    1380

aatcctactt actttctata tatctaaaat aaattacaaa actaaaaata acaaatataa    1440

acaaaaaata aaaaactccc ttatactcct aaaatcaacc tcaaccctac catttcctac    1500

ccaataccac aaatacaact aacataaaac ccaaaaatat ataataaaac accaaaactt    1560

cacctaacca cccctcccaa acacttacct acaatcaaca aaacttttcc aacccaaatc    1620

taaaccctac aaactccaaa tacctctacc ccacaacact aacaaaatca aaacccaaaa    1680

acacccacca caacaataca aaaaccactt ctaaaataaa actaaaacta caactacctc    1740

cctttcacac caaaaacccc aaccttcaac aacacaacca ccactcccac ccaaactaaa    1800

aaacactata aaaatccaac acttcaaata accaaaaaac aactttctct aattccaaaa    1860

ccctaacaaa acaaactcca ataccacact caacaaaaac caaaaatcta taaaacaaaa    1920

caaaaattcc tcataccatc aaaaaaacca aaaacaaaaa aaaacactaa ttcactcccc 1980
actaaactaa caacaacacc aaatcccaca ataaccacaa aaaaccaaaa taaaaactac 2040
taaaaacacc aaaaacaaat ataaaaaaaa acaactccaa aaaacaaaaa acccacctac 2100
ctcaccacca caactaccct aaatcaacca cccacacttt cccctaaatc acaacctcca 2160
aatccaacta tacccaactc ctaccccttc ccctttacta ctactacaca aactataact 2220
taacatctaa actaaaacat ccaaaacaca atctccactc aaatcaccaa cactccactc 2280
actccactaa accacaaacc cactaacact aaaatcccac taacactaaa atcccactaa 2340
cactaaaatc ccactaacac taaaatccca ctaacactaa aatcccacta acactaaaat 2400
cccactaaca ctaaaatccc acaattatac acataaatcc acataacccc atcccacaca 2460
attatacaca taaatccaca caaccacatc ccaccccact ccaaaaaacc aacacaccac 2520
cacctaaaat attaaaactc tcattatatt ctttaaacta ctttaaaaaa tttcaacata 2580
cccctaaaat ctaaaaattt taaaaattat ttaaaaaaaa ccaactctaa aaaaacctca 2640
catccaatct cctatctcaa cttcaaacca aaattccaaa ttaaaatact caaaaaaaca 2700
actaatcctt ctacccttac acaaaacaaa aatccccaaa cctctttcta aaatacaaaa 2760
tcttaatctt tctcaaaaaa tcaataaacc taaactccca atctcttaaa cccaaacata 2820
aataaaaaac taaaaaccac caccatactc atatttctaa taaccaaaaa atccaaacaa 2880
acttttaccc actacaaaca aatctctttc ttcaaaaatc aaaaaaaact ctatacaata 2940
aaccaaccaa accccaccaa caaaactaca ctacaaccaa attcctaata tctttcccac 3000
actcccaatt ccaaacaaca caaaacacaa ccccaattcc acctcccccc acccacttcc 3060
taccctatta cctcatcttc cctactacac actccaaatc atccacccaa cccccaactc 3120
ctaataactc aaccccaact cctaaaaatc caaccccaac cctactacca ccacttccac 3180
cttaacctcc cctaaaaaca aataaaaaac aaataaaacc caaacttaaa ttcactcatt 3240
taaaaaacaa ctcaaaccaa accctataaa aacaatacct acctaaaaca cctcaaactc 3300
atatacacct ctaaattctt tcaaacacct accccaaata taaaaaaact actacccaat 3360
acctaaaccc ctctaactct ctactcctct ctaactatct ctctattttt atcatctctc 3420
accttcccct atcataacca atacttaatc ctcaaaccaa aaaaaataaa tcccatatcc 3480
ctaaatttta ctaataccaa aaatcacata aatactccta tatatacaca aaaaaaaccc 3540
acacatactt aaaaatcctc aaataccaaa aaacaaaata caaaacacaa caaaaatact 3600
atacatatta aaaaatatat acatccccca aatacaaaaa aatacacacc aacatacaaa 3660
aacacacctc acaaacacat tcacaaaaac aaacacactc acactccaaa aaatactaaa 3720
acacccataa aaaaaacatt tcaaacatac acaaaaccct aaactcacat aaacacaaac 3780
acccaaaaac aaataaaccc taactcaaat acacacaacc taacacacaa tactcacaca 3840
caaactcccc taaatctaaa taccacaaaa aactcccaca aaaaaaaaat tcccccaaaa 3900
acctccaaaa ccctacctaa aaaaaaaaaa aaaaaaatac ccaccaaaca ccaaaatact 3960
aaaacactat aaaataaaaa tcatttcccc aactccataa aaacaacact tccattccca 4020

tctcttaaaa aaacaaaaaa ataatttaac tcattctaca aatcaactcc aaatccacaa 4080
aaataccctc cccctcaaca aaaaaaaacc aaactcccaa tactacaacc caataaatac 4140
taatccacta aaacccaccc caaaccacaa aatactaaac aaactcacca aacccacaca 4200
tctacctaac atctaactaa accaaaaaaa atacctaata aacacccaac ctaacaacac 4260
acaaaaaaca caaccaataa aaaaaataaa tctatacacc aaaaacctac accatctcac 4320
caaactctac cctaacaaac cattccttac ctttcttaat cacaaactaa tctaaaaaac 4380
tcattcccaa ctcatccatt acctacaaat aacaaaaaac aacaataaaa acacataaaa 4440
ccctaaccac ccatatcttc ccaaacccct accacccaaa atctctatct actccaccca 4500
aaacacacca acttcactta aaacaacaac aaacaaaccc aaaaatcact atatccaaac 4560
actaaactaa aaccctaaac atctcactat acaatacaaa aaaaaaattt taaatcttac 4620
actaataact attatacttc tcttcacccc aaatacaaac taccccataa ataccacaca 4680
taacaacaaa aaaaaaaaaa ataattcaat ccctaccaaa aattactacc acccacacac 4740
tactaacaaa ctcaatacca aacaacctta ataaccaaac aacatctaac ccaaaaaata 4800
aaacacatcc tcaaaatcac caataaaata taacaaacta aaaaaaaaat caacttccta 4860
catttatcac cttctacctc caaccataat aataaaccac cacaaccaat cccaacccat 4920
cccaaatccc tcaaatctca cctacaaatc ctccaaacca aaaaccacca ccaaccccaa 4980
aaaaccaaaa aatacatcta ccaaaccata cacaccatca accaaaccat acaaaaacca 5040
aaacacaaca ataacataat cacaacacaa atcaaaaccc aaaacacaaa caaacaatac 5100
caacaaacca aaaaactcct cataaacaca aacaactaca caaaacacaa cccaaacaac 5160
ctaaaaaaac tacaactaca ccaaaaacac caaaccacca tataaatccc ctaccaaata 5220
aaaaaaaact acaacaacat caaacaccta accataaaac aacaataact acaaataaaa 5280
caacaaaaaa taaaacaact aaaattcaac aacaactata acaaccaaca tataacaaaa 5340
caaaaacacc aacccataaa ccacctaaaa caaaaacaac aaacaaaccc caccaacaac 5400
tactcccaac ccatcaaaac actcctaaaa aaaaaaaaaa ataccttaaa taacccacta 5460
acaactaaaa aaaccccaac ctaaaaaaat cctcctcaac tcaaaaaaat tcaactacaa 5520
tccctcccat ttcttaaaac ccaaaattta tttccaaacc acccacaaac acaaaaacct 5580
caaactaaaa acaacaatca cttacacatc acattctcaa ctaacaaaat ccctacaacc 5640
ccctccctcc aaaatttcat taacacaaaa caaaaaaatt aaaaaatcca acactcaaac 5700
accccaatcc cccaaccaaa tatatcccaa acccaaaact actcaccata acaaaataaa 5760
tatacaccaa cataaaacaa taaataccaa acacaaaaca taacaacaac aaaaccctaa 5820
cccaacaaca aacactacaa acaaaaacac caaaaaccaa cacaacccac aaaaatcaca 5880
atacaataac taaatacaac ccaaccaaaa tcctaataaa cataaaatcc tcaacaataa 5940
ccattcccaa acaaatccat aaaaacaaaa caaacaaaca ctaatcacac ttaaaaaaat 6000
caatcaacaa atacccaaca cccacccttc ctcctctaaa cataacctac tccatacact 6060
ccaattataa taacaaaaac acaccaaacc tccaaaaaca cacattaaaa aaacaaacta 6120

taacaattct ctcccttttc cccaacaact accacaaaaa aaacaataac tttaacaaaa 6180

tcaaataaaa taaataaaaa acataaattc aacccacatt aatcaaaacc accttctaaa 6240

ctcacaactc acaactacaa taaaacaaaa atattcaaaa ctaaaacaca aaaaccctac 6300

atttacctaa ctctatcaca tccttacctt taaacaaaac actattcttc acta 6354

<210> 6
<211> 1936
<212> DNA
<213> Homo sapiens

<400> 6
taggcaacca cccactctcc tccagaggga acgagggatc catctcagtt ctcatccttg 60

accactcccc tccagctctg attgtataac ctgaaaccca cacacggttt gtaacttttt 120

tttttttttt ccgagacgga gtctcgctct gtttccaggc tggagtgcag tggcgcgatc 180

ttggctcact gcaatctccg cctcccgggt tcaagcgatt cccctggctc agcctcccta 240

gtagctggga ctacagacgc gcaccaccat gcccggctaa tttgtttgta acctttttaa 300

cctgtctggc ttttccacag ggagaggatt tgcatatgct gtgtcctgtg tgatgaaagg 360

agcccttttt acacaccccc tctttcaatg cactcagact tcaaagccca tcactcattc 420

attcaacaaa cacccactaa gcacctcttg aacccggttc aactatctcc cctctaggca 480

ctcacccaac ctcgggcact ctccgcgcct cctctgagct cctcccgcgt cccatacagc 540

ccctgctcac tgctggaaaa tattcctgtt aagctccgtc tccccaccag tcctgcttgc 600

tttctgtgtg tctgggatag gttgcaaaac tggaggtgac aaatgtgggc aggaaatgga 660

gggctccctt atactcctag ggtcggcctc agccctgcca tttcctgccc aataccgcgg 720

atgcaactgg catgggaccc ggaagtgtgt ggtaaagcgc cggggcttcg cctggccgcc 780

cctcccggac gcttgcctgc agtcagcgaa gcttttccaa cccaggtctg ggccctgcga 840

gctccaggtg cctctgcccc gcggcgctgg cgaagtcgaa gcccgagaac gcccaccgca 900

gcgatgcgaa ggccgcttct ggggtggggc tgaggctgca gctgcctccc tttcgcacca 960

aggaccccaa ccttcagcga cgcagccgcc gctcccgccc aggctgggag gcactgtagg 1020

gatccgacgc ttcaggtggc caaagagcga ctttctctga ttccagggcc ctggcggggc 1080

aggctccagt accgcactcg gcggaggccg aaggtctgtg gggcaggaca ggagttcctc 1140

gtgccgtcgg aagggccgag gacgaaggag ggcgctaatt cactccccac tgggctggcg 1200

gcaacgccga atcccgcagt gaccgcggag ggccaaggtg aaaactgctg ggggcgccga 1260

gggcaggtgt ggggaggggc ggctccaggg agcaaggagc ccacctgcct cgccgccgca 1320

gctgccctgg gtcgaccgcc cacgctttcc cctgggtcac gacctccgga tccaactgtg 1380

cccggctcct gccccttccc ctttgctgct gctgcgcggg ctgtaacttg acgtctaggc 1440

tggggcgtcc agggcgcagt ctccgctcag gtcgccagcg ctccactcgc tccactgggc 1500

cacagacccg ctggcgctgg ggtcccgctg gcgctggggt cccgctggcg ctggggtccc 1560

gctggcgctg gggtcccgct ggcgctgggg tcccgctggc gctggggtcc cgctggcgct 1620

ggggtcccgc ggttgtgcac gtgagtccgc gtggccccgt cccgcgcggt tgtgcacgtg 1680

agtccgcgcg gccgcgtccc gccccgctcc agggagccag cgcgccgcca cctgggatgt 1740

taggactctc gttgtgttct ttggactgct ttgggggatt tcggcgtacc cctaggatct 1800

aggagttttg gaagttgttt gagagaaacc agctctggga gggcctcgca tccagtctcc 1860

tgtctcggct tcggaccggg gttccgggtt aaggtgctca gaggaacagc tgatccttct 1920

acccttgcgc agggca 1936

<210> 7
<211> 1936
<212> DNA
<213> Artificial Sequence

<220>
<223> TFAP2E Sense Bisulfite Sequence CpG island 1 (793 nt - 2728
 nt) methylated

<400> 7
taggtaatta tttatttttt tttagaggga acgagggatt tattttagtt tttatttttg 60

attatttttt tttagttttg attgtataat ttgaaattta tatacggttt gtaatttttt 120

tttttttttt tcgagacgga gtttcgtttt gtttttaggt tggagtgtag tggcgcgatt 180

ttggtttatt gtaattttcg tttttcgggt ttaagcgatt tttttggttt agttttttta 240

gtagttggga ttatagacgc gtattattat gttcggttaa tttgtttgta atttttttaa 300

tttgtttggt ttttttatag ggagaggatt tgtatatgtt gtgttttgtg tgatgaaagg 360

agtttttttt atatattttt ttttttaatg tatttagatt ttaaagttta ttatttattt 420

atttaataaa tatttattaa gtattttttg aattcggttt aattattttt tttttaggta 480

tttatttaat ttcgggtatt tttcgcgttt tttttgagtt tttttcgcgt tttatatagt 540

ttttgtttat tgttggaaaa tatttttgtt aagtttcgtt tttttattag ttttgtttgt 600

tttttgtgtg tttgggatag gttgtaaaat tggaggtgat aaatgtgggt aggaaatgga 660

gggttttttt atatttttag ggtcggtttt agttttgtta ttttttgttt aatatcgcgg 720

atgtaattgg tatgggattc ggaagtgtgt ggtaaagcgt cggggtttcg tttggtcgtt 780

tttttcggac gtttgtttgt agttagcgaa gtttttttaa tttaggtttg ggttttgcga 840

gttttaggtg tttttgtttc gcggcgttgg cgaagtcgaa gttcgagaac gtttatcgta 900

gcgatgcgaa ggtcgttttt ggggtggggt tgaggttgta gttgtttttt tttcgtatta 960

aggattttaa tttttagcga cgtagtcgtc gttttcgttt aggttgggag gtattgtagg 1020

gattcgacgt tttaggtggt taaagagcga ttttttttga ttttagggtt ttggcggggt 1080

aggttttagt atcgtattcg gcggaggtcg aaggtttgtg gggtaggata ggagtttttc 1140

gtgtcgtcgg aagggtcgag gacgaaggag ggcgttaatt tattttttat tgggttggcg 1200

gtaacgtcga atttcgtagt gatcgcggag ggttaaggtg aaaattgttg ggggcgtcga 1260

gggtaggtgt ggggaggggc ggttttaggg agtaaggagt ttatttgttt cgtcgtcgta 1320

gttgttttgg gtcgatcgtt tacgtttttt tttgggttac gattttcgga tttaattgtg 1380

ttcggttttt gttttttttt ttttgttgtt gttgcgcggg ttgtaatttg acgtttaggt 1440

tggggcgttt agggcgtagt tttcgtttag gtcgttagcg ttttattcgt tttattgggt 1500

tatagattcg ttggcgttgg ggtttcgttg gcgttggggt ttcgttggcg ttggggtttc 1560

gttggcgttg gggtttcgtt ggcgttgggg tttcgttggc gttggggttt cgttggcgtt 1620

ggggtttcgc ggttgtgtac gtgagttcgc gtggtttcgt ttcgcgcggt tgtgtacgtg 1680

agttcgcgcg gtcgcgtttc gtttcgtttt agggagttag cgcgtcgtta tttgggatgt 1740

taggattttc gttgtgtttt ttggattgtt ttgggggatt tcggcgtatt tttaggattt 1800

aggagttttg gaagttgttt gagagaaatt agttttggga gggtttcgta tttagttttt 1860

tgtttcggtt tcggatcggg gtttcgggtt aaggtgttta gaggaatagt tgattttttt 1920

atttttgcgt agggta 1936

<210> 8
<211> 1936
<212> DNA
<213> Artificial Sequence

<220>
<223> TFAP2E Sense Bisulfite Sequence CpG island 1 (793 nt - 2728
 nt) non-methylated

<400> 8
taggtaatta tttatttttt tttagaggga atgagggatt tattttagtt tttatttttg 60

attatttttt tttagttttg attgtataat ttgaaattta tatatggttt gtaatttttt 120

tttttttttt ttgagatgga gttttgtttt gtttttaggt tggagtgtag tggtgtgatt 180

ttggtttatt gtaatttttg ttttttgggt ttaagtgatt tttttggttt agttttttta 240

gtagttggga ttatagatgt gtattattat gtttggttaa tttgtttgta atttttttaa 300

tttgtttggt ttttttatag ggagaggatt tgtatatgtt gtgttttgtg tgatgaaagg 360

agtttttttt atatattttt ttttttaatg tatttagatt ttaaagttta ttatttattt 420

atttaataaa tatttattaa gtattttttg aatttggttt aattattttt tttttaggta 480

tttatttaat tttgggtatt ttttgtgttt tttttgagtt ttttttgtgt tttatatagt 540

ttttgtttat tgttggaaaa tatttttgtt aagttttgtt tttttattag ttttgtttgt 600

tttttgtgtg tttgggatag gttgtaaaat tggaggtgat aaatgtgggt aggaaatgga 660

gggttttttt atatttttag ggttggtttt agttttgtta ttttttgttt aatattgtgg 720

atgtaattgg tatgggattt ggaagtgtgt ggtaaagtgt tggggttttg tttggttgtt 780

ttttttggat gtttgtttgt agttagtgaa gtttttttaa tttaggtttg ggttttgtga 840

gttttaggtg tttttgtttt gtggtgttgg tgaagttgaa gtttgagaat gtttattgta 900

gtgatgtgaa ggttgttttt ggggtggggt tgaggttgta gttgtttttt ttttgtatta 960

aggattttaa tttttagtga tgtagttgtt gtttttgttt aggttgggag gtattgtagg 1020

gatttgatgt tttaggtggt taaagagtga ttttttttga ttttagggtt ttggtggggt 1080

aggttttagt attgtatttg gtggaggttg aaggtttgtg gggtaggata ggagtttttt 1140

gtgttgttgg aagggttgag gatgaaggag ggtgttaatt tattttttat tgggttggtg 1200

gtaatgttga attttgtagt gattgtggag ggttaaggtg aaaattgttg ggggtgttga 1260

gggtaggtgt ggggaggggt ggttttaggg agtaaggagt ttatttgttt tgttgttgta 1320

gttgttttgg gttgattgtt tatgtttttt tttgggttat gatttttgga tttaattgtg 1380

tttggttttt gttttttttt ttttgttgtt gttgtgtggg ttgtaatttg atgtttaggt 1440

tggggtgttt agggtgtagt ttttgtttag gttgttagtg ttttatttgt tttattgggt 1500

tatagatttg ttggtgttgg ggttttgttg gtgttggggt tttgttggtg ttggggtttt 1560

gttggtgttg gggttttgtt ggtgttgggg ttttgttggt gttggggttt tgttggtgtt 1620

ggggttttgt ggttgtgtat gtgagtttgt gtggttttgt tttgtgtggt tgtgtatgtg 1680

agtttgtgtg gttgtgtttt gttttgtttt agggagttag tgtgttgtta tttgggatgt 1740

taggattttt gttgtgtttt ttggattgtt ttgggggatt ttggtgtatt tttaggattt 1800

aggagttttg gaagttgttt gagagaaatt agttttggga gggttttgta tttagttttt 1860

tgttttggtt ttggattggg gttttgggtt aaggtgttta gaggaatagt tgattttttt 1920

atttttgtgt agggta 1936

<210> 9
<211> 1936
<212> DNA
<213> Artificial Sequence

<220>
<223> TFAP2E Antisense Bisulfite Sequence CpG island 1 (793 nt -
 2728 nt) methylated

<400> 9
taaacaacca cccactctcc tccaaaaaaa acgaaaaatc catctcaatt ctcatcctta 60

accactcccc tccaactcta attatataac ctaaaaccca cacacgattt ataacttttt 120

tttttttttt ccgaaacgaa atctcgctct atttccaaac taaaatacaa taacgcgatc 180

ttaactcact acaatctccg cctcccgaat tcaaacgatt cccctaactc aacctcccta 240

ataactaaaa ctacaaacgc gcaccaccat acccgactaa tttatttata acctttttaa 300

cctatctaac ttttccacaa aaaaaaaatt tacatatact atatcctata taataaaaaa 360

aacccttttt acacaccccc tctttcaata cactcaaact tcaaaaccca tcactcattc 420

attcaacaaa cacccactaa acacctctta aacccgattc aactatctcc cctctaaaca 480

ctcacccaac ctcgaacact ctccgcgcct cctctaaact cctcccgcgt cccatacaac 540

ccctactcac tactaaaaaa tattcctatt aaactccgtc tccccaccaa tcctacttac 600

tttctatata tctaaaataa attacaaaac taaaaataac aaatataaac aaaaaataaa 660

aaactccctt atactcctaa aatcgacctc aaccctacca tttcctaccc aataccgcga 720

atacaactaa cataaaaccc gaaaatatat aataaaacgc cgaaacttcg cctaaccgcc 780

cctcccgaac gcttacctac aatcaacgaa acttttccaa cccaaatcta aaccctacga 840

actccaaata cctctacccc gcgacgctaa cgaaatcgaa acccgaaaac gcccaccgca 900

acgatacgaa aaccgcttct aaaataaaac taaaactaca actacctccc tttcgcacca 960

aaaaccccaa ccttcaacga cgcaaccgcc gctcccgccc aaactaaaaa acactataaa 1020

aatccgacgc ttcaaataac caaaaaacga ctttctctaa ttccaaaacc ctaacgaaac 1080

aaactccaat accgcactcg acgaaaaccg aaaatctata aaacaaaaca aaaattcctc 1140

gtaccgtcga aaaaaccgaa aacgaaaaaa aacgctaatt cactccccac taaactaacg 1200

acaacgccga atcccgcaat aaccgcgaaa aaccaaaata aaaactacta aaaacgccga 1260

aaacaaatat aaaaaaaaac gactccaaaa aacaaaaaac ccacctacct cgccgccgca 1320

actaccctaa atcgaccgcc cacgctttcc cctaaatcac gacctccgaa tccaactata 1380

cccgactcct accccttccc ctttactact actacgcgaa ctataactta acgtctaaac 1440

taaaacgtcc aaaacgcaat ctccgctcaa atcgccaacg ctccactcgc tccactaaac 1500

cacaaacccg ctaacgctaa aatcccgcta acgctaaaat cccgctaacg ctaaaatccc 1560

gctaacgcta aaatcccgct aacgctaaaa tcccgctaac gctaaaatcc cgctaacgct 1620

aaaatcccgc gattatacac gtaaatccgc gtaaccccgt cccgcgcgat tatacacgta 1680

aatccgcgcg accgcgtccc gccccgctcc aaaaaaccaa cgcgccgcca cctaaaatat 1740

taaaactctc gttatattct ttaaactact ttaaaaaatt tcgacgtacc cctaaaatct 1800

aaaaatttta aaaattattt aaaaaaaacc aactctaaaa aaacctcgca tccaatctcc 1860

tatctcgact tcgaaccgaa attccgaatt aaaatactca aaaaaacaac taatccttct 1920

acccttacgc aaaaca 1936

<210> 10
<211> 1936
<212> DNA
<213> Artificial Sequence

<220>
<223> TFAP2E Antisense Bisulfite Sequence CpG island 1 (793 nt -
 2728 nt) non-methylated

<400> 10
taaacaacca cccactctcc tccaaaaaaa acaaaaaatc catctcaatt ctcatcctta 60

accactcccc tccaactcta attatataac ctaaaaccca cacacaattt ataacttttt 120

tttttttttt ccaaaacaaa atctcactct atttccaaac taaaatacaa taacacaatc 180

ttaactcact acaatctcca cctcccaaat tcaaacaatt cccctaactc aacctcccta 240

ataactaaaa ctacaaacac acaccaccat acccaactaa tttatttata acctttttaa 300

cctatctaac ttttccacaa aaaaaaaatt tacatatact atatcctata taataaaaaa 360

aacccttttt acacaccccc tctttcaata cactcaaact tcaaaaccca tcactcattc 420

attcaacaaa cacccactaa acacctctta aacccaattc aactatctcc cctctaaaca 480

ctcacccaac ctcaaacact ctccacacct cctctaaact cctcccacat cccatacaac 540

ccctactcac tactaaaaaa tattcctatt aaactccatc tccccaccaa tcctacttac 600

tttctatata tctaaaataa attacaaaac taaaaataac aaatataaac aaaaaataaa 660

aaactccctt atactcctaa aatcaacctc aaccctacca tttcctaccc aataccacaa 720

atacaactaa cataaaaccc aaaaatatat aataaaacac caaaacttca cctaaccacc 780

cctcccaaac acttacctac aatcaacaaa acttttccaa cccaaatcta aaccctacaa 840

actccaaata cctctacccc acaacactaa caaaatcaaa acccaaaaac acccaccaca 900

acaatacaaa aaccacttct aaaataaaac taaaactaca actacctccc tttcacacca 960

aaaaccccaa ccttcaacaa cacaaccacc actcccaccc aaactaaaaa acactataaa 1020

aatccaacac ttcaaataac caaaaaacaa ctttctctaa ttccaaaacc ctaacaaaac 1080

aaactccaat accacactca acaaaaacca aaaatctata aaacaaaaca aaaattcctc 1140

ataccatcaa aaaaaccaaa aacaaaaaaa aacactaatt cactccccac taaactaaca 1200

acaacaccaa atcccacaat aaccacaaaa aaccaaaata aaaactacta aaaacaccaa 1260

aaacaaatat aaaaaaaaac aactccaaaa aacaaaaaac ccacctacct caccaccaca 1320

actaccctaa atcaaccacc cacactttcc cctaaatcac aacctccaaa tccaactata 1380

cccaactcct accccttccc ctttactact actacacaaa ctataactta acatctaaac 1440

taaaacatcc aaaacacaat ctccactcaa atcaccaaca ctccactcac tccactaaac 1500

cacaaaccca ctaacactaa aatcccacta acactaaaat cccactaaca ctaaaatccc 1560

actaacacta aaatcccact aacactaaaa tcccactaac actaaaatcc cactaacact 1620

aaaatcccac aattatacac ataaatccac ataaccccat cccacacaat tatacacata 1680

aatccacaca accacatccc accccactcc aaaaaaccaa cacaccacca cctaaaatat 1740

taaaactctc attatattct ttaaactact ttaaaaaatt tcaacatacc cctaaaatct 1800

aaaaatttta aaaattattt aaaaaaaacc aactctaaaa aaacctcaca tccaatctcc 1860

tatctcaact tcaaaccaaa attccaaatt aaaatactca aaaaaacaac taatccttct 1920

acccttacac aaaaca 1936

<210> 11
<211> 139
<212> DNA
<213> Homo sapiens

<400> 11
ccggacgctt gcctgcagtc agcgaagctt ttccaaccca ggtctgggcc ctgcgagctc 60

caggtgcctc tgccccgcgg cgctggcgaa gtcgaagccc gagaacgccc accgcagcga 120

tgcgaaggcc gcttctggg 139

<210> 12
<211> 139
<212> DNA
<213> Artificial Sequence

<220>
<223> TFAP2E MSP amplificate region Bisulfite Sense (1577 nt - 1717 nt)
      methylated

<400> 12
tcggacgttt gtttgtagtt agcgaagttt ttttaattta ggtttgggtt ttgcgagttt 60

taggtgtttt tgtttcgcgg cgttggcgaa gtcgaagttc gagaacgttt atcgtagcga 120

tgcgaaggtc gtttttggg 139

<210>  13
<211>  139
<212>  DNA
<213>  Artificial Sequence

<220>
<223>  TFAP2E MSP amplificate region Bisulfite Sense (1577 nt - 1717 nt)
       non-methylated

<400>  13
ttggatgttt gtttgtagtt agtgaagttt ttttaattta ggtttgggtt ttgtgagttt       60

taggtgtttt tgttttgtgg tgttggtgaa gttgaagttt gagaatgttt attgtagtga      120

tgtgaaggtt gtttttggg                                                   139


<210>  14
<211>  139
<212>  DNA
<213>  Artificial Sequence

<220>
<223>  TFAP2E MSP amplificate region Bisulfite Antisense (1577 nt - 1717
       nt) methylated

<400>  14
ccgaacgctt acctacaatc aacgaaactt ttccaaccca aatctaaacc ctacgaactc       60

caaatacctc taccccgcga cgctaacgaa atcgaaaccc gaaaacgccc accgcaacga      120

tacgaaaacc gcttctaaa                                                   139


<210>  15
<211>  139
<212>  DNA
<213>  Artificial Sequence

<220>
<223>  TFAP2E MSP amplificate region Bisulfite Antisense (1577 nt - 1717
       nt) non-methylated

<400>  15
ccaaacactt acctacaatc aacaaaactt ttccaaccca aatctaaacc ctacaaactc       60

caaatacctc taccccacaa cactaacaaa atcaaaaccc aaaaacaccc accacaacaa      120

tacaaaaacc acttctaaa                                                   139


<210>  16
<211>  5507
<212>  DNA
<213>  Homo sapiens

<400>  16
gagtagctgg gattacagac atctgccacc atgcccagct aattttttgt attttttagt       60

agagacaggg ttttacagtg ttggccagtc tggtctcaaa ctcttgacct cgtgatccac      120

ccacctctgc ctcccaaagt gctgggatta caggcgtgag ccaccatgcc tggcttcaat      180

tcttaaagat ttgaattaat tgagtcccta gattagaaat aatttttttg gttacagagt      240

cccatcagtt ttctggaaaa acggacaaaa ggctaaagct agtttcattc atcacaaggt      300

cgccatctct tagttctcta gtttcaggct atttcacttg ctacaagatt tgattttaag      360

agagatctgt atggaagcat tttaaagtta atgtccaaga ttgagctcaa atgcaatgtg      420

gattcttctt tattttgaaa tatttatagc ttttctattt tttggcatac tcttaatcga 480
gcatgctgcc gattgctggt caattggctt cgacacagta gtccagggcc acagtcgcaa 540
cgctggaaga tttctggagc ttgagcagtg tctttatgcc ctcttctgga gcagacctgt 600
ccctccaaaa ggactggctt acaaattttc gtccaaaaat gacgagcaca gcaaagtcca 660
gggccacagt caaaagttct cagacaactt tctccctctt gtcctgtaac aaggttaatg 720
tgggctttag tgataaataa ggaaagtggc ctgctggggg tttgctactg ggataaggga 780
atgggaccta tgactgacag ccacaaagta aaaacagaca cagcacgcag gtctcacaga 840
gctaggtgtt acgctaaaag cagtaatgcc atggcgactc cacccctacc catcagaatt 900
gatcgttgtt actggctgca gactacctta ggctactctc tacgtgaccc ccacagcttc 960
ttacagaaag agataacaaa cagcacatgc ttcctaagtg tttctgggat ggagaagaat 1020
aaaatcagtc tcaccctatc cttaagaggc tgggaggaga aaatgcattt ctgaaataaa 1080
atatgctgcg aatgctgttc ttacccttcc tgccttgtga tttcttaata cttggcttcc 1140
ttttgggttg gttttcctgg actgggtgcc cagttgttcc ttctgcatgt gtgccatctt 1200
gctcatcaag ctgcctttct aatattgggg ttgcatcttc catcgtagta caaacatctg 1260
agggacaacc aggtgtaact agaattatta acttcaaggg ggagaaccac agacacacta 1320
attatcctac tgttgcatag tttggcagca cctctgcctt cagtttatat ataactttct 1380
agatttctgg aacatgtgct aaatatttac atgctcgcat gtggcagaga aagtagctaa 1440
gtggaaattg cattccatta aaggtagtta atggtatcca agtggaaatg gaagtatttg 1500
aatgattaca acttgagtct ttcctttaaa atcattatct ccaagtcatt ggcaagagct 1560
gttgccagga aataaattat tttgtttcat taagaaaagg caagccgggt ccggtggctt 1620
acgcttgtaa tcccagcact ttgggaggcc gaggtaggtg gatcacctga ggtcagaagt 1680
tcgagactag cctgaccaac atggtgaaac cctatctcta ctaaaaataa caaaaattag 1740
ccgggcatgg tggtacatgc ctgtagtcct agttactcca gaggctgagg caggagaacc 1800
acctgaaccc ggaggccgag gctgcagtga gctgtggaca acaagggcga aactccgcct 1860
cagaaaaaaa aaaaaaagga aaagaaaagg cagctgcata catttacttc aagcatgaat 1920
ctaggaagca ccattagaga gtgcctaccc tggaatattc tagtctatgg ggcttagaca 1980
ctaccttcgg tttaaaaacc actgttggcg ttatgtcgct caccgttcac acagagtgtc 2040
ccagggcagc acatggcatc tcgctggcac ctcctccgca acccacgaca tgtagcacag 2100
aacggcttct catcgcgggg ctggaggcag aactttctgg tattgcagtc cgtgtcagac 2160
aggcactgtg agccctgtgg agggaatctg ttatcacaag gctaggaaac ccccaacacg 2220
atggaagatg acttattatt gaaggcagga ggcggaggga gccaaaggac agtggcgctg 2280
tgacaaaccc tgggcagcat cttactctcc agtggaaatg gttttctccc acaaagggaa 2340
gctcatgtca cccggggaga cctaggcgct gcaaaggcta tttgagtttg aaaaattgac 2400
ttaactcaga atttagtttt cctgtgctct ttgtgaaaat gggaaaaaat gttttcctat 2460
gtgtgtctta acaggtgagt atgagatttt caatatatct taactctaga agctggctac 2520

agtttcatta aacatactat tgatgtttgt ggcattccta tttgattttt tactctagtg 2580
aatcacattt agcttagaga cccttcccta agtgaactgt cctttgattt tcaatgatgc 2640
ctcattgaca tagagaaaga gataccgaag atcaattgtt ctcatacaca gttgatgctt 2700
ctacatagac atacaacgta ggtgtagcat atatgatata tgtaataatc tatacgatat 2760
ataataacat atacacatac ataaagcgat tcctaaaaca actctctctg tgaagttaga 2820
tggtgacaag gcaatgcatc atggccctct gtttaaattc tcattcccat gtttgcttgc 2880
tggctgtgtg agcttgggta agctcttcaa catctctgag tcttatttcc tcatctggaa 2940
ttagggaatg tttcacctat ctcaaattgt gaggaagagt acatgacgta ttacatgtaa 3000
aagagctggc acatagtaga gcttccaaag tgttcgttta tttcgtgatt tatagacaaa 3060
cgtatggccg aatgatgtga aaagccactg tgtttatttc atacattcac actaaggcca 3120
ctgatcctac caggatgccc tcccaaatcc gaaaccaatt caatttacct gctaggtaag 3180
acatttcata catgaatcag aatgacttac taaagcctaa acaaaacacc ccagctgtca 3240
gcagtcccgt acctcgcccc cctccctagc agccttacct tccgggcccc atgcaggtca 3300
gcagagctcc tgatgttgtt gaagtccagg accagagctc ccaggggaga gcagagccag 3360
ctcagcccca gcaggacggc cgccaccatc cttcaatccc ggggctcctg gagggtccca 3420
gcactgtgcg tcaccaaagc gaggctgctc tccacccaga gcagagcttc cactaagctg 3480
gcagctcagc acgtcgtctg tttgtcactg ctttttctga aagaagtaaa ccttagtctg 3540
agtaaggtgc gtgaaatcgg ctgagcaaag tctgaccagc aggttcctcc tgaaactatt 3600
tataaccaga tgtgcctcct ccccgctccg ttccttcctc ctgccccaca caggcttcaa 3660
caaatccctt caaatctgtt tgatcaatag ttcaaaacaa ggactcaata gaaggagaga 3720
agaggcaggg ccatgcggct tctcctccag gggttcaatc cctcttaatt attgcatatc 3780
aaagttagtt caaagggcca cggcagagca ggatgtctgt atcaaaggca aactctgata 3840
atcacctgca tttcctacct tcagtaatcc tttgggggta gggagggagt ctgccctaag 3900
atctgttttc acagctaatt gtgctcaaag aaaggatgaa tcatactggc caatatgcta 3960
tagaggatgc caaaaactaa tgaggtacaa aatacactga gaagctgtaa actgggcttc 4020
agatagtgag gacagataga aaaagagaaa acgccccctg attagctctg gcatccagat 4080
gtcaaaatcc caaccgctta aaagttgtga acgtaacagc acctgttctg actaaatgcc 4140
atttgggaaa ttactttcct ttaaatgaaa attgaactgt ttgaagttag ggttattagg 4200
aaaataatgt aatttatgaa aatcccattt ttcaatggtg ggaacaatga tagcgcattt 4260
tacacaattc agtacgaagc tttgggtcca ttttatcagc aacatattta atttgtgttg 4320
cattatgtaa aactcactta agcagaacaa ggatccaagt ctctggctga tttcagatga 4380
aatttcatgt atttccaatt ttcctaatac gaatttttta aaacttctgc ttcatccctt 4440
tcctgtctga tttttttcca tttggactat tagtcaggta atttagacat gtgcttatca 4500
ctgtcttaaa tactttaagg gtagaacaat ctagatctgg atcaaaaaat cattttttct 4560

cttcattgtt tcattgctct tagcatagag tcatctaaaa tgcatttttt ctatgccata 4620

aatgataatc ttattattta ctacattttc ctgcagaaca acctaacatc aattcattga 4680

catgaatctc agcattctgt gcatgggaac attctcaatt ttgagtaatg cagtgccatt 4740

ccgagggtga tgcaggtgat tcgagccttc ctactacttg gcccttgtcc ttgggttggt 4800

attgcttgtc tacctctcgt atggaagtta cttgttttaa tcccttaaac tgacatcaaa 4860

caacaccctt tgatagcatg ataaccctgg tgactagggg tgttacagaa tttattttca 4920

agttgtacaa ttatgaccaa ggataaagga aagagccccc agggttctgt ctctgccggg 4980

tttctctgct tcctgctgcc tcactgtaag ggaatttcag gttaccaaag gcccctggca 5040

atatacacag gcgaaagaac tgacgggaac tcctgtaaca cggcacaaac agccactgag 5100

cacttagagc acttggcatg gaacagagcc tcggattaca tgttaggaat aaaatttgaa 5160

actccaatgg ccgggcacag tggctcacac ctgtaatccc agcactttgg gaggccaagg 5220

tgggaagatc tcctgagctc aggagttcaa gacgagcctg ggcaacatat tgagatcctg 5280

tctccacaaa aaaaaaaaaa aaaaaaaaag taggccgcgt gcggtggctg acgcctgtaa 5340

tctcaccact ttgggaggct gaggtggatg gatcacctga ggtcaggagt tcgagaccag 5400

cctggccaat atggtgaaac cctgtctcta ctaaaaatat aaaaactagt cgggcgtggt 5460

ggtggtcacc tgtaatccca gctactcggg aggctgaggc aggagaa 5507

<210> 17
<211> 20
<212> DNA
<213> Artificial Sequence

<220>
<223> MSP_genomic_for

<400> 17
ccggacgctt gcctgcagtc 20

<210> 18
<211> 21
<212> DNA
<213> Artificial Sequence

<220>
<223> MSP_genomic_rev

<400> 18
cccagaagcg gccttcgcat c 21

<210> 19
<211> 22
<212> DNA
<213> Artificial Sequence

<220>
<223> MSP_genomic_probe

<400> 19
agcccgagaa cgcccaccgc ag 22

<210> 20

<211>  20
<212>  DNA
<213>  Artificial Sequence

<220>
<223>  MSP_sense_bis_for

<400>  20
tcggacgttt gtttgtagtt                                                    20


<210>  21
<211>  21
<212>  DNA
<213>  Artificial Sequence

<220>
<223>  MSP_sense_bis_rev

<400>  21
cccaaaaacg accttcgcat c                                                  21


<210>  22
<211>  22
<212>  DNA
<213>  Artificial Sequence

<220>
<223>  MSP_sense_bis_probe

<400>  22
agttcgagaa cgtttatcgt ag                                                 22


<210>  23
<211>  20
<212>  DNA
<213>  Artificial Sequence

<220>
<223>  MSP_antisense_rev

<400>  23
ccgaacgctt acctacaatc                                                    20


<210>  24
<211>  21
<212>  DNA
<213>  Artificial Sequence

<220>
<223>  MSP_antisense_for

<400>  24
tttagaagcg gttttcgtat c                                                  21


<210>  25
<211>  22
<212>  DNA
<213>  Artificial Sequence

<220>
<223>  MSP_antisense_probe

<400>  25
ttgcggtggg cgttttcggg tt                                                 22

<210>  26
<211>  24
<212>  DNA
<213>  Artificial Sequence

<220>
<223>  BSP_sense_bis_for

<400>  26
   tagtttgaaa ggattttttt tagt                                              24


<210>  27
<211>  26
<212>  DNA
<213>  Artificial Sequence

<220>
<223>  BSP_sense_bis_rev

<400>  27
actctaatca atataaacta aattca                                           26


<210>  28
<211>  24
<212>  DNA
<213>  Artificial Sequence

<220>
<223>  Taenzer_BSP_antisense_bis_for

<400>  28
caacctaaaa aaatcctcct caac                                             24


<210>  29
<211>  26
<212>  DNA
<213>  Artificial Sequence

<220>
<223>  BSP_antisense_bis_rev

<400>  29
gttttgatta atgtgggttg aattta                                           26


<210>  30
<211>  24
<212>  DNA
<213>  Artificial Sequence

<220>
<223>  BSP_genomic_for

<400>  30
cagcctgaaa ggatcctcct cagc                                             24


<210>  31
<211>  26
<212>  DNA
<213>  Artificial Sequence

<220>
<223>  BSP_genomic_rev

<400>  31

gctctgatca atgtgggctg aattca                                          26

<210>  32
<211>  24
<212>  DNA
<213>  Artificial Sequence

<220>
<223>  DKK Primer forward 206bp

<400>  32
atattagaaa ggcagcttga tgag                                            24

<210>  33
<211>  25
<212>  DNA
<213>  Artificial Sequence

<220>
<223>  DKK4 Reverse Primer bp 406-587

<400>  33
ttacaaattt tcgtccaaaa atgac                                           25

<210>  34
<211>  24
<212>  DNA
<213>  Artificial Sequence

<220>
<223>  DKK4 Forward primer 1kb

<400>  34
gaaagggatg aagcagaagt ttta                                            24

<210>  35
<211>  23
<212>  DNA
<213>  Artificial Sequence

<220>
<223>  DKK4 primer Reverse 2 put. AP2 BS

<400>  35
gtcgtctgtt tgtcactgct ttt                                             23

<210>  36
<211>  20
<212>  DNA
<213>  Artificial Sequence

<220>
<223>  DKK4 primer Forward 2kb

<400>  36
ctcccaaagt gctgggatta                                                 20

<210>  37
<211>  20
<212>  DNA
<213>  Artificial Sequence

<220>
<223>  DKK4 primer Reverse 4 put. AP2 BS

<400>  37
gcacgtcgtc tgtttgtcac                                                   20


<210>  38
<211>  20
<212>  DNA
<213>  Artificial Sequence

<220>
<223>  DKK4 primer Forward 500bp

<400>  38
tttaagcggt tgggattttg                                                   20


<210>  39
<211>  20
<212>  DNA
<213>  Artificial Sequence

<220>
<223>  DKK4 primer Reverse 0 put. AP2 BS

<400>  39
taaccagatg tgcctcctcc                                                   20


<210>  40
<211>  20
<212>  DNA
<213>  Artificial Sequence

<220>
<223>  DKK4 primer Forward 114bp

<400>  40
ttcgcctgtg tatattgcca                                                   20


<210>  41
<211>  20
<212>  DNA
<213>  Artificial Sequence

<220>
<223>  DKK4 primer Reverse 2 put. AP2 BS ChIP 1

<400>  41
gataaaggaa agagccccca                                                   20


<210>  42
<211>  20
<212>  DNA
<213>  Artificial Sequence

<220>
<223>  DKK4 primer Forward 300bp

<400>  42
taagcggttg ggattttgac                                                   20


<210>  43
<211>  20
<212>  DNA
<213>  Artificial Sequence

<220>
<223>  DKK4 primer Reverse 2 put. AP2 BS chip2

<400>  43
ggcagagcag gatgtctgta                                                   20


<210>  44
<211>  25
<212>  DNA
<213>  Artificial Sequence

<220>
<223>  TFAP2E primer Forward 310bp

<400>  44
tagaccagtc cgtgatcaag aaagt                                             25


<210>  45
<211>  24
<212>  DNA
<213>  Artificial Sequence

<220>
<223>  TFAP2E primer Reverse bp 747-1056

<400>  45
aggttgagcc caatcttctc taac                                              24


<210>  46
<211>  19
<212>  DNA
<213>  Artificial Sequence

<220>
<223>  TFAP2E primer Forward 1331bp

<400>  46
cacctactcc gccatggag                                                    19


<210>  47
<211>  22
<212>  DNA
<213>  Artificial Sequence

<220>
<223>  TFAP2E primer Reverse CDS

<400>  47
gtgggagaag cagttatttc cg                                                22


<210>  48
<211>  25
<212>  DNA
<213>  Artificial Sequence

<220>
<223>  ACTB Primer Forward 132bp

<400>  48
tggtgatgga ggaggtttag taagt                                             25


<210>  49
<211>  27
<212>  DNA

<213>  Artificial Sequence

<220>
<223>  ACTB reverse primer

<400>  49
aaccaataaa acctactcct cccttaa                                          27


<210>  50
<211>  30
<212>  DNA
<213>  Artificial Sequence

<220>
<223>  ACTB Probe

<400>  50
accaccaccc aacacacaat aacaaacaca                                       30


<210>  51
<211>  22
<212>  DNA
<213>  Artificial Sequence

<220>
<223>  TFAP2A forward

<400>  51
ctcgatccac tccttacctc ac                                               22


<210>  52
<211>  22
<212>  DNA
<213>  Artificial Sequence

<220>
<223>  TFAP2A reverse

<400>  52
cctgcaggca gatttaatcc ta                                               22


<210>  53
<211>  22
<212>  DNA
<213>  Artificial Sequence

<220>
<223>  TFAP2B Reverse

<400>  53
aatggaagac gtccagtcag tt                                               22


<210>  54
<211>  22
<212>  DNA
<213>  Artificial Sequence

<220>
<223>  TFAP2B reverse

<400>  54
agtgaacagc ttctccttcc ac                                               22


<210>  55

<211>  22
<212>  DNA
<213>  Artificial Sequence

<220>
<223>  TFAP2C forward

<400>  55
gatcagacag tcattcgcaa ag                                                22


<210>  56
<211>  22
<212>  DNA
<213>  Artificial Sequence

<220>
<223>  TFAP2C reverse

<400>  56
caaagtccct agccaaatga ac                                                22


<210>  57
<211>  22
<212>  DNA
<213>  Artificial Sequence

<220>
<223>  TFAP2D forward

<400>  57
gccaaggtgg agtgataaga ag                                                22


<210>  58
<211>  22
<212>  DNA
<213>  Artificial Sequence

<220>
<223>  TFAP2D

<400>  58
ggcaagatgt tctcctactg ct                                                22

## Description

### FIELD OF THE INVENTION

The present invention relates to novel methods and kits for predicting, prognosing, and/or monitoring therapeutic efficacy of cancer therapy on a subject having malignant tumour or cell proliferative disorder. In particular, the present invention relates to novel methods and kits for predicting, prognosing, and/or monitoring therapeutic efficacy of chemotherapy or chemoradiotherapy, in particular therapeutic efficacy of chemotherapeutic agents on a subject having malignant tumour or cell proliferative disorder. In addition, the present invention relates to use of the said methods and kits.

### BACKGROUND

Oncologists have a number of treatment options available to them, including different combinations of chemotherapeutic drugs. In recent years the treatment options for various cancers have profoundly improved through the introduction of polychemotherapies together with targeted antibody therapy. Since patients now undergo several consecutive lines of treatment, the molecular alterations underlying chemoresistance need to be better defined in order to individualize cancer therapy. Best likelihood of good treatment outcome requires that patients be assigned to optimal available cancer treatment. In particular, it is important to determine the likelihood of patient response to chemotherapy because chemotherapeutic drugs have limited efficacy and are toxic.

Progression free and overall survival has improved even in patients with distant metastasis. Since these therapies are now given in different treatment lines, identifying the molecular mechanisms of treatment response and/or resistance is becoming an important factor to better individualize cancer chemotherapy. So far knowledge on the molecular mechanisms of chemotherapy resistance in this and other cancers is very limited, despite the fact that the molecular biology of cancer initiation and progression has been elucidated in recent decades.

Most patients with advanced disease receive 5-FU based polychemotherapies, which in combination with novel targeted agents have demonstrated improved progression free and overall survival. The following publications which are incorporated by reference show the role of 5-FU in treatment of cell proliferative disorders, in particular colorectal, pancreatic, gastric, head and neck, breast, non-small-cell lung, leukaemia, and/or gynaecologic cancers: Comella P, Casaretti R, Sandomenico C, Avallone A, Franco L. Capecitabine, alone and in combination, in the management of patients with colorectal cancer: a review of the evidence. Drugs. 2008;68(7):949-61. Review. PubMed PMID: 18457461; Geh JI, Bond SJ, Bentzen SM, Glynne-Jones R. Systematic overview of preoperative (neoadjuvant) chemoradiotherapy trials in oesophageal cancer: evidence of a radiation and chemotherapy dose response. Radiother Oncol. 2006 Mar;78(3):236-44. Epub 2006 Mar 20. Review. PubMed PMID: 16545878; Gerszten K, Selvaraj RN, Kelley J, Faul C. Preoperative chemoradiation for locally advanced carcinoma of the vulva. Gynecol Oncol. 2005 Dec;99(3):640-4. Epub 2005 Sep 19. PubMed PMID: 16169579; Bourhis J, Calais G, Lapeyre M, Tortochaux J, Alfonsi M, Sire C, Bardet E, Rives M, Bergerot P, Rhein B, Desprez B; French Head and Neck Cancer Group (GORTEC). Concomitant radiochemotherapy or accelerated radiotherapy: analysis of two randomized trials of the French Head and Neck Cancer Group (GORTEC); Semin Oncol. 2004 Dec;31(6):822-6. Review. PubMed PMID: 15599861; Aftimos PG, Nasr EA, Nasr DI, Noun RJ, Nasr FL, Ghosn MG, El Helou JA, Chahine GY. Adjuvant chemo-radiation for gastric adenocarcinoma: an institutional experience. Radiat Oncol. 2010 Jun 4;5(1):50. PubMed PMID: 20525367; PubMed Central PMCID: PMC2887890; Chua TC, Koh JL, Yan TD, Liauw W, Morris DL. Cytoreductive surgery and perioperative intraperitoneal chemotherapy for peritoneal carcinomatosis from small bowel adenocarcinoma. J Surg Oncol. 2009 Aug 1;100(2):139-43. PubMed PMID: 19544356; Sultana A, Tudur Smith C, Cunningham D, Starling N, Neoptolemos JP, Ghaneh P; Meta-analyses of chemotherapy for locally advanced and metastatic pancreatic cancer: results of secondary end points analyses. Br J Cancer. 2008 Jul 8;99(1):6-13. Epub 2008 Jun 24. PubMed PMID: 18577990; PubMed Central PMCID: PMC2453014; Hosono Y, Osada S, Nawa M, Takahashi T, Yamaguchi K, Kawaguchi Y, Yoshida K. Combination therapy of 5-fluorouracil with rapamycin for hormone receptor-negative human breast cancer. Anticancer Res. 2010 Jul;30(7):2625-30. PubMed PMID: 20682991; and Auerbach M, Elias EG, Orford J. Experience with methotrexate, 5-fluorouracil, and leucovorin (MFL): a first line effective, minimally toxic regimen for metastatic breast cancer. Cancer Invest. 2002;20(1):24-8. PubMed PMID: 11852998. Fluoropyrimidines are the backbones of polychemotherapy in cancer therapy. Recently, drugs targeting the EGFR and VEGF have also been approved for the first-line treatment of patients with colorectal cancer. For instance, the overall survival of patients with colorectal cancer has increased from a median of 6-8 months in the era of 5-FU monotherapy to 24 months in patients receiving polychemotherapies with targeted drugs. Therefore, administration of these drugs in several lines of treatment is becoming a common strategy. Thus, individualization of cancer therapy is becoming routine and drugs need to be chosen on an individual basis with respect to certain clinical and genetic response predictors. Accordingly, recent studies have demonstrated that patients with mutant *KRAS-* or *BRAF-*cancer cells do not respond to EGFR inhibition. However, further molecular mechanisms underlying chemoresistance or allowing response prediction in this and other cancers are largely unknown.

5-FU is in the category of chemotherapeutic agents called antimetabolites. Antimetabolites attach specific phases of the cell cycle and most of them interfere with nucleic acid synthesis or nucleotide synthesis. 5-FU is a thymidylate synthase inhibitor and blocks synthesis of thymidine, leading to inhibition of DNA replication, inducing cell cycle arrest and apoptosis. 5-FU acts also as a pyrimidine analogue, inhibits replication enzymes and is transformed inside the cell into different cytotoxic metabolites. Other pyrimidine anatagonists are cytarabine (also called arabinosylcytosine or araC) which blocks DNA synthesis, decitabine which is a chemical analogue of cytidine which blocks DNA, RNA and protein synthesis, floxuridine which is a precursor to 5FU and which is metabolized in the body to 5FU, capecitabine which functions similar to floxuridine, Gemcitabine which is deoxycytidine analogue that has a broad spectrum of antitumour activity in many solid tumors. Resistance to 5-FU can be partly explained by mutations or polymorphisms in genes encoding drug catalyzing enzymes, like dihydropyrimidine dehydrogenase (DYPD) and methylenetetrahydrofolate reductase (MTHFR), leading to an increased risk of toxicity. Other genes have also been implicated in the mediation of 5-FU response including heat shock proteins and hypoxia induced factors, as well as cell adhesion molecules, and an apoptosis inhibitor. In addition, several methylated genes have been identified as potential markers for 5-FU resistance, such as MLH1 and MGMT.

Recently, epigenetic alterations underlying the pathogenesis of various cancers have been reported by several groups. These epigenetic alterations include hypo- and hypermethylation of the DNA, as well as modification of histones which all show a profound impact on transcriptional gene regulation. Further, aberrant methylation of CpG islands has been shown to lead to the transcriptional silencing of certain genes that have been previously linked to the pathogenesis of various cell proliferative disorders and cancers. CpG islands are sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting. For example, multiple genes have been shown to be hypermethylated in the early steps of cancer, including APC, hMLH1 among others and increased expression of HDACs has been reported in this disease as well. However, the role of these molecular alterations in the context of response prediction and treatment resistance are far less well understood. The AP2-transcription factor family consists of five members and plays important roles in both developmental (namely eye, skin and neural structures) and cancer biology. TFAP2A is a known tumour suppressor in various cancers and has been shown to be hypermethylated in renal cell carcinoma and B-Cell lymphoma. Similar results have been recently published for TFAP2C in breast cancer which acts as a tumour suppressor in this cancer. In prostate and colorectal cancer, TFAP2D and TFAP2E have been shown to be hypermethylated and were evaluated as potential methylation biomarkers in these diseases. The U.S. Patent application No. 12/374,650 which is incorporated by reference, discloses association of hypermethylated TFAP2E with colorectal cancer. TFAP2E has 2 CpG islands, underscoring the potential for regulation of gene expression by CpG hypermethylation.

### DISCLOSURE OF THE INVENTION

Although a variety of biomarkers have been identified, there still exists a need to identify a biomarker that can reliably predict, prognose, and/or monitor therapeutic efficacy of cancer therapy and its outcome on individual subjects having cell proliferative disorders, in particular on individual subjects having colorectal, pancreatic, gastric, head and neck, breast, non-small-cell lung, leukaemias, and/or gynaecologic cancers who have undergone or are undergoing chemotherapy, polychemothrapies, or chemoradiotherapy, preferably, 5-Flurouracil based chemotherapy or polychemothrapies.

Further, there still exists a need to identify a biomarker in order to select individual subjects having cell proliferative disorder or cancer for a particular cancer therapy and to identify individual subjects with cell proliferative disorder or cancer where a particular cancer therapy is unlikely to exhibit any clinical benefit.

Therefore, it would be highly desirable to be able to identify whether a subject with cell proliferative disorder or cancer who has undergone or is undergoing cancer therapy including chemotherapy, polychemotherapy, chemoradiotherapy, treatments with chemotherapeutic agents such as antimetabolites, in particular treatments with 5-Flurouracil based chemotherapy or polychemotherapies will be responsive to such treatments.

To solve the problems mentioned above, the present invention provides a method for predicting, prognosing, and/or monitoring therapeutic efficacy of cancer therapy on a subject having cell proliferative disorder by determining the methylation and/or expression levels of TFAP2E in a biological sample isolated from said subject. Further, the present invention identifies association of TFAP2E with treatment resistance towards chemotherapeutic agents, in particular 5-fluoruracil based chemotherapy or polychemotherapies *,in vitro* and *in vivo,* in various cell proliferative disorders and cancers, preferably colorectal, pancreatic, gastric, head and neck, breast, non-small-cell lung, leukaemia, and/or gynaecologic cancers.

### SUMMARY OF THE INVENTION

The present invention provides a method of predicting, prognosing, and/or monitoring, therapeutic efficacy of cancer therapy on a subject having malignant tumor or cell proliferative disorder comprising determining the methylation and/or expression levels of TFAP2E in a biological sample isolated from said subject, and predicting, prognosing, and/or monitoring therapeutic efficacy of cancer therapy on said subject based on methylation and/or expression levels of TFAP2E.

In further embodiment, the present invention provides a method of predicting, prognosing, and/or monitoring therapeutic efficacy of cancer therapy on a subject having malignant tumor or cell proliferative disorder by detecting the presence, absence or amount of CpG methylation within TFAP2E or sequence thereof.

In an embodiment, the present invention provides a method of predicting, prognosing, and/or monitoring therapeutic efficacy of cancer therapy on a subject having malignant tumor or cell proliferative disorder by detecting the presence, absence or level of mRNA transcribed from TFAP2E. In further embodiment, the said method according to the present invention is performed detecting the presence, absence or level of a polypeptide encoded by TFAP2E or sequence thereof.

In an embodiment, the present invention provides a method of predicting, prognosing, and/or monitoring therapeutic efficacy of cancer therapy on a subject having malignant tumor or cell proliferative disorder wherein hypermethylation and/or decreased expression of TFAP2E is indicative of resistance to cancer therapy, while hypomethylation and/or increased expression of TFAP2E is indicative of sensitivity to cancer therapy.

In an embodiment, the cell proliferative disorder according to the present invention, preferably, is colorectal, pancreatic, gastric, head and neck, breast, non-small-cell lung, leukaemia, and/or gynaecologic cancers.

In another embodiment, the cancer therapy according to the present invention includes chemotherapy, polychemotherapy, or chemoradiotherapy treatments on a subject having malignant tumor or cell proliferative disorder, in particular includes treatments with chemotherapeutic agents, more in particular, treatments with antimetabolites, preferably pyrimidine antagonists including cytarabine, decitabine, floxuridine, capecitabine, and Gemcitabine, and more preferably, 5-Flurouracil based chemotherapy or polychemothrapies.

In further embodiment, the present invention provides a method of predicting, prognosing, and/or monitoring therapeutic efficacy of cancer therapy on a subject having malignant tumor or cell proliferative disorder by detecting the presence, absence or amount of CpG methylation within TFAP2E or sequence thereof comprising contacting genomic DNA isolated from a biological sample obtained from said subject with at least one reagent, or series of reagents that distinguishes between methylated and non-methylated CpG dinucleotides within at least one target region of the genomic DNA, wherein the target region comprises, or hybridizes under stringent conditions to a sequence of contiguous nucleotides of SEQ ID NO.: 1, wherein said contiguous nucleotides comprise at least one CpG dinucleotide sequence.

In addition to the above embodiment, the present invention provides a method of predicting, prognosing, and/or monitoring therapeutic efficacy of cancer therapy on a subject having malignant tumor or cell proliferative disorder by detecting the presence, absence or amount of CpG methylation within TFAP2E or sequence thereof, further comprising contacting the treated genomic DNA, or the treated fragment thereof, with an amplification enzyme and at least one oligonucleotide comprising, a contiguous sequence that is complementary to, or hybridizes under moderately stringent or stringent conditions to a sequence selected from a group consisting of SEQ ID NOs: 2-5, and complements thereof, wherein the treated genomic DNA or the fragment thereof is either amplified to produce at least one amplificate, or is not amplified; and determining, based on a presence, absence or amount of, or on a property of said amplificate, the methylation state or level of at least one CpG dinucleotide of SEQ ID NO: 1, or an average, or a value reflecting an average methylation state or level of a plurality of CpG dinucleotides of SEQ ID NO: 1, whereby predicting, prognosing, and/or monitoring of said subject is afforded.

In further embodiment, the present invention provides a method of predicting, prognosing, and/or monitoring therapeutic efficacy of cancer therapy on a subject having malignant tumor or cell proliferative disorder wherein treating the genomic DNA, or the fragment thereof, comprises use of a reagent selected from the group comprising of bisulfite, hydrogen sulfite, disulfite, and combinations thereof.

In further embodiment, the present invention provides a method of predicting, prognosing, and/or monitoring therapeutic efficacy of cancer therapy on a subject having malignant tumor or cell proliferative disorder wherein the biological sample obtained from the subject is selected from the group comprising metastatic cells, cell lines, histological slides, biopsies, paraffin-embedded tissue, frozen tissue, formalin fixed tissue, body fluids, stool, colonic effluent , urine, blood plasma, blood serum, whole blood, isolated blood cells, cells isolated from the blood and combinations thereof.

In further embodiment, the present invention provides a method of predicting, prognosing, and/or monitoring therapeutic efficacy of cancer therapy on a subject having malignant tumor or cell proliferative disorder comprising digesting the genomic DNA, or a fragment thereof, isolated from a biological sample obtained from said subject with one or more methylation sensitive restriction enzymes; contacting the DNA restriction enzyme digest of above with an amplification enzyme and at least one oligonucleotide suitable for the amplification of a sequence comprising at least one CpG dinucleotide of SEQ ID NO: 1; determining, based on a presence, absence or class of an amplificate the methylation state or level of at least one CpG dinucleotide of a sequence of SEQ ID NO: 1, and therefrom predicting, predicting, prognosing, and/or monitoring and/or monitoring of said subject is afforded.

In further embodiment, the present invention provides a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) one or more reagents to convert unmethylated cytosine bases to uracil or to anther base that is detectably dissimilar to cytosine in terms of hybridization properties (b) at least one oligonucleotide or polynucleotide able to hybridize under stringent or moderately stringent conditions to a sequence selected from a group consisting of SEQ ID NOs.: 2-5 in order to detect the presence, absence or amount of CpG methylation within TFAP2E, in particular a sequence SEQ ID NO.: 1, or sequence thereof c) containers suitable for containing the said one or more reagents and the biological sample of the patient; and optionally (d) instructions for use.

In further embodiment, the present invention provides a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) a means for detecting TFAP2E polypeptides; (b) a container suitable for containing the said means and the biological sample of the patient comprising the polypeptides wherein the means can form complexes with the polypeptides; (c) a means to detect the complexes of (b).

In further embodiment, the present invention provides a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to a sequence of SEQ ID NO: 1; and optionally (d) instructions for use and interpretation of the kit results.

In further embodiment, the present invention relates to 5-azacytidine for treatment of a subject having malignant tumor or cell proliferative disorder, in particular colorectal cancer, wherein TFAP2E is hypermethylated.

Additional embodiments provide use of the methods and/or kits for predicting, prognosing, and/or monitoring therapeutic efficacy of cancer therapy on a subject having colorectal cancer.

### DRAWINGS

Those of skill in the art will understand that the drawings, described below, are for illustrative purposes only. The drawings are not intended to limit the scope of the present teachings in any way.
Figure 1. Illustrates Luciferase reporter assays for (A) CACO-2 cells transfected with DKK4 promoter (1kb and 2kb fragments) and TFAP2E CDS, (B) SW480 cells transfected with DKK4 promoter (1kb and 2kb fragments) and TFAP2E CDS, (C) HT29 transfected with DKK4 promoter (1kb fragment) and TFAP2E CDS, (D) Hek cells used as controls.* p<0.01.
Figure 2. Illustrates chromatin immunoprecipitation. Relative quantification results for FLAG tagged stable TFAP2E overexpressing clones (FTS4, FTS 10) with (A) putative AP2 protein binding sites 2kb upstream in the promoter of the DKK4 gene and (B) putative AP2 protein binding sites 1kb upstream in the promoter of the DKK4 gene.
Figure 3. Illustrates drug resistance assays. Resistance to treatment with fluorouracil (5-FU), (A) SW480 cells transiently transfected with DKK4-CDS, TFAP2E-CDS and both (50.000 cells per well, fluorouracil 50 µg/ml daily on day 1-3); and (B) TFAP2E-CDS stable overexpressing clones and empty vector controls (4 clones per group, 50.000 cells per well; fluorouracil 50 µg/m daily on day 1-3); mean of 3 independent experiments performed in triplicates.
Figure 4. Illustrates treatment responses in four patient cohorts and association with TFAP2E methylation. Response effects due to non-methylation with respect to the center affiliation of patients (A) as well as to the response evaluation technique (B).
Figure 5. Shows sequence of the TFAP2E (A) and DKK4 (B) gene.
Figure 6. Shows analysis of TFAP2E methylation in 74 colorectal cancer patients.
Figure 7. Illustrates expression of TFAP2E and DKK4 mRNA in colorectal cancer cell, lines where n = untreated, a = 5-azacytidine, T = Trichostatin A.

### DETAILED DESCRIPTION OF THE INVENTION

### Definitions:

Unless defined otherwise, all technical and scientific terms used herein have the same meaning as is commonly understood by one of skill in art to which the subject matter herein belongs. As used herein, the following definitions are supplied in order to facilitate the understanding of the present invention.

The terms "therapeutic efficacy" or " efficacy of cancer therapy" means to assess the reaction of a cancer to one or series of anti-cancer treatment(s) including but not limited to chemotherapy, chemoradiotherapy, polychemotherapy, chemotherapeutic agents, antimetabolites, pyrimidine antagonists, and 5-Flurouracil based chemotherapy or polychemothrapies. In other words, it refers to assessing the ability of a cancer to respond favourably or to resists cancer therapy.

The term "predicting" can comprise determining if a patient's tumour will respond to a particular type of therapy. A biomarker that is present prior to any therapy and which predicts that outcome.

The term "prognosing" can comprise determining the outcome of a patient's condition, the chance of recovery, or how the disease will progress. For example, a patient can obtain a prognosis of having a 50% chance of recovery based on results from obtaining a sample from the patient. It may also comprise providing information about the patient's likely course of disease in the absence of suitable therapy (how aggressive or non-aggressive a tumour is).

The term "Monitoring" can comprise reporting recurrence or progression of disease.

The term RECIST (Response Evaluation Criteria In Solid Tumors) refers to a set of published rules that define when cancer patients improve ("respond"), stay the same ("stable") or worsen ("progression") during treatments.

The known term DMEM (Dulbecco's modified Eagle's medium) refers to a cell culture medium that can be used to maintain cells in tissue culture.

"Transcription Element Search System" or TESS refers to a web tool for predicting transcription factor binding sites in DNA sequences. It can identify binding sites using site or consensus strings and positional weight matrices from the TRANSFAC, JASPAR, IMD, and our CBIL-GibbsMat database. The abbreviation "CRC" used throughout the present application refers to colorectal cancer.

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence, absence or class of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemimethylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "Methylation assay" refers to any assay for determining the methylation state of one or more CpG dinucleotide sequences within a sequence of DNA.

The term"Methylation-Sensitive High Resolution Melting (MS-RHM) refers to a new approach for sensitive and high-throughput assessment of methylation. MS-HRM allows for estimation of the methylation level by comparing the melting profiles of unknown PCR products to the melting profiles of PCR products derived from standards with a known unmethylated to methylated template ratio. The term "hybridisation" is to be understood as a bond of an oligonucleotide to a complementary sequence along the lines of the Watson-Crick base pairings in the sample DNA, forming a duplex structure.

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

In particular preferred embodiments, the term "expression", "expression level", "over-expression", "increased expression", "increased expression level", "under-expression", "decreased expression level" or "decreased expression" refers to expression of mRNA, polypeptides or proteins as it is known in the art and which is based or influenced at least in parts by the presence or absence of methylation of a particular gene and/or its regulatory sequences. "over-expression", "increased expression", or "increased expression level" refers to expression which is based or influenced at least in parts by the absence of methylation. "under-expression", "decreased expression level" or "decreased expression" refers to expression which is based or influenced at least in parts by the presence of methylation.

Primer extension: Several embodiments of the primer extension method exist. These methods are based on the conversion of unmethylated cytosines to uracil while methylated cytosines remain unchanged by means of bisulfite. The resulting converted DNA is then subjected to pre-amplification, in particular PCR. Thereafter a methylation specific elongation of a primer is carried out. The binding site of said primer is located up to 10 bases before the cytosine position to be analyzed, is adjacent to it, or encompasses it. After hybridization the primer is elongated by one up to 10 nucleotides. The said methods are for example described in US 6,251,594, WO 01/62961, WO 01/062960, and WO 01/062064.

QM (Quantitative Methylation) method: According to this method, DNA is subjected to treatment, wherein methylated cytosine remains unchanged and unmethylated cytosine is converted to uracil or another base having different base pairing behaviour than cytosine. The converted DNA is then subjected to a real time PCR amplification in the presence of two probe systems. One probe system is specific for the case of methylation of the CpG positions of interest. The other probe system is specific for the case the CpG positions of interest are not methylated. Suitable probe systems are for example Taqman probes or LightCycler probes. In case of Taqman probes, each probes system consist of one probe. In case of LightCycler probes, each probe system consists of two probes acting cooperatively. By means of the said probe systems, the amplification products derived from correspondent converted methylated or unmethylated DNA are detected during amplification. From this, the amount of original DNA being methylated or being unmethylated is deduced. The QM method is for example described in WO 2005/098035.

HQM (Heavy Quantitative Methylation) method: According to this method, unmethylated cytosines of a double stranded DNA are converted to uracil or a base having a different base pairing behaviour than cytosine while methylated cytosine remains unchanged. In preferred embodiments of the HQM method both converted strands are subjected to methylation analysis, in particular wherein originally reverse complementary regions are analysed in the subjected DNA. In particular preferred is the analysis of both converted DNA strands in methylation specific reactions. Such reactions may be: PCR, isothermal amplification, LCR, MSP, HeavyMethyl, bisulfite sequencing, microarrays, methylation specific restriction enzymes, real-time PCR, HeavyMethyl, MethyLight, QM, methylation sensitive primer extension. The HQM method is descirbed in detail in WO 2008/017411

The term "MethyLight™" refers to the art-recognized fluorescence-based real-time PCR technique described by Eads et al., Cancer Res. 59:2302-2306, 1999. According to this method, DNA is treated with a modifying agent wherein unmethylated cytosines become modified. Thereafter the treated DNA is analyzed by real-time PCR with one or more probe. The binding site of said probe or probes comprises one or more CpG positions. Thus, the amount of amplification product derived either from DNA with methylated CpG positions or from DNA with unmethylated CpG positions is detected during amplification. From this, the amount of original DNA being methylated or unmethylated at the analysed CpG positions is deduced. Suitable probe systems for analysis are Taqman probe, LightCycler probes or Scorpion primers. The MethyLight method is for example described in WO 00/70090; US 6,331,393; or Trinh et al.: DNA methylation analysis by MethyLight technology. Methods, 25:456-62, 2001).

The term "HeavyMethyl™" assay or "HM", in the embodiment thereof implemented herein, refers to an assay, wherein methylation specific *blocking* probes (also referred to herein as *blockers)* covering CpG positions between, or covered by the amplification primers enable methylation-specific selective amplification of a nucleic acid sample. According to this method, in a first step, unmethylated cytosines of a DNA are converted to uracil while methylated cytosines remain unchanged. The converted DNA is then subjected to an amplification reaction. The amplification of the converted methylated or unmethylated DNA of interest is detected during the amplification while undesired background DNA is simultaneously suppressed by means of a blocker. Said blocker is either specific for background converted methylated DNA or background converted unmethylated DNA. Preferably, the HM method is carried out as a real time variant. The HM method is described for example in WO 02/072880.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146. According to this method, DNA is treated with bisulfite resulting in a conversion of unmethylated cytosines to uracil while methylated cytosines remain unaltered. After said conversion, the DNA is subjected to PCR amplification by means of methylation-specific primers. The MSP method is for example described in Herman et al.: Methylation specific PCR: a novel PCR assay for methylation status of CpG islands. Proc Natl Acad Sci USA. 93:9821-6, 1996. When carried out as a real-time variant, the MSP allows a quantification of amplified products. Thus, it is possible to deduce the amount of subjected DNA being methylated or unmethylated at the cytosine positions of interest.

The term "COBRA" (Combined Bisulfite Restriction Analysis) refers to the art-recognized methylation assay described by Xiong & Laird, Nucleic Acids Res. 25:2532-2534, 1997. According to this method, DNA is treated with bisulfite. After the treatment, the DNA is amplified by means of PCR. Thereafter, the amplified DNA is digested by means of restriction enzymes. Said enzymes are specific for bisulfite treated unmethylated sites. Digested or undigested fragments are finally detected for example by gel electrophoresis and in if the case may be hybridization of a specific labelled probe. Methylation levels in the original DNA sample are represented by the relative amounts of digested and undigested PCR product. The COBRA method is for example described in Xiong & Laird, Nucleic Acids Res. 25:2532-2534, 1997.

MLA (Methylation Sensitive Ligation and Amplification): According to this method, DNA is subjected to a treatment wherein unmethylated cytosine is converted to uracil, while methylated cytosine remains unchanged. Preferably, said treatment is a treatment with bisulfite. Subsequently, the treated DNA is amplified by means of at least two pairs of essentially complementary probe oligonucleotides. The methylation status of the cytosine of interest in the original DNA is deduced from the presence or absence of amplificates. The MLA method is for example described in WO 03/057909.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

Direct sequencing: this sequencing method uses bisulfite-treated DNA utilized with PCR. Primers are designed to be strand-specific as well as bisulfite-specific. Both methylated and unmethylated sequences are amplified. All sites of unmethylated cytosines are portrayed as thymines in the resulting amplified sequence of the sense strand, and as adenines in the amplified antisense strand. Pyrosequencing: Pyrosequencing is a real-time sequencing technology based on luminometric detection of pyrophosphate release upon nucleotide incorporation which is suited for simultaneous analysis and quantification of the methylation degree of several CpG positions. After bisulfite, modification of genomic DNA, a region of interest is amplified by polymerase chain reaction (PCR) with one of the two primers being biotinylated, The PCR-generated template is rendered single stranded and a Pyrosequencing primer is annealed to analyze quantitatively CpG positions. After bisulfite treatment and PCR, the degree of each methylation at each CpG position in a sequence is determined from the ratio of T and C signals reflecting the proportion of unmethylated and methylated cytosines at each CpG site in the original sequence.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5. "Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

In reference to composite array sequences, the phrase "contiguous nucleotides" refers to a contiguous sequence region of any individual contiguous sequence of the composite array, but does not include a region of the composite array sequence that includes a "node," as defined herein above.

The term "pre-cancerous" or "pre-neoplastic" and equivalents thereof shall be taken to mean any cellular proliferative disorder which is undergoing malignant transformation. Examples of such conditions include, in the context of colorectal cellular proliferative disorders, cellular proliferative disorders with a high degree of dysplasia and the following classes of adenomas:
Level 1: penetration of malignant glands through the muscularis mucosa into the submucosa, within the polyp head
Level 2: the same submucosal invasion, but present at the junction of the head to the stalk
Level 3: invasion of the stalk
Level 4: invasion of the stalk's base at the connection to the colonic wall (this level corresponds to stage Dukes A)

The term gene or genomic sequence shall be taken to include all transcript variants thereof and all promoter and regulatory elements thereof. Furthermore as a plurality of SNPs are known within said gene the term shall be taken to include all sequence variants.

As used herein the term "prognosing" shall be taken to mean an indicator of the predicted progression of the disease (including but not limited to aggressiveness and metastatic potential) and/or predicted patient survival time.

In the context of the present invention the term 'aggressiveness' is taken to mean one or more of high likelihood of relapse post surgery; below average or below median patient survival; below average or below median disease free survival; below average or below median relapse-free survival; above average tumor-related complications; fast progression of tumor or metastases.

Unless stated otherwise as used herein the term "survival" shall be taken to include all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (e.g. time of diagnosis or start of treatment) and end point (e.g. death, recurrence or metastasis).

### Method of present invention

The present invention provides a method of predicting, prognosing, and/or monitoring therapeutic efficacy of cancer therapy on a subject having malignant tumor or cell proliferative disorder comprising determining the methylation and/or expression levels of TFAP2E in a biological sample isolated from said subject, and predicting, prognosing, and/or monitoring therapeutic efficacy of cancer therapy on said subject based on methylation and/or expression levels of TFAP2E. In one embodiment, the cancer therapy is chemotherapy, polychemotherapy, or chemoradiotherapy. In further embodiment, cancer therapy includes therapy with chemotherapeutic agents, in particular with antimetabolites. Cancer therapy also comprises treatments with pyrimidine antagonists including cytarabine, decitabine, floxuridine, capecitabine, and Gemcitabine. In further embodiment cancer therapy comprises 5-Flurouracil based chemotherapy or polychemothrapies.

The malignant tumor or cell proliferative disorder according to the present invention comprises colorectal, pancreatic, gastric, head and neck, breast, non-small-cell lung, leukaemia, and/or gynaecologic cancers.

The present invention provides for the use of the bisulfite technique, in combination with one or more methylation assays, for determination of the methylation status of CpG dinucleotide sequences within SEQ ID NO. 1, preferably SEQ ID NO. 6, and more preferably SEQ ID NO. 11. Genomic CpG dinucleotides can be methylated or unmethylated (alternatively known as up- and down- methylated respectively). Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off' is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) of TFAP2E.

According to the present invention, determination of the methylation status of CpG dinucleotide sequences within SEQ ID NO. 1, preferably SEQ ID NO. 6, and more preferably SEQ ID NO. 11 has utility in the predicting, prognosing, and/or monitoring therapeutic efficacy of cancer therapy on a subject having malignant tumor or cell proliferative disorder.

### Methylation Assay Procedures.

Various methylation assay procedures are known in the art, and can be used in conjunction with the present invention. For example, the U.S. Patent application No. 12/374,650 which is incorporated by reference, discloses methylation assay procedures of TFAP2E. These assays allow for determination of the methylation state of one or a plurality of CpG dinucleotides (e.g., CpG islands) within a DNA sequence. Such assays involve, among other techniques, DNA sequencing of bisulfite-treated DNA, RT-PCR or PCR (for sequence-specific amplification), Southern blot analysis. For example, genomic sequencing has been simplified for analysis of DNA methylation patterns and 5-methylcytosine distribution by using bisulfite treatment (Frommer et al., Proc. NatL Acad. Sci. USA 89:1827-1831, 1992). Additionally, restriction enzyme digestion of PCR products amplified from bisulfite-converted DNA is used, *e.g.,* the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

It is particularly preferred that said reagent converts cytosine bases which are unmethylated at the 5'-position to uracil, thymine, or another base which is dissimilar to cytosine in terms of hybridization behaviour. However in an alternative embodiment said reagent may be a methylation sensitive restriction enzyme.

Wherein the genomic DNA sample is treated in such a manner that cytosine bases which are unmethylated at the 5'-position are converted to uracil, thymine, or another base which is dissimilar to cytosine in terms of hybridization behaviour It is preferred that this treatment is carried out with bisulfite (hydrogen sulfite, disulfite) and subsequent alkaline hydrolysis. Such a treatment results in the conversion of SEQ ID NO: 1, 6, and 11 to SEQ ID NO: 2-5, 7-10, and 12-15, respectively.

The treated genomic DNA is then analyzed in order to determine the methylation state of the target gene sequences. It is particularly preferred that the target region comprises, or hybridizes under stringent conditions to contiguous nucleotides of TFAP2E, preferably at least 16 contiguous nucleotides of TFAP2E. It is preferred that the sequence of said gene according to SEQ ID NO: 1 is analyzed, preferably SEQ ID NO. 6, and more preferably SEQ ID NO. 11. The method of analysis may be selected from those known in the art, including those listed herein. Particularly preferred are MS-RHM, MethyLight, MSP and the use of blocking oligonucleotides (HeavyMethyl) as described herein. It is further preferred that any oligonucleotides used in such analysis (including primers, blocking oligonucleotides and detection probes) should be reverse complementary, identical, or hybridize under stringent or highly stringent conditions to (preferably, an at least 9-base-pair long segment of) the base sequences of one or more of SEQ ID NO: 2-5, preferably SEQ ID NO.:7-10, and more preferably SEQ ID NO.:12-15 and sequences complementary thereto. Preferably,

The genomic sequences according to SEQ ID NO. 1,6, 11 and non-naturally occurring treated variants thereof according to SEQ ID NO. 2-5, 7-10, 12-15, respectively were determined to have novel utility for predicting, prognosing, and/or monitoring therapeutic efficacy of cancer therapy on a subject having malignant tumor or cell proliferative disorder.

In one embodiment the method of the invention comprises the following steps: a) determining the methylation and/or expression levels of TFAP2E in a biological sample isolated from said subject; and predicting, prognosing, and/or monitoring therapeutic efficacy of cancer therapy on said subject based on step a). In a preferred embodiment said cancer therapy is chemotherapy, polychemotherapy or chemoradiotherapy. In further embodiment, cancer therapy includes therapy with chemotherapeutic agents, in particular with antimetabolites. Cancer therapy also comprises treatments with pyrimidine antagonists including cytarabine, decitabine, floxuridine, capecitabine, and Gemcitabine. In further embodiment cancer therapy comprises 5-Flurouracil based chemotherapy or polychemothrapies.

The malignant tumor or cell proliferative disorder according to the present invention comprises colorectal, pancreatic, gastric, head and neck, breast, non-small-cell lung, leukaemia, and/or gynaecologic cancers.

In further embodiment of the present invention, predicting, prognosing, and/or monitoring therapeutic efficacy of cancer therapy on a subject having malignant tumor or cell proliferative disorder comprises a) determining the methylation and/or expression levels of TFAP2E in a biological sample isolated from said subject; b) predicting, prognosing, and/or monitoring therapeutic efficacy of cancer therapy on said subject based on step a). Preferably thereby, hypermethylation and/or decreased expression of TFAP2E is indicative of resistance to cancer therapy, while hypomethylation and/or increased expression of TFAP2E is indicative of sensitivity to cancer therapy.

The method of the invention may be enabled by means of any analysis of the expression of an RNA transcribed therefrom or polypeptide or protein translated from said RNA, preferably by means of mRNA expression analysis or polypeptide expression analysis.

Accordingly the present invention also provides prediction, prognostic, and/or monitoring assays and methods, both quantitative and qualitative for detecting the expression of TFAP2E in a subject and determining therefrom the predicting, prognosing, and/or monitoring therapeutic efficacy of cancer therapy on a subject having malignant tumor or cell proliferative disorder.

In further embodiment, the present invention provides a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) one or more reagents to convert unmethylated cytosine bases to uracil or to anther base that is detectably dissimilar to cytosine in terms of hybridization properties (b) at least one oligonucleotide or polynucleotide able to hybridize under stringent or moderately stringent conditions to a sequence selected from a group consisting of SEQ ID NOs.: 2-5, preferably SEQ ID NOs.: 7-10, and more preferably SEQ ID NOs.: 12-15 in order to detect the presence, absence or amount of CpG methylation within TFAP2E, in particular a sequence SEQ ID NO.: 1, preferably SEQ ID NO.: 6, more preferably SEQ ID NO.:11, respectively, or sequence thereof c) containers suitable for containing the said one or more reagents and the biological sample of the patient; and optionally (d) instructions for use.

In further embodiment, the present invention provides a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) a means for detecting TFAP2E polypeptides; (b) a container suitable for containing the said means and the biological sample of the patient comprising the polypeptides wherein the means can form complexes with the polypeptides; (c) a means to detect the complexes of (b).

In further embodiment, the present invention provides a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical , are complementary, or hybridize under stringent or moderately stringent conditions to a sequence of SEQ ID NO: 1, preferably SEQ ID NO.: 6, more preferably SEQ ID NO.:11; and optionally (d) instructions for use and interpretation of the kit results.

In a further embodiment of the present invention the said kit further comprising at least one oligonucleotide which is identical, is complementary, or hybridizes under stringent or moderately stringent conditions to at least 9 base long segment of a sequence selected from SEQ ID NOs.: 1, 2-5, preferably SEQ ID NO.: 6, 7-10, more preferably SEQ ID NO.:11, 12-15.

The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase. It is further preferred that said kit further contains an Rnase reagent.

The present invention further provides for methods for the detection of the presence of the polypeptide encoded by TFAP2E sequences in a sample obtained from said subject.

Aberrant levels of polypeptide expression of the polypeptides encoded by TFAP2E are associated with the prediction, predicting, prognosing, and/or monitoring, and or monitoring of a subject having malignant tumor or cell proliferative disorder.

According to the present invention under-expression of said polypeptides is associated with a negative prediction and/or predicting, prognosing, and/or monitoring therapeutic efficacy of cancer therapy on a subject having malignant tumor or cell proliferative disorder. Any method known in the art for detecting polypeptides can be used. Such methods include, but are not limited to mass-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide(s) encoded by TFAP2E. Such antibodies are useful for predicting, prognosing, and/or monitoring therapeutic efficacy of cancer therapy on a subject having malignant tumor or cell proliferative disorder. In certain embodiments production of monoclonal or polyclonal antibodies can be induced by the use of an epitope encoded by a polypeptide of TFAP2E as an antigene. Such antibodies may in turn be used to detect expressed polypeptides. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase) . The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference in its entirety). Methods for preparation of the polypeptides or epitopes thereof include, but are not limited to chemical synthesis, recombinant DNA techniques or isolation from biological samples.

In the final step of the method the predicting, prognosing, and/or monitoring of the subject is determined, whereby under-expression (of mRNA or polypeptides) is indicative of non-responsivenss to chemotherapy, in particular 5-FU based chemotherapy on a subject having malignant tumor or cell proliferative disorder. The term under-expression shall be taken to mean expression at a detected level less than a pre-determined cut off which may be selected from the group consisting of the mean, median or an optimised threshold value. The term over-expression shall be taken to mean expression at a detected level greater than a pre-determined cut off which may be selected from the group consisting of the mean, median or an optimised threshold value.

Another aspect of the invention provides a kit for use in determining the predicting, prognosing, and/or monitoring therapeutic efficacy of cancer therapy on a subject having malignant tumor or cell proliferative disorder according to the methods of the present invention, comprising: a means for detecting TFAP2E polypeptides. The means for detecting the polypeptides comprise preferably antibodies, antibody derivatives, or antibody fragments. The polypeptides are most preferably detected by means of Western Blotting utilizing a labelled antibody. In another embodiment of the invention the kit further comprising means for obtaining a biological sample of the subject.

### METHYLATION ANALYSIS

Particular embodiments of the present invention provide a novel application of the analysis of methylation levels and/or patterns within TFAP2E that enables prediction, prognosing, and/or monitoring of a subject having malignant tumor or cell proliferative disorder. Prediction, prognosing, and/or monitoring of a patient having having malignant tumor or cell proliferative disorder enables the physician to make better and more informed treatment decisions. In one embodiment said predicting, prognosing, and/or monitoring is on a subject with having malignant tumor or cell proliferative disorder who has undergone or is undergoing cancer therapy, In a preferred embodiment said cancer therapy is chemotherapy, polychemotherapy or chemoradiotherapy. In further embodiment, cancer therapy includes therapy with chemotherapeutic agents, in particular with antimetabolites. Cancer therapy also comprises treatments with pyrimidine antagonists including cytarabine, decitabine, floxuridine, capecitabine, and Gemcitabine. In further embodiment cancer therapy comprises 5-Flurouracil based chemotherapy or polychemothrapies.

In the preferred embodiment of the method, the predicting, prognosing, and/or monitoring therapeutic efficacy of cancer therapy on a subject having malignant tumor or cell proliferative disorder is determined by analysis of the methylation status of one or more CpG dinucleotides of TFAP2E gene. In one embodiment the invention of said method comprises the following steps: i) contacting genomic DNA (preferably isolated from metastatic cells, cell lines, histological slides, biopsies, paraffin-embedded tissue, frozen tissue, formalin fixed tissue, body fluids, stool, colonic effluent, urine, blood plasma, blood serum, whole blood, isolated blood cells, cells isolated from the blood and combinations thereof) obtained from the subject with at least one reagent, or series of reagents that distinguishes between methylated and non-methylated CpG dinucleotides within TFAP2E (including promoter and regulatory regions thereof) and ii) predicting, prognosing, and/or monitoring of said subject having malignant tumor or cell proliferative disorder based on results of step i).

It is preferred that said one or more CpG dinucleotides of TFAP2E are comprised within a respective genomic target sequence thereof as provided in SEQ ID NO. 1 and complements thereof, preferably SEQ ID NO. 6, and more preferably SEQ ID NO. 11 and their complements thereof. The present invention further provides a method for ascertaining genetic and/or epigenetic parameters of TFAP2E and/or the genomic sequence according to SEQ ID NO. 1 preferably SEQ ID NO. 6, and more preferably SEQ ID NO. 11 within a subject by analyzing cytosine methylation. Said method comprises contacting a nucleic acid comprising SEQ ID NO. 1, preferably SEQ ID NO. 6, and more preferably SEQ ID NO. 11 in a biological sample obtained from said subject with at least one reagent or a series of reagents, wherein said reagent or series of reagents, distinguishes between methylated and non-methylated CpG dinucleotides within the target nucleic acid.

In a preferred embodiment, said method comprises the following steps: In the *first step,* a sample of the tissue to be analyzed is obtained. The source may be any suitable source, such as metastatic cells, cell lines, histological slides, biopsies, paraffin-embedded tissue, frozen tissue, formalin fixed tissue, body fluids, stool, colonic effluent, urine, blood plasma, blood serum, whole blood, isolated blood cells, cells isolated from the blood and combinations thereof.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g. ultrafiltration , silica surfaces or membranes, magnetic particles, polystyrol particles, polystyrol surfaces, positively charged surfaces, and positively charged membranes, charged membranes, charged surfaces, charged switch membranes, charged switched surfaces.

Once the nucleic acids have been extracted, the genomic double stranded DNA is used in the analysis, methylation analysis may be carried out by any means known in the art including but not limited to methylation sensitive restriction enzyme analysis and chemical reagent analysis.

### CHEMICAL ANALYSIS

In the *second step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/011715, which is incorporated by reference in its entirety). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2, 5,7,8, -tetramethylchromane 2-carboxylic acid or trihydroxybenzoe acid and derivates thereof, e.g. Gallic acid (see: PCT/EP2004/011715 which is incorporated by reference in its entirety). The bisulfite conversion is preferably carried out at a reaction temperature between 30°C and 70°C, whereby the temperature is increased to over 85°C for short periods of times during the reaction (see: PCT/EP2004/011715 which is incorporated by reference in its entirety). The bisulfite treated DNA is preferably purified priori to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol (see: PCT/EP2004/011715 which is incorporated by reference in its entirety).

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR) or Real Time polymerase chain reaction (RT-PCR). Preferably said amplificates are 100 to 2,000 base pairs in length. The primer oligonucleotides includes at least one oligonucleotides whose sequence is reverse complementary, identical, or hybridize under stringent, moderately or highly stringent conditions to the base sequences of one of SEQ ID NOs. 2-5, preferably SEQ ID NOs. 7-10, and more preferably SEQ ID NOs: 12-15 and sequences complementary thereto, preferably to an at least 9, 16, or 20-base-pair long segment of the base sequences of one of SEQ ID NOs. 2-5 preferably SEQ ID NOs. 7-10, and more preferably SEQ ID NOs: 12-15.

In an alternate embodiment of the method, the methylation status of pre-selected CpG positions within TFAP2E and preferably within the nucleic acid sequences according to SEQ ID NO.1 preferably SEQ ID NO 6, and more preferably SEQ ID NO. 11 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers comprise a sequence having a length of at least 9, 16, or 20 nucleotides which hybridizes to a treated nucleic acid sequence according to one of SEQ ID NOs. 2-5 preferably SEQ ID NOs. 7-10, and more preferably SEQ ID NOs: 12-15 and sequences complementary thereto, wherein the base sequence of said oligomers comprises at least one CpG dinucleotide. A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker—a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* comprise a sequence having a length of at least 9, 16, or 20 nucleotides which hybridizes to a treated nucleic acid sequence according to one of SEQ ID NOs. 2-5 preferably SEQ ID NOs. 7-10, and more preferably SEQ ID NO.s: 12-15 and sequences complementary thereto, wherein the base sequence of said oligonucleotides comprises at least one CpG, TpG or CpA dinucleotide. It is particularly preferred that the base sequence of said *blocking oligonucleotides* comprise a sequence having a length of at least 9, 16, or 20 nucleotides which hybridizes to a treated nucleic acid sequence according to one of SEQ ID NOs. 2-5 preferably SEQ ID NOs. 7-10, and more preferably SEQ ID NOs: 12-15 and sequences complementary thereto, wherein the base sequence of said oligonucleotides comprises at least one TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallization. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analyzed in order to ascertain the methylation status of the CpG dinucleotides prior to the treatment. In embodiments where the amplificates were obtained by means of MSP amplification, the presence, absence or class of an amplificate is in itself indicative of the methylation state of the CpG positions covered by the primer, according to the base sequences of said primer.

Amplificates obtained by means of both standard and methylation specific PCR may be further analyzed by means of based-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9, 16, or 20 nucleotides which is reverse complementary or identical to a segment of the base sequences specified in the present Sequence Listing; and the segment comprises at least one CpG, TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence SEQ ID NO.1 preferably SEQ ID NO. 6, and more preferably SEQ ID NO. 11 and the equivalent positions within SEQ ID NOs. 2-5, preferably SEQ ID NOs. 7-10, and more preferably SEQ ID NOs: 12-15. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridized to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridize to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLightTM™ assay. Variations on the TaqMan™ detection methodology that are also suitable for use with the described invention include the use of dual-probe technology (LightCycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, *the fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, *the fourth step* of the method comprises sequencing and subsequent sequence analysis of the amplificate generated in the *third step* of the method (Sanger F., et al., Proc Natl Acad Sci USA 74:5463-5467, 1977).

Bisulfite modification of DNA is an art-recognized tool used to assess CpG methylation status. The most frequently used method for analyzing DNA for the presence of 5-methylcytosine is based upon the reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

An overview of art-recognized methods for detecting 5-methylcytosine is provided by Rein, T., et al., Nucleic Acids Res., 26:2255, 1998.

The bisulfite technique, barring few exceptions (e.g., Zeschnigk M, et al., Eur J Hum Genet. 5:94-98, 1997), is currently only used in research. In general, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

In further embodiment, the method of the invention is enabled by means of any analysis of the expression of an RNA transcribed therefrom or polypeptide or protein translated from said RNA, preferably by means of mRNA expression analysis or polypeptide expression analysis. However, in the most preferred embodiment of the invention predicting, prognosing, and/or monitoring therapeutic efficacy of cancer therapy on a subject having malignant tumor or cell proliferative disorder, is enabled by means of analysis of the *methylation status* of TFAP2E, and/or promoter or regulatory elements thereof.

Accordingly the present invention also provides predicting, prognostic and/or monitoring assays and methods, both quantitative and qualitative for detecting the expression of TFAP2E in a subject and predicting, prognosing, and/or monitoring therapeutic efficacy of cancer therapy on a subject having malignant tumor or cell proliferative disorder in said subject.

Aberrant expression of mRNA transcribed from TFAP2E is associated with the progression of having malignant tumor or cell proliferative disorder in a subject.

To detect the presence of mRNA encoding a gene or genomic sequence, a sample is obtained from the subject. The sample may be any suitable sample comprising cellular matter of the tumor. Suitable sample types include metastatic cells, cell lines, histological slides, biopsies, paraffin-embedded tissue, frozen tissue, formalin fixed tissue, body fluids, stool, colonic effluent , urine, blood plasma, blood serum, whole blood, isolated blood cells, cells isolated from the blood and combinations thereof. and all possible combinations thereof.

The sample may be treated to extract the RNA contained therein. The resulting nucleic acid from the sample is then analyzed. Many techniques are known in the state of the art for determining absolute and relative levels of gene expression, commonly used techniques suitable for use in the present invention include in situ hybridization (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridization probes (e.g. TaqMan, LightCycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (*Ausubel et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression. DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of TFAP2E and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy^{®}3- or Cy^{®}5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy^{®}3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridisation using a fluorescent microarray scanner. The fluorescent intensity of each spot indicates the level of expression of the analysed gene; bright spots correspond to strongly expressed genes, while dim spots indicate weak expression.

Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridisation, array imperfections or variability in the array set-up, cDNA labelling, hybridisation or washing. Data normalization allows the results of multiple arrays to be compared.

### METHYLATION SENSITIVE RESTRICTION ENZYME ANALYSIS

In an alternative embodiment of the invention the above described second step may be carried out by means of methylation sensitive or methylation specific restriction enzyme analysis. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of Msel, BfaI, Csp6I, Tru1I, Tvu1I, Tru9I, Tvu9I, MaeI and XspI. Particularly preferred is the use of two or three such enzymes. Particularly preferred is the use of a combination of Msel, BfaI and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of TFAP2E.

Preferably, the methylation-specific restriction enzyme is selected from the group consisting of Bsi E1, Hga I HinPl, Hpy99I, Ava I, Bce AI, Bsa HI, BisI, BstUI, BshI236I, AccII, BstFNI, McrBC, GlaI, MvnI, HpaII (HapII), HhaI, AciI, SmaI, HinP1I, HpyCH4IV, EagI and mixtures of two or more of the above enzymes. Preferred is a mixture containing the restriction enzymes BstUI, HpaII, HpyCH4IV and HinP 1I.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence of nucleotides that is complementary to, or hybridizes under moderately stringent or stringent conditions to SEQ ID NO. 1 preferably SEQ ID NO. 6, and more preferably SEQ ID NO.11, and complements thereof. Preferably said contiguous sequence is at least 16, 20 or 25 nucleotides in length. In an alternative embodiment said primers may be complementary to any adaptors linked to the fragments.

In *the fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis. Preferably said detection is carried out by hybridization to at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO. 1 preferably SEQ ID NO. 6, and more preferably SEQ ID NO.11, and complements thereof. Preferably said contiguous sequence is at least 16, 20 or 25 nucleotides in length.

Subsequent to the determination of the methylation state or level of the genomic nucleic acids the predicting, prognosing, and/or monitoring therapeutic efficacy of cancer therapy on a subject having malignant tumor or cell proliferative disorder, is deduced based upon the methylation state or level of at least one CpG dinucleotide sequence of TFAP2E, or an average, or a value reflecting an average methylation state of a plurality of CpG dinucleotide sequences of SEQ ID NO. 1 wherein methylation is associated with the predicting, prognosing, and/or monitoring therapeutic efficacy of cancer therapy on a subject having malignant tumor or cell proliferative disorder. Said methylation is in particular associated with the subject's predicting, prognosing, and/or monitoring subsequent to chemotherapy.

### CUT OFF VALUE

Wherein said methylation is determined by quantitative means the cut-off value for determining said presence of methylation is preferably zero (i.e. wherein a sample displays any degree of methylation it is determined as having a methylated status at the analyzed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are cut-off values that are at least 0.1%, 1%, 10%, 15%, 25%, and 30%.

Upon determination of the methylation and/or expression of the TFAP2E the predicting, prognosing, and/or monitoring of the subject is determined. Hypermethylation and/or decreased expression of TFAP2E is indicative of resistance to cancer therapy while hypomethylation and/or increased expression of TFAP2E is indicative of sensitivity to said cancer therapy.

### FURTHER EMBODIMENTS

The disclosed invention provides treated nucleic acids, derived from genomic SEQ ID NO. 1, preferably SEQ ID NO. 6, and more preferably SEQ ID NO.: 11, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. In a preferred embodiment of the invention, the invention provides a non-naturally occurring modified nucleic acid comprising a sequence of contiguous nucleotide bases of a sequence selected from the group consisting of SEQ ID NOS. 2-5, preferably SEQ ID NO. 7-10, and more preferably SEQ ID NO.: 12-15. In further preferred embodiments of the invention said nucleic acid is at least 50, 100, 150, 200, 250 or 500 base pairs in length of a segment of the nucleic acid sequence disclosed in SEQ ID NOs. 2-5 preferably SEQ ID NO. 7-10, and more preferably SEQ ID NO.: 12-15. Particularly preferred is a nucleic acid molecule that is identical or complementary to all or a portion of the sequences SEQ ID NOs. 2-5, preferably SEQ ID NO. 7-10, and more preferably SEQ ID NO.: 12-15 but not SEQ ID NOs. 1, 6, or 11 or other naturally occurring DNA.

It is preferred that said sequence comprises at least one CpG, TpA or CpA dinucleotide and sequences complementary thereto. The sequences of SEQ ID NOS. 2-5, 7-10, 12-15 provide non-naturally occurring modified versions of the nucleic acid according to SEQ ID NO. 1, 6, 11, respectively, wherein the modification of each genomic sequence results in the synthesis of a nucleic acid having a sequence that is unique and distinct from said genomic sequence as follows. For each sense strand genomic DNA, e.g., SEQ ID NO. 1, four converted versions are disclosed. A first version wherein "C" is converted to "T," but "CpG" remains "CpG" *(i.e.,* corresponds to case where, for the genomic sequence, all "C" residues of CpG dinucleotide sequences are methylated and are thus not converted); a second version discloses the complement of the disclosed genomic DNA sequence (i.e. *antisense* strand), wherein "C" is converted to "T," but "CpG" remains "CpG" *(i.e.,* corresponds to case where, for all "C" residues of CpG dinucleotide sequences are methylated and are thus not converted). The 'upmethylated' converted sequences of SEQ ID NO. 1 correspond to SEQ ID NOs. 2 and 4. A third chemically converted version of each genomic sequences is provided, wherein "C" is converted to "T" for all "C" residues, including those of "CpG" dinucleotide sequences *(i.e.,* corresponds to case where, for the genomic sequences, all "C" residues of CpG dinucleotide sequences are *un*methylated); a final chemically converted version of each sequence, discloses the complement of the disclosed genomic DNA sequence (i.e. *ant*isense strand), wherein "C" is converted to "T" for all "C" residues, including those of "CpG" dinucleotide sequences *(i.e.,* corresponds to case where, for the complement *(ant*isense strand) of each genomic sequence, all "C" residues of CpG dinucleotide sequences are *un*methylated). For example, the 'downmethylated' converted sequences of SEQ ID NO. 1 corresponds to SEQ ID NOs. 3 and 5.

In an alternative preferred embodiment, the invention further provides oligonucleotides or oligomers suitable for use in the methods of the invention for detecting the cytosine methylation state within genomic or treated (chemically modified) DNA, according to SEQ ID NOs. 1-16. Said oligonucleotide or oligomer nucleic acids provide novel prognostic means. Said oligonucleotide or oligomer comprising a nucleic acid sequence having a length of preferably nine (9), sixteen (16), or twenty (20) nucleotides which is identical to, hybridizes, under moderately stringent or stringent conditions (as defined herein above), to a treated nucleic acid sequence according to SEQ ID NOs. 2-5 preferably SEQ ID NO. 7-10, and more Preferably SEQ ID NO.: 12-15 and/or sequences complementary thereto, or to a genomic sequence according to SEQ ID NO. 1, 6, and 11 respectively and/or sequences complementary thereto. Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID No. 2-5 preferably SEQ ID NO. 7-10, and more Preferably SEQ ID NO.: 12-15 or to the complements thereof. The identical or hybridizing portion of the hybridizing nucleic acids is typically at least 9, 16, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO. 2-5 preferably SEQ ID NO. 7-10, and more Preferably SEQ ID NO.: 12-15, or to the complements thereof.

Hybridizing nucleic acids of the type described herein can be used, for example, as a primer (e.g., a PCR primer), or a prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO. 1, preferably SEQ ID NO. 6, and more Preferably SEQ ID NO.: 11 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of inventive oligonucleotides of length X (in nucleotides), as indicated by polynucleotide positions with reference to, e.g., SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-1));
where n=1, 2, 3,...(Y-(X-1));
where Y equals the length (nucleotides or base pairs) of SEQ ID NO: 1 (6354);
where X equals the common length (in nucleotides) of each oligonucleotide in the set (e.g., X=20 for a set of consecutively overlapping 20-mers); and
where the number (Z) of consecutively overlapping oligomers of length X for a given SEQ ID NO 1 of length Y is equal to Y- (X-1). For example Z= 6354 -19= 6335 for either sense or antisense sets of SEQ ID NO: 1, where X=20.

Preferably, the set is limited to those oligomers that comprise at least one CpG, TpG or CpA dinucleotide.

Examples of inventive 20-mer oligonucleotides include the following set of 6335 oligomers (and the antisense set complementary thereto), indicated by polynucleotide positions with reference to SEQ ID NO: 1:
1-20, 2-21, 3-22, 4-23, 5-24, .............. and 6335- 6354.

Preferably, the set is limited to those oligomers that comprise at least one CpG, TpG or CpA dinucleotide.

Likewise, examples of inventive 25-mer oligonucleotides include the following set of 6330 oligomers (and the antisense set complementary thereto), indicated by polynucleotide positions with reference to SEQ ID NO: 1:
1-25, 2-26, 3-27, 4-28, 5-29, ...... and 6330- 6354.

Preferably, the set is limited to those oligomers that comprise at least one CpG, TpG or CpA dinucleotide.

The present invention encompasses, for *each* of SEQ ID Nos. 1-6 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO. 1 preferably SEQ ID NO. 6, and more Preferably SEQ ID NO.: 11. Preferred sets of such oligonucleotides or modified oligonucleotides of length X are those consecutively overlapping sets of oligomers corresponding to SEQ ID NO. 2-5 preferably SEQ ID NO. 7-10, and more Preferably SEQ ID NO.: 12-15 (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophores, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, *e.g.,* a chromophore, fluorophore, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of each of the CpG dinucleotides of a genomic sequence selected from the group consisting SEQ ID NO. 1 preferably SEQ ID NO. 6, and more Preferably SEQ ID NO.: 11 and sequences complementary thereto, or to the corresponding CpG, TpG or CpA dinucleotide within a sequence of the treated nucleic acids according to SEQ ID NOs. 2-5, preferably SEQ ID NO. 7-10, and, more Preferably SEQ ID NO.: 12-15 and sequences complementary thereto. However, it is anticipated that for economic or other factors it may be preferable to analyse a limited selection of the CpG dinucleotides within said sequences, and the content of the set of oligonucleotides is altered accordingly.

Therefore, in particular embodiments, the present invention provides preferably a set of at least two (2) (oligonucleotides and/or PNA-oligomers) useful for detecting the cytosine methylation state in treated genomic DNA (SEQ ID NOs. 2-5 preferably SEQ ID NO. 7-10, and more Preferably SEQ ID NO.: 12-15), or in genomic DNA (SEQ ID NO. 1, 6, and 11 respectively) and sequences complementary thereto). These probes enable determination of the predicting, prognosing, and/or monitoring therapeutic efficacy of cancer therapy on a subject having having malignant tumor or cell proliferative disorder. The set of oligomers may also be used for detecting single nucleotide polymorphisms (SNPs) in treated genomic DNA (SEQ ID NOs. 2-5 preferably SEQ ID NO. 7-10, and more Preferably SEQ ID NO.: 12-15), or in genomic DNA (SEQ ID NO. 1, 6, 11 respectively) and sequences complementary thereto).

In preferred embodiments, at least one, and more preferably all members of a set of oligonucleotides is bound to a solid phase.

In further embodiments, the present invention provides preferably a set of at least two (2) oligonucleotides that are used as 'primer' oligonucleotides for amplifying DNA sequences of one of SEQ ID NO. 1-16 and sequences complementary thereto, or segments thereof.

It is anticipated that the oligonucleotides may constitute all or part of an "array" or "DNA chip" *(i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (i.e., each analyte) can be uniquely bound by a unique label and thus detected (each label is directly counted, resulting in a digital read-out of each molecular species in the mixture).

It is particularly preferred that the oligomers according to the invention are utilized for predicting, prognosing, and/or monitoring therapeutic efficacy of cancer therapy on a subject having malignant tumor or cell proliferative disorder. It is further particularly preferred that the oligomers according to the invention are utilized for determining the predicting, prognosing, and/or monitoring of said subject subsequent to chemotherapy.

Preferred is a method of predicting, prognosing, and/or monitoring therapeutic efficacy of cancer therapy on a subject having malignant tumor and/or cell proliferative disorder comprising:
a) determining the methylation and/or expression levels of TFAP2E in a biological sample isolated from said subject; and
b) predicting, prognosing, and/or monitoring therapeutic efficacy of cancer therapy on said subject based on step a),
wherein hypermethylation and/or decreased expression of TFAP2E is indicative of resistance to cancer therapy, while hypomethylation and/or increased expression of TFAP2E is indicative of sensitivity to cancer therapy.

Preferred is a method, wherein said predicting, prognosing, and/or monitoring is determined by detecting the presence, absence or amount of CpG methylation within TFAP2E or sequence thereof.

Preferred is a method, wherein said expression level is determined by detecting the presence, absence or level of mRNA transcribed from TFAP2E.

Preferred is a method, wherein said expression level is determined by detecting the presence, absence or level of a polypeptide encoded by TFAP2E or sequence thereof.

Preferred is a method, wherein said malignant tumor and/or cell proliferative disorder is colorectal, pancreatic, gastric, head and neck, breast, non-small-cell lung, leukaemia, and/or gynaecologic cancer.

Preferred is a method, wherein said cancer therapy comprises chemotherapy, polychemotherapy, chemoradiotherapy, treatments with chemotherapeutic agents, treatments with antimetabolites, pyrimidine antagonists, cytarabine, decitabine, floxuridine, capecitabine, or Gemcitabine and/or combination thereof.

Preferred is a method, wherein said cancer therapy is 5-Flurouracil based chemotherapy or polychemothrapies.

Preferred is a method, comprising contacting genomic DNA isolated from a biological sample obtained from said subject with at least one reagent, or series of reagents that distinguishes between methylated and non-methylated CpG dinucleotides within at least one target region of the genomic DNA, wherein the target region comprises, or hybridizes under stringent conditions to a sequence of contiguous nucleotides of SEQ ID NO: 1, wherein said contiguous nucleotides comprise at least one CpG dinucleotide sequence.

More preferred is a method which further comprises:
a) contacting the treated genomic DNA, or the treated fragment thereof, with an amplification enzyme and at least one oligonucleotide comprising a contiguous sequence of nucleotides that is complementary to, or hybridizes under moderately stringent or stringent conditions to a sequence selected from the group consisting of SEQ ID NOs: 2-5, and complements thereof, wherein the treated genomic DNA or the fragment thereof is either amplified to produce at least one amplificate, or is not amplified; and
b) determining, based on a presence, absence or amount of, or on a property of said amplificate, the methylation state or level of at least one CpG dinucleotide of SEQ ID NO: 1, or an average, or a value reflecting an average methylation state or level of a plurality of CpG dinucleotides of SEQ ID NO: 1, whereby predicting, prognosing, and/or monitoring of said subject is afforded.

More preferred is a method, wherein treating the genomic DNA, or the fragment thereof, comprises use of a reagent selected from the group comprising of bisulfite, hydrogen sulfite, disulfite, and combinations thereof.

Preferred is also a method, wherein the biological sample obtained from the subject is selected from the group comprising metastatic cells, cell lines, histological slides, biopsies, paraffin-embedded tissue, frozen tissue, formalin fixed tissue, body fluids, stool, colonic effluent , urine, blood plasma, blood serum, whole blood, isolated blood cells, cells isolated from the blood and combinations thereof.

Preferred is also a method which further comprises:
a) digesting the genomic DNA, or a fragment thereof, isolated from a biological sample obtained from said subject with one or more methylation sensitive restriction enzymes;
b) contacting the DNA restriction enzyme digest of a), with an amplification enzyme and at least one oligonucleotide suitable for the amplification of a sequence comprising at least one CpG dinucleotide of SEQ ID NO: 1;
c) determining, based on a presence, absence or class of an amplificate the methylation state or level of at least one CpG dinucleotide of at least one sequence selected from the group consisting of SEQ ID NO: 1, and therefrom predicting, prognosing, and/or monitoring of said subject is afforded.

Another preferred aspect of the invention is a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) one or more reagents to convert unmethylated cytosine bases to uracil or to anther base that is detectably dissimilar to cytosine in terms of hybridization properties (b) at least one oligonucleotide or polynucleotide able to hybridize under stringent or moderately stringent conditions to at least one sequence selected from the group consisting of SEQ ID NOs.: 2-5 in order to detect the presence, absence or amount of CpG methylation within TFAP2E, c) containers suitable for containing the said one or more reagents and the biological sample of the patient; and optionally (d) instructions for use.

Another preferred aspect of the invention is a kit for predicting, prognosing, and/or monitoring therapeutic efficacy of cancer therapy in a subject having malignant tumor or cell proliferative disorder who has undergone or is undergoing chemotherapy and/or chemoradiotherapy, characterized in that said kit comprises (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to a sequence of SEQ ID NO: 1 in order to detect the presence, absence or amount of CpG methylation within TFAP2E; and optionally (d) instructions for use.

Moreover, another preferred aspect of the invention is the use of a method according to claims 1 to 12, and/or a kit according to claims 13 or 14 in predicting, prognosing, and/or monitoring therapeutic efficacy of cancer therapy in a subject having malignant tumor or cell proliferative disorder.

**Table 1 Genomic Sequences and Treated Variants Thereof According to the Invention**

| Gene | Genomic SEQ ID NO: | Pretreated methylated sequence (sense) SEQ ID NO: | Pretreated unmethylated sequence (sense) SEQ ID NO: | Pretreated methylated strand (antisense) SEQ ID NO: | Pretreated unmethylated sequence (antisense) SEQ ID NO: |
|---|---|---|---|---|---|
| TFAP2E | 1 | 2 | 3 | 4 | 5 |
| TFAP2E (793 nt ― 2728 nt) | 6 | 7 | 8 | 9 | 10 |
| TFAP2E (1577 nt ― 1717 nt) | 11 | 12 | 13 | 14 | 15 |
| DKK4 | 16 | - | - | - | - |

**Table 2 Primer and probe sequences.**

| SEQ ID NO. | GENE | Primer sequence | Product length (when applicable) | Accession/Location (when applicable) | Usage |
|---|---|---|---|---|---|
| **TFAP2E** | | | | | |
| 17 | Forward | CCGGACGCTTGCCTGCAGTC | | | MSP (Genomic) |
| 18 | Reverse | CCCAGAAGCGGCCTTCGCATC | | | MSP (Genomic) |
| 19 | Probe | AGCCCGAGAACGCCCACCGCAG | | | MSP (Genomic) |
| 20 | Forward | TCGGACGTTTGTTTGTAGTT | | | MSP (Treated) |
| 21 | Reverse | CCCAAAAACGACCTTCGCATC | | | MSP (Treated) |
| 22 | Probe | AGTTCGAGAACGTTTATCGTAG | | | MSP (Treated) |
| 23 | Reverse | CCGAACGCTTACCTACAATC | CpG Island | Intron3 | Methylight/ MS-HRM |
| 24 | Forward | TTTAGAAGCGGTTTTCGTATC | 139bp | NC_000001 .10 | Methylight/ MS-HRM |
| 25 | Probe | TTGCGGTGGGCGTTTTCGGGTT | 3'BHQ1-5'FAM | | Methylight/ MS-HRM |
| 26 | Forward | TAGTTTGAAAGGATTTTTTTTAGT | | | BSP (Treated) |
| 27 | Reverse | ACTCTAATCAATATAAACTAAATTCA | | | BSP (Treated) |
| 28 | Reverse | CAACCTAAAAAATCCTCCTCAAC | CpG Island | Promoter/E xon1 | BSP (Treated) |
| 29 | Forward | GTTTTGATTAATGTGGGTTGAATTTA | 753bp | NC_000001 .10 | BSP (Treated) |
| 30 | Forward | CAGCCTGAAAGGATCCTCCTCAGC | | | BSP (Genomic) |
| 31 | Reverse | GCTCTGATCAATGTGGGCTGAATTCA | | | BSP (Genomic) |
| 44 | Forward | TAGACCAGTCCGTGATCAAGAAAGT | 310bp | NM_17854 8.3 | Expression |
| 45 | Reverse | AGGTTGAGCCCAATCTTCTCTAAC | bp 747-1056 | Exon 3-5 | Expression |
| 46 | Forward | CACCTACTCCGCCATGGAG | 1331bp | NM_178548.3 | cDNA |
| 47 | Reverse | GTGGGAGAAGCAGTTATTTCCG | CDS | Exon 1-7 | cDNA |

| **DKK4** | | | | | |
|---|---|---|---|---|---|
| 32 | Forward | ATATTAGAAAGGCAGCTTGATGAG | 206bp | NM_01442 0.2 | Expression |
| 33 | Reverse | TTACAAATTTTCGTCCAAAAATGAC | bp 406-587 | Exon 3-4 | |
| 34 | Forward | GAAAGGGATGAAGCAGAAGTTTTA | 1kb | NC_000008 .10 | Luciferase |
| 35 | Reverse | GTCGTCTGTTTGTCACTGCTTTT | 2 put. AP2 BS | Promoter | |
| 36 | Forward | CTCCCAAAGTGCTGGGATTA | 2kb | NC_000008 .10 | Luciferase |
| 37 | Reverse | GCACGTCGTCTGTTTGTCAC | 4 put. AP2 BS | Promoter | |
| 38 | Forward | TTTAAGCGGTTGGGATTTTG | 500bp | NC_000008 .10 | Luciferase |
| 39 | Reverse | TAACCAGATGTGCCTCCTCC | 0 put. AP2 BS | Promoter | |
| 40 | Forward | TTCGCCTGTGTATATTGCCA | 114bp | NC_000008 .10 | ChIP 1 |
| 41 | Reverse | GATAAAGGAAAGAGCCCCCA | 2 put. AP2 BS | Promoter | |
| 42 | Forward | TAAGCGGTTGGGATTTTGAC | 300bp | NC_000008 .10 | ChIP 2 |
| 43 | Reverse | GGCAGAGCAGGATGTCTGTA | 2 put. AP2 BS | Promoter | |

| **ACTB** | | | | | |
|---|---|---|---|---|---|
| 48 | Forward | TGGTGATGGAGGAGGTTTAGTAAGT | 132bp | NG_007992 .1 | Methylight |
| 49 | Reverse | AACCAATAAAACCTACTCCTCCCTTAA | | | |
| 50 | Probe | ACCACCACCCAACACACAATAACAAA CACA | 3'TAMRA-5'FAM | | |

| **TFAP2A** | | | | | |
|---|---|---|---|---|---|
| 51 | Forward | CTCGATCCACTCCTTACCTCAC | 396bp | NM_00322 0.2 | Expression |
| 52 | Reverse | CCTGCAGGCAGATTTAATCCTA | bp 693-1088 | Exon4-7 | |

| **TFAP2B** | | | | | |
|---|---|---|---|---|---|
| 53 | Forward | AATGGAAGACGTCCAGTCAGTT | 431bp | NM_00322 1.3 | Expression |
| 54 | Reverse | AGTGAACAGCTTCTCCTTCCAC | bp 691-1121 | Exon 2-6 | |

| **TFAP2C** | | | | | |
|---|---|---|---|---|---|
| 55 | Forward | GATCAGACAGTCATTCGCAAAG | 388bp | NM_00322 2.3 | Expression |
| 56 | Reverse | CAAAGTCCCTAGCCAAATGAAC | bp 808-1195 | Exon 3-6 | |

| **TFAP2D** | | | | | |
|---|---|---|---|---|---|
| 57 | Forward | GCCAAGGTGGAGTGATAAGAAG | 392bp | NM_17223 8.3 | Expression |
| 58 | Reverse | GGCAAGATGTTCTCCTACTGCT | bp 1087-1478 | Exon 3-6 | |

**Table 3.** Micro Array analysis.

Data obtained by microarray were submitted to Gene Expression Omnibus www.ncbi.nlm.nih.gov/geo

**Table 4. Characteristics of patients with colorectal cancer screened with MethyLight for TFAP2E methylation (discovery cohort).**

| | **Total** | **Positive for TFAP2E methylation (cutoff 30%)** | **Negative for TFAP2E methylation (cutoff 30%)** | **p-value** |
|---|---|---|---|---|
| Patients | 74 | 38 | 36 | - |
| Age mean +/-SD range | 67.2 +/- 9.7 37-93 | 69.6 +/-9.2 55-93 | 64.8 +/-10.2 37-83 | 0.23 |
| Gender male | 43 (58%) | 23 (60%) | 20 (55%) | 0.81 |
| female | 31 (42 %) | 15 (40%) | 16 (45%) | |
| Tumor Type | | | | |
| Mucinous | 16/64 (25%) | 9/34 (27%) | 7/30 (23%) | >0.99 |
| Grading | | | | |
| G0-G1 | 59/64 (92%) | 30/34 (88%) | 29/30 (97%) | 0.36 |
| G2-G3 | 5/64 (8%) | 4/34 (11%) | 1/30 (3%) | |
| Localisation | | | | |
| Rectum | 19/64 (30%) | 8/34 (24%) | 11/30 (37%) | 0.25 |
| Sigma | 19/64 (30%) | 9/34 (26%) | 10/30 (33%) | |
| Colon | 26/64 (40%) | 17/34 (50%) | 9/30 (30%) | |
| Staging | | | | |
| T1-T2 | 17/64 (27%) | 11/34 (32%) | 6/30 (20%) | 0.40 |
| T3-T4 | 47/64 (73%) | 23/34 (68%) | 24/30 (80%) | |
| Lymph Nodes | | | | |
| N0 | 26/64 (41%) | 14/34 (41%) | 12/30 (40%) | >0.99 |
| N1-N2 | 38/64 (59%) | 20/34 (59%) | 18/30 (60%) | |
| Metastasis | | | | |
| M0 | 59/64 (92%) | 31/34 (91%) | 28/30 (93%) | >0.99 |
| M1 | 5/64 (8%) | 3/34 (9%) | 2/30 (7%) | |

### EXPERIMENTS AND EXAMPLES

While the present invention has been described with specificity in accordance with certain of its preferred embodiments, the following example serve only to illustrate the invention and are not intended to limit the invention within the principles and scope of the broadest interpretations and equivalent configurations thereof.

### Patients and tissue samples

Samples were obtained from patients undergoing colorectal cancer surgery or chemotherapy at the University Hospitals in Munich, Mannheim, Bochum, Berlin and Dresden (all Germany). Tissues were obtained during resection of the primary tumor or by biopsy. Samples were either frozen tissue, formalin-fixed, paraffin-embedded (FFPE) and histology was verified by experienced pathologists (CR, AT; RL, PS). Response to chemotherapy was assessed by RECIST criteria, response to chemoradiotherapy by histology as previously described. Informed consent was obtained prior to enrollment in the study and the study was approved by the Human Subjects Committee of the Technische Universität München.

### Cell culture and 5-aza-cytidine treatment

Colon cancer cell lines LOVO and DLD-1 were obtained from the *Deutsche Sammlung von Mikroorganismen und Zellkulturen GmbH* (Braunschweig, Germany). SW480, HT-29, HCT-116 and CACO-2 colon cancer cell lines were obtained from ATCC (Washington, DC, USA). Cells were cultured in 90% DMEM medium supplemented with 10% fetal bovine serum and 2 mM L-glutamine (Invitrogen, Karlsruhe, Germany). CACO-2 cells were maintained in 80% DMEM with 20% FBS. Cells were seeded at a density of 1x10⁶ cells/60-mm dish, after twenty-four hours incubated with 5-aza-cytidine (final concentration 10 µmo1/L; Sigma, Munich, Germany) or the same amount of DMSO as control. Culture medium was changed daily for 3 days with recurrent 5-aza-2-deoxycytidine additions. After 3 days, the cells were harvested for total RNA extraction with the RNeasy Total RNA Mini Kit (Qiagen, Hilden, Germany).

### Primer design

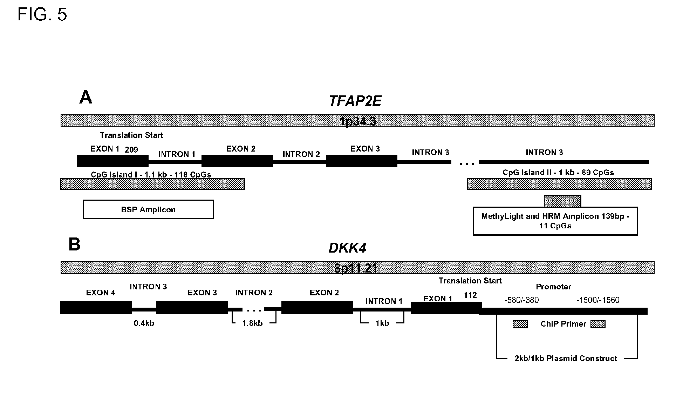

Primers for methylated TFAP2E covered CpGs in the second CpG island (Figure 5). Results from the MethyLight assays were confirmed using bisulfite sequencing (in selected cases) and Methylation-Sensitive High Resolution Melting (MS-RHM) Analysis. To avoid assay deviations due to limited DNA content/degradation in the clinical samples, only such samples were included in the analysis that showed sufficient expression of the reference gene (ACTB), reflected by a Cp of>37 cycles, and value deviations of>1 cycle in the replicate measurements. Since MS-HRM tends to be more sensitive then classical MSP or MethyLight, MS-HRM was also used with the same primers. Primer sequences are listed in Table 2. MethyLight and MS-HRM conditions were as follows: 10 min 95°C for activation and then 50 cycles consisting of 15 sec 95°C and 30 sec 60°C and 10 sec 72°C (data acquisition at this step), for MS-HRM this was followed by 1 min 95°C, 1 min 40°C, 1sec 65°C and then continuous melting to 95°C.

### DNA and RNA extraction

The RNA and DNA extraction from cell lines or 10-25 mg of frozen tissue samples was carried out using the RNeasy Total RNA Mini Kit or the QIAmp DNA Mini Kit (Qiagen, Hilden, Germany) respectively, using the manufacturer's instructions.

### RT-PCR and MethyLight/MS-HRM analysis

Genomic DNA was treated with the Epitect Bisulfite Conversion Kit (Qiagen, Hilden, Germany) according to the manufacturer's instructions and then analyzed using the MethyLight technique or MS-HRM as previously described. Cutoff values giving the best discrimination between non-neoplastic mucosa and tumor samples were determined by receiver operating characteristic (ROC) analysis. The expression analysis of the mRNA was performed by RT-PCR with the Verso cDNA Kit (Thermo Fischer, Darmstadt, Germany) according to the manufacturer's instructions.

### Expression Microarray and verification of target candidates

pTFAP2E SW480 clones and SW480 pTarget clones were used for global expression analysis using a Human Gene 1.0 ST Expression Array (Affymetrix, High Wycombe, United Kingdom) according to the manufacturer's protocol. Genes with a more than 3-fold expression change were verified by quantitative RT-PCR (see table 3).

### Reporter and expression vectors

Full length TFAP2E coding sequence was amplified from SW480 cells and cloned with and without FLAG epitope (pTFAP2E and pTFAP2eFlag) into the pTarget vector (Promega, Mannheim, Germany) according to the manufacturer's instructions. The DKK4 promoter sequence was also amplified and cloned into pGL3 basic vector (Promega) - pGL3-DKK4-1kb (insert size: 1kb, from minus 1kb of the transcription start site). The pRL-TK reporter plasmid (Promega) was used as an internal control reporter vector. Full length DKK4-CDS (pcDNA3-Dkk4) and a DDK4 promoter plasmid - pGL3-DKK4-2kb (pGL3 basic vector- insert: 2kb, minus 2000 bp of the transcription start) were obtained from Dr. Kolligs, München.

### Generating of clones with stable overexpression of TFAP2E

SW480 clones stably overexpressing TFAP2E and control clones using an empty pTarget vector were obtained after transfection with pTFAP2E and pTFAP2eFlag using Lipofectamine 2000 (Invitrogen, Karlsruhe, Germany) according to the manufacturer's instructions. Stable transfectants were selected upon G418 treatment (Invitrogen, Karlsruhe, Germany) for 2 weeks. Single colonies were picked and grown further in selective media. TFAP2E expression was assessed by quantitative RT-PCR.

### Transient transfections and luciferase assays

SW480, CACO-2 and HT-29 cells were transfected with either pGL3-DKK4-1kb or pGL3-DKK4-2kb, and pRL-TK as transfection control (renilla luciferase). Transfections were carried out with Satisfection Transfection Reagent (Agilent, Waldbronn, Germany). After 3 days, cells were harvested and firefly and renilla luciferase activities were measured on a luminometer using the Luciferase Dual Reporter Assay (Promega, Mannheim, Germany). All experiments were repeated independently 3 times and all cells were seeded in triplicate on the 96 well plates.

### Chromatin immunoprecipitation

pTFAP2eFlag SW480 clones were used for ChIP according to the protocol of ChIP assay kit (Upstate Biotechnology Inc, New York, USA) with anti-FLAG antibody (Sigma, Munich, Germany). Primer sequences of the DKK4 promoter region flanking putative AP-2 protein binding sites are given in table 2.

### Stress resistance and cell survival assays after drug exposure

Cells were seeded in 96 well plates (20.000 per well) and cell proliferation was measured by MTT (Sigma, Munich, Germany) and BrDU (Roche, Penzberg, Germany) assays according to the manufacturer's protocol. For assessment of stress induced apoptosis, cells were seeded in 96 well plates (50.000 per well) and treated with TNF-alpha (100 ng/µ1) and chlorhexamide (100 µg/µ1) for 4 days. Resistance of cells to chemotherapeutic drugs was investigated after treatment with 5-fluorouracil (50 µg/ml or 38 µM), oxaliplatin (60 µM or 24 µg/ml) or irinotecan (20 µM or 13 µg/ml) and assessed by MTT assay.

### Invasion and Migration

Cells were seeded on Transwell Matrigel Chambers with reduced growth factors (BD Biosciences, Heidelberg, Germany) according to the manufacturer's protocol (25.000 cells) in serum free media.

### Statistics

Receiver Operating Characteristic analysis was performed in order to determine the optimal methylation (PMR value) cutoff. As result, PMR values above 30% were considered as methylation positive and classified as '1', whereas PMR levels below 30% were classified as '0'. Correlations of the methylation event with clinicopathological features, such as primary tumor site, histological grade of differentiation or stage of cancer were assessed by the Fisher's exact test. The Mann-Whitney U test was used to compare the distribution of quantitative data between two independent samples. To consider potential cluster effects by different study centers involved, random effect models were employed for statistical analysis of response probability in relation to PMR. Based on these models, estimates of response ratios (relative risks) were provided with 95% (fixed effect) confidence intervals. All tests were two-sided, and a *p*-value of <0.05 was considered statistically significant. Statistical calculations were done with Graphpad Instat 3, Graphpad Prism 5 (GraphPad Software, Inc., La Jolla, USA), MIX 1.7 software¹³ and the R statistical software v2.9 (R Foundation for Statistical Computing, Vienna, Austria).

### RESULTS

### Frequent hypermethylation of TFAP2E in human colorectal cancer

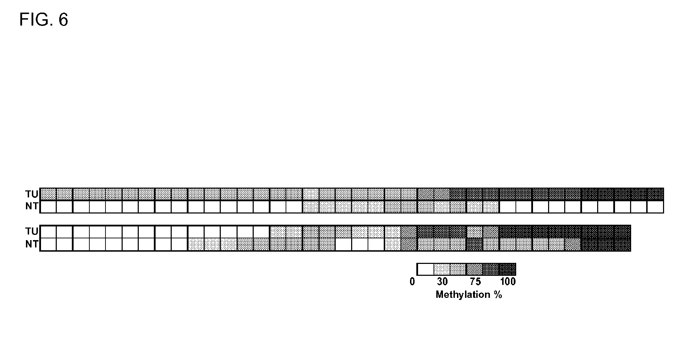

Genomic DNA was obtained from primary CRC and adjacent mucosa from 74 CRC patients (initial cohort, all snap frozen) and was analyzed for TFAP2E methylation. With a cut-off value of 30%, 38/74 patients (51.4%) were classified as methylated (Figure 6). None of the common clinicopathological characteristics of CRC patients, including primary tumor site, histological grade of differentiation or stage of cancer, age or gender, correlated with TFAP2E methylation status (Table 4).

### Hypermethylation of TFAP2E in CRC is associated with decreased gene expression

Tumor-mRNA from 28/74 CRCs (initial cohort) was analyzed for TFAP2E expression by RT-PCR. In 12 cases TFAP2E methylation and decreased TFAP2E expression was observed, while 8 cases showed no methylation and strong expression. The remaining 8 cases showed either expression with methylation or no expression without methylation. There was a significant correlation between methylation and expression (Fisher's exact test, p < 0.05).

### Analysis of TFAP2E expression and methylation in CRC cell lines

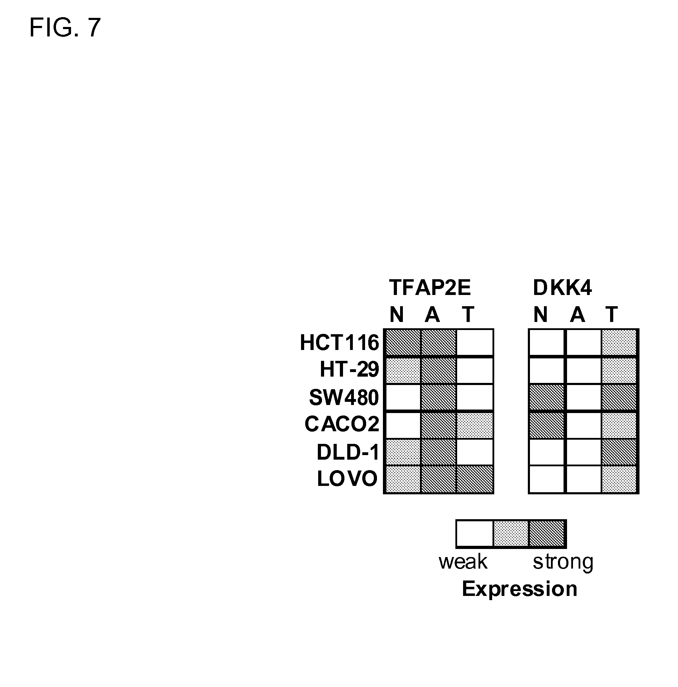

HCT-116, HT-29, DLD-1, LOVO, CACO-2, and SW480 cells were analyzed by RT-PCR for the expression of TFAP2E. While HCT-116, HT-29, DLD-1 and LOVO had constitutive TFAP2E expression, CACO-2 and SW480 cells showed expression after 5-aza-cytidine treatment only. Treatment with Trichostatin A showed no effect on TFAP2E mRNA levels, except for CACO-2 which showed a weak expression after treatment. MethyLight revealed a high degree of TFAP2E methylation in all CRC cell lines, which declined after 5-azacytidine treatment (Figure 7).

### Expression of TFAP2E affects migration of human CRC cells

Migration of cancer cells was assessed in stable clones of SW480 transfected with pTFAP2E or pTarget (empty vector control), designated SW480-pTFAP2E and SW480-pTarget, respectively. A minor 1.3-fold increase of migrating cells was observed in control versus clones stably overexpressing TFAP2E (p < 0.05). In contrast, no differences in proliferation and stress resistance were observed (data not shown).

### Identification of DKK-4 as a downstream target of TFAP2E

For the identification of potential downstream targets of TFAP2E, SW480-pTFAP2E and SW480-pTarget cell clones were subjected to microarray analysis (Table 3). The verification of these results by quantitative real-time PCR confirmed only DKK4 as a target gene that was significantly downregulated (mean 6-fold, range 3- to 9- fold) in all stable SW480-pTFAP2E clones versus empty SW480-pTarget vector controls. DKK4 mRNA levels were then analyzed in untransfected CRC cell lines. SW480 and CACO-2 showed strong DKK4 expression that was lost upon re-expression of TFAP2E through 5-azacytidine treatment. All cell lines with basal expression levels of TFAP2E (HCT-116, HT-29, DLD-1, LOVO) showed no DKK4 expression (Figure 7).

### TFAP2E binds to the DKK-4 promoter and represses expression in vitro

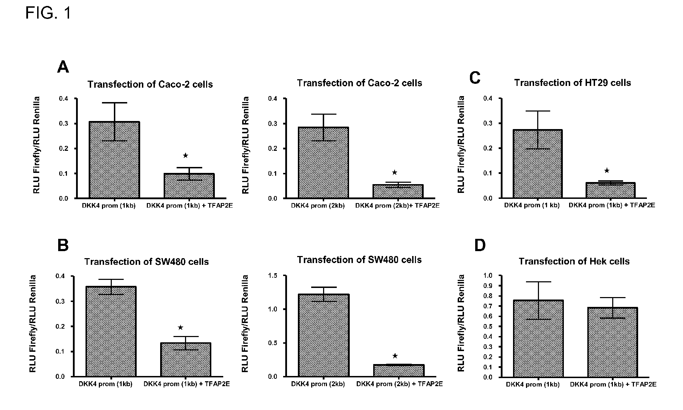

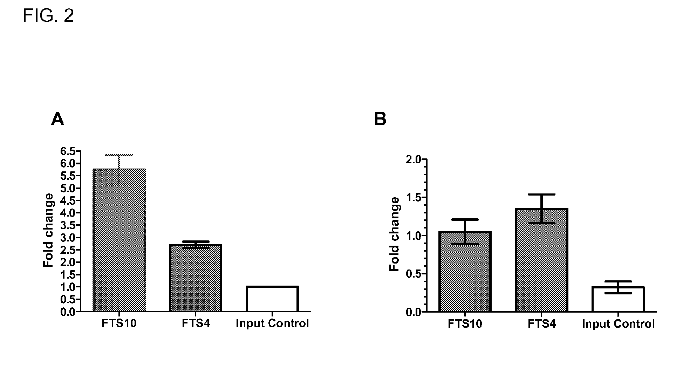

Using the Transcription Element Search System we found 2 putative binding sites for AP-2 proteins within 2kb upstream and 2 sites within 1kb upstream of the DKK4 mRNA transcription start site (Figure 5). To analyze binding of TFAP2E to these sites, 1kb and 2kb of the DKK4 promoter were cloned into a Luciferase reporter vector (pGL3) and transfected together with pTFAP2E and pRL-TK (internal control) into SW480, CACO-2 (no TFAP2E expression) and HT-29 (weak TFAP2E expression) cells. Luciferase activity was decreased in CACO-2 cells transfected with TFAP2E compared to cells without TFAP2E transfection, 3-fold with the pGL3-DKK4-1kb vector and 5-fold with the pGL3-DKK4-2kb vector (Figure 1A). SW480 cells showed the same effect with 3- to 7-fold downregulation (7-fold with pGL3-DKK4-2kb vector, Figure 1B) as well as in HT-29 cells (3-fold with pGL3-DKK4-1kb vector, Figure 1C; Wilcoxon test p < 0.005). HEK cells showed no difference in luciferase activity with or without TFAP2E transfection. This might be due to the high endogenous TFAP2E expression level (Figure ID). As a control experiment, a 500bp vector construct of the DKK4 promoter flanking, but not including the 2 binding sites present in the pGL3-DKK4-1kb vector, was transfected into SW480 cells together with TFAP2E-CDS, showing no reduction of luciferase activity and indicating that these sites are indeed crucial for TFAP2E binding (data not shown). To prove that the TFAP2E protein binds directly to the DKK4 promoter, chromatin immunoprecipitation was performed with cells of two stable SW480 pTFAP2eFlag clones. The recovered DNA of the input control, negative control and the SW480 pTFAP2eFlag clones was used for real time PCR with two primer pairs flanking 4 putative AP-2 protein binding sites in the DKK4 promoter (two sites 2kb upstream and two sites 1kb upstream of the DKK4 transcription start site), respectively. While the negative control showed no amplification, both clones showed a 4-fold higher amplification for the two sites 2kb upstream (Figure 2A) and 1.5-fold higher for the two sites 1kb upstream (Figure 2B), indicating a direct binding of TFAP2E protein to the DKK4 promoter.

### Inverse TFAP2E and DKK4 expression pattern in colorectal cancer patients

Tumor-mRNA from 28 CRCs (out of 74 CRCs) was used for expression analysis. In 11 cases the DKK4 expression pattern (strong expression) was inversely related to the expression of TFAP2E (decreased expression) and vice versa in 10 cases (no DKK4 expression and TFAP2E expression), 7 cases showed comparable expression levels in both tissue types. The inverse correlation between TFAP2E and DKK4 expression proved to be significant (Fisher's exact test, p < 0.01).

Expression of TFAP2E affects resistance to 5-FU in human CRC cells

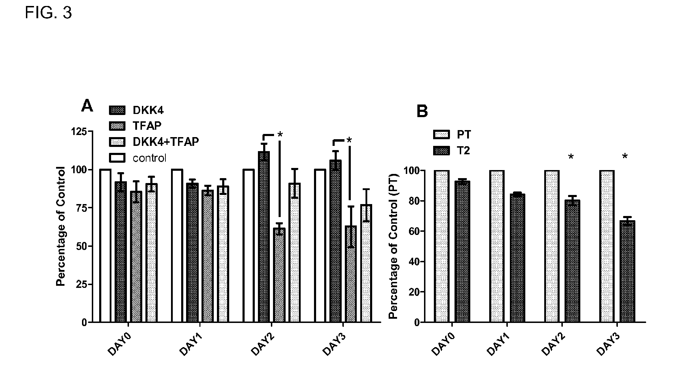

Since DKK4 has been implicated in the responsiveness to chemotherapy and the present invention findings point to DKK4 as a potential downstream target of TFAP2E, the role of TFAP2E and DKK4 in mediating drug resistance in CRC cancer cells *in vitro* was assessed. SW480 cells were transiently transfected with pTFAP2E or pcDNA3-Dkk4 and treated with oxaliplatin, irinotecan or 5-FU. Surviving cells were measured by MTT assays. All experiments were performed in triplicates. SW480-pTFAP2E transfected cells showed a significant decrease (about 20% compared to controls) and SW480 pcDNA3-Dkk4 transfected cells showed a significant increase in survival (about 10% compared to controls) when treated with 5-FU after 2 days (Kruskal-Wallis test, p < 0.01) (Figure 3A). Cells transfected with both pTFAP2E and pcDNA3-Dkk4 exhibited intermediate responsiveness. In addition, these results were confirmed in SW480-pTFAP2E clones and SW480-pTarget vector controls which were treated with 5-FU (Mann-Whitney U test, p < 0.001, Figure 3B).

### TFAP2E methylation predicts resistance to cancer chemotherapy

Next, TFAP2E methylation was assessed in 4 different cohorts of colorectal cancer patients undergoing chemotherapy or chemoradiotherapy with a 5-FU based regimen in order to analyse chemoresistance *in vivo.*

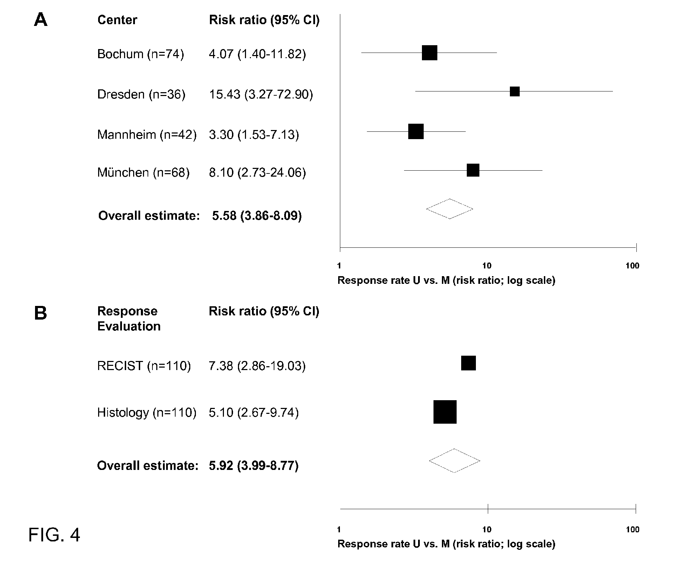

Cohort I (Bochum) included 76 patients who were enrolled in a prospective trial comparing the oral fluoropyrimidine capecitabine with intravenous 5-FU together with oxaliplatin (CAPOX versus FUFOX) in metastatic CRC patients. Of the 76 samples, 74 cases yielded enough DNA for methylation analysis and clinical response data was available for all of them. Of these patients, 36 (49%) where classified as responders and 38 (51%) as nonresponders according to RECIST criteria. TFAP2E methylation was found in 3 responders and 17 nonresponders (23%); 33 (45%) responders and 21 nonresponders (28%) were unmethylated. TFAP2E methylation was significantly more frequent in non-responders (Fisher's exact test p < 0.0005; Table 1 and Figure 4A).

Cohort II (Dresden) consisted of 44 samples from patients with metastatic CRCs undergoing 5-FU based polychemotherapy (FOLFIRI/FOLFOX). Of these, 43 yielded DNA for methylation analysis, clinical response data was available for 39 patients but 3 patients stopped chemotherapy due to intolerable toxicity and were excluded from the analysis. Of these remaining 36 patients, 14 (39%) were classified as responders and 22 (61%) were classified as nonresponders according to RECIST criteria. TFAP2E methylation was observed in all 22 nonresponders and 1 responder (64%), 13 (36%) responders showed no methylation. The 3 patients who stopped chemotherapy due to toxicity (excluded from the response analysis) showed also no TFAP2E methylation. Again, the correlation between TFAP2E methylation and treatment response was significant (Fisher's exact test p < 0.0001; Table 1 and Figure 4A).

For cohort III (Mannheim), DNA from 50 patients with rectal cancer undergoing chemoradiotherapy with 5-FU in combination with Irinotecan, Cetuximab was analyzed with response based on histological evaluation. 49 samples yielded DNA, but for only 42 patients matching clinical data was available. Of these, 25 (60%) responders and 17 (40%) nonresponders were classified by histological criteria. TFAP2E methylation was found in 5 (12%) responders and 14 (33%) nonresponder, while 20 (48%) responders and 3 nonresponders were unmethylated. The correlation between TFAP2E methylation and treatment response was significant (Fisher's exact test p < 0.0001; Table 1 and Figure 4A) in this cohort as well.

Cohort IV (München) consisted of 70 patients with primary rectal cancer who underwent 5-FU based chemoradiotherapy (intravenous 5-FU and 45.0 Gy). Treatment response was determined again by histological response criteria. From 70 samples, 69 yielded enough DNA and clinical response data were available in 68 cases. Of the 68 patients analyzed, 32 (47%) were classified as responders and 36 (53%) as nonresponders. TFAP2E methylation was observed in 3 (4%) responders and 28 (41%) nonresponders, while 29 (43%) responders and 8 (12%) nonresponders showed no methylation. The correlation between TFAP2E methylation and treatment response was also significant in this cohort (Fisher's exact test p < 0.0001; Table 1 and Figure 4A).

The association of methylation and treatment response was apparent in all four patient series (higher response for non-methylated patients), which is shown by a significant difference in response rates. A substantial effect size was estimated for the pooled cohort data (Figure 4A) with a response ratio of 5.58 (95%CI: 3.86-8.09). When the cohorts were pooled according to response evaluation criteria (histology or RECIST), the overall estimated effect was even stronger with a response ratio of 5.92 (95%CI: 3.99- 8.77; Figure 4B).

The above results show that the TFAP2E gene is frequently hypermethylated in cancers from CRC patients and in these cancers TFAP2E mRNA expression is either lost or downregulated. Loss or downregulation of TFAP2E expression was also observed in CRC cell lines but was restored by the treatment with the methylation inhibitor 5-azacytidine. Further, the functional role of TFAP2E in CRC was assessed. After stable transfection of CRC cells *in vitro,* no difference in apoptosis or proliferation was observed, however, a significant difference in cell migration was observed after TFAP2E overexpression.

In order to characterize the down-stream targets of TFAP2E microarray analysis of TFAP2E overexpressing cells was performed and found DKK4 to be a potential target gene which was significantly down regulated via TFAP2E.

DKK4 is a member of the dickkopf family, comprising various antagonists of WNT-signaling by binding to WNT-coreceptors LRP5/6. DKK4 overexpression has been observed in the colon mucosa of patients with colitis. However, the precise role of DKK4 in the colon mucosa and its contribution to carcinogenesis is so far unknown. Recently, DKK4 was implicated in 5-FU resistance in CRC cell lines. The present invention *in vitro* data from cell lines treated with 5-FU, oxaliplatin and irinotecan confirmed this observation. DKK4 overexpression led to increased 5-FU chemoresistance in CRC cell lines, while introduction of TFAP2E was associated with an increased sensitivity towards 5-FU treatment. According to the above results, there exists a strong association between TFAP2E methylation and lack of chemotherapy response in the tumor. Fixed effect model analysis of the pooled cohort data revealed an estimated 6-fold higher response probability for non-methylated patients. Interestingly, the correlation was observed in primary rectal cancers and metastatic colorectal cancers, independent of the treatment with 5-FU based chemotherapy or chemoradiotherapy. Also, assessment of response in these cohorts, either by standard RECIST criteria or by histological response, did not influence this strong association, indicating that TFAP2E methylation is valuable for response prediction in either setting.

## Claims

1. A method of predicting, prognosing, and/or monitoring therapeutic efficacy of cancer therapy on a subject having malignant tumor and/or cell proliferative disorder comprising:
a) determining the methylation and/or expression levels of TFAP2E in a biological sample isolated from said subject; and
b) predicting, prognosing, and/or monitoring therapeutic efficacy of cancer therapy on said subject based on step a),
wherein hypermethylation and/or decreased expression of TFAP2E is indicative of resistance to cancer therapy, while hypomethylation and/or increased expression of TFAP2E is indicative of sensitivity to cancer therapy.

2. The method according to claim 1, wherein said predicting, prognosing, and/or monitoring is determined by detecting the presence, absence or amount of CpG methylation within TFAP2E or sequence thereof.

3. The method according to claim 1, wherein said expression level is determined by detecting the presence, absence or level of mRNA transcribed from TFAP2E.

4. The method according to claim 3, wherein said expression level is determined by detecting the presence, absence or level of a polypeptide encoded by TFAP2E or sequence thereof.

5. The method according to claim 1, wherein said malignant tumor and/or cell proliferative disorder is colorectal, pancreatic, gastric, head and neck, breast, non-small-cell lung, leukaemia, and/or gynaecologic cancer.

6. The method according to claim 1, wherein said cancer therapy comprises chemotherapy, polychemotherapy, chemoradiotherapy, treatments with chemotherapeutic agents, treatments with antimetabolites, pyrimidine antagonists, cytarabine, decitabine, floxuridine, capecitabine, or gemcitabine and/or combination thereof.

7. The method according to claim 1, wherein said cancer therapy is 5-Flurouracil based chemotherapy or polychemotherapy.

8. The method according to claim 2, comprising contacting genomic DNA isolated from a biological sample obtained from said subject with at least one reagent, or series of reagents that distinguishes between methylated and non-methylated CpG dinucleotides within at least one target region of the genomic DNA, wherein the target region comprises, or hybridizes under stringent conditions to a sequence of contiguous nucleotides of SEQ ID NO: 1, wherein said contiguous nucleotides comprise at least one CpG dinucleotide sequence.

9. The method according to claim 8, further comprising:
a) contacting the treated genomic DNA, or the treated fragment thereof, with an amplification enzyme and at least one oligonucleotide comprising a contiguous sequence of nucleotides that is complementary to, or hybridizes under moderately stringent or stringent conditions to a sequence selected from the group consisting of SEQ ID NOs: 2-5, and complements thereof, wherein the treated genomic DNA or the fragment thereof is either amplified to produce at least one amplificate, or is not amplified; and
b) determining, based on a presence, absence or amount of, or on a property of said amplificate, the methylation state or level of at least one CpG dinucleotide of SEQ ID NO: 1, or an average, or a value reflecting an average methylation state or level of a plurality of CpG dinucleotides of SEQ ID NO: 1, whereby predicting, prognosing, and/or monitoring of said subject is afforded.

10. The method according to claim 8, wherein treating the genomic DNA, or the fragment thereof, comprises use of a reagent selected from the group comprising of bisulfite, hydrogen sulfite, disulfite, and combinations thereof.

11. The method according to claim 1, wherein the biological sample obtained from the subject is selected from the group comprising metastatic cells, cell lines, histological slides, biopsies, paraffin-embedded tissue, frozen tissue, formalin fixed tissue, body fluids, stool, colonic effluent, urine, blood plasma, blood serum, whole blood, isolated blood cells, cells isolated from the blood and combinations thereof.

12. The method according to claim 2, further comprising:
a) digesting the genomic DNA, or a fragment thereof, isolated from a biological sample obtained from said subject with one or more methylation sensitive restriction enzymes;
b) contacting the DNA restriction enzyme digest of a), with an amplification enzyme and at least one oligonucleotide suitable for the amplification of a sequence comprising at least one CpG dinucleotide of SEQ ID NO: 1;
c) determining, based on a presence, absence or class of an amplificate the methylation state or level of at least one CpG dinucleotide of at least one sequence selected from the group consisting of SEQ ID NO: 1, and therefrom predicting, prognosing, and/or monitoring of said subject is afforded.

13. A kit for predicting, prognosing, and/or monitoring therapeutic efficacy of cancer therapy in a subject having malignant tumor or cell proliferative disorder who has undergone or is undergoing chemotherapy and/or chemoradiotherapy, **characterized in that** said kit comprises (a) one or more reagents to convert unmethylated cytosine bases to uracil or to anther base that is detectably dissimilar to cytosine in terms of hybridization properties (b) at least one oligonucleotide or polynucleotide able to hybridize under stringent or moderately stringent conditions to at least one sequence selected from the group consisting of SEQ ID NOs.: 2-5 in order to detect the presence, absence or amount of CpG methylation within TFAP2E, c) containers suitable for containing the said one or more reagents and the biological sample of the patient; and optionally (d) instructions for use.

14. A kit for predicting, prognosing, and/or monitoring therapeutic efficacy of cancer therapy in a subject having malignant tumor or cell proliferative disorder who has undergone or is undergoing chemotherapy and/or chemoradiotherapy, **characterized in that** said kit comprises (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to a sequence of SEQ ID NO: 1 in order to detect the presence, absence or amount of CpG methylation within TFAP2E; and, optionally, (d) instructions for use.

15. Use of a method according to any of claims 1 to 12, and/or a kit according to claims 13 or 14 in predicting, prognosing, and/or monitoring therapeutic efficacy of cancer therapy in a subject having malignant tumor or cell proliferative disorder.
